# EUROPEAN PATENT SPECIFICATION

(11) **EP 1 053 304 B1**
(45) Date of publication and mention of the grant of the patent: **04.07.2007**
(21) Application number: 99905615.3
(22) Date of filing: 03.02.1999
(51) Int. Cl.: C12N 9/64

(54) **CRYSTALLINE TNF-ALPHA-CONVERTING ENZYME AND USES THEREOF**
KRISTALLINES TNF-ALPHA-KONVERTIERENDES ENZYM UND VERWENDUNG DAVON
ENZYME DE CONVERSION CRISTALLINE DU FACTEUR TNF ALPHA ET SES UTILISATIONS

(30) Priority: 04.02.1998 US 73709 P; 30.03.1998 US 50083; 27.01.1999 US 117476 P
(43) Date of publication of application: 22.11.2000
(73) Proprietor: IMMUNEX CORPORATION, Seattle Washington 98101 (US); Max-Planck-Institute for Biochemistry, 82152 Martinsreid-Planegg (DE); Wyeth, Madison, New Jersey 07940 (US)
(72) Inventor: BLACK, Roy, A., Seattle, WA 98115 (US); PAXTON, Raymond, James, Bellevue, WA 98006 (US); BODE, Wolfram, D-82131 Gauting (DE); MASKOS, Klaus, D-83607 Holzkirchen (DE); FERNANDEZ-CATALAN, Carlos, D-82152 Martinsreid-Planegg (DE); CHEN, James, Ming, Bedminster, NJ 07921 (US); LEVIN, Jeremy, Ian, New City, NY 10956 (US)
(74) Representative: Wakerley, Helen Rachael
(86) International application number: PCT/US1999/002185
(87) International publication number: WO 1999/040182

(56) References cited:
- WO-A-96/41624
- WO-A-97/08300
- WO-A-97/15588
- WO-A-97/35538
- US-A- 5 594 106
- US-A- 5 702 935
- CIRILLI ET AL: "2.ANG. X-ray structure of adamalysin II complexed with a peptide phosphonate inhibitor adopting a retro-binding mode" FEBS LETTERS, vol. 418, 1 January 1997 (1997-01-01), pages 319-322, XP002089575 ISSN: 0014-5793
- GOMIS-RÜTH F ET AL: "Structures of adamalysin II with peptide inhibitors. Implications for the design of tumor necrosis factor alpha convertase inhibitors" PROTEIN SCIENCE, vol. 7, February 1998 (1998-02), pages 283-292, XP002111816
- DECICCO, CARL P. ET AL: "Novel cyclophane inhibitors of matrix metalloproteinases and TNF -alpha converting enzyme." ABSTRACTS OF PAPERS AMERICAN CHEMICAL SOCIETY, (1997) VOL. 214, NO. 1-2, PP. MEDI 96. MEETING INFO.: 214TH AMERICAN CHEMICAL SOCIETY NATIONAL MEETING LAS VEGAS, NEVADA, USA SEPTEMBER 7-11, 1997, XP002111817
- MASKOS K ET AL: "Crystal structure of the catalytic domain of human tumor necrosis factor-alpha-converting enzyme." PROCEEDINGS OF THE NATIONAL ACADEMY OF SCIENCES OF THE UNITED STATES OF AMERICA, (1998 MAR 31) 95 (7) 3408-12., XP002111818

## Description

### BACKGROUND OF THE INVENTION

The cytokine tumor necrosis factor-α (TNFα) plays a role in the induction of inflammatory reactions and is known to be cytotoxic towards tumor cells. TNFα, however, also may cause severe damage to the human body when produced in excess by eventually leading to multiple organ failure and death. See Bemelmans et al., "Tumor Necrosis Factor: Function, Release and Clearance," Crit. Rev. Immun. 16: 1-11 (1996).

Tumor necrosis factor-α is produced by activated cells, such as mononuclear phagocytes, T-Cells, B-Cells, mast cells and NK cells. TNFα exists in two forms: a type II membrane protein having a relative molecular mass of 26 kD and a soluble 17 kD form generated from the membrane form by proteolytic cleavage. The TNFα membrane protein is synthesized as a 223 amino acid membrane-anchored precursor. The soluble TNFα is released from the membrane-bound precursor by a membrane-anchored proteinase. This proteinase was recently identified as a multidomain metalloproteinase called TNFα-converting enzyme (TACE). See, Black et al., "A metalloproteinase disintigrin that releases tumor-necrosis factor-α from cells," Nature 385: 729-733 (1997), Moss et al., "Cloning of a disintigrin metalloproteinase that processes precursor tumor-necrosis factor-α," Nature 385: 733-736 (1997). TACE has recently been identified as a zinc endopeptidase consisting of an extracellular region comprising an N-terminal signal peptide, a prodomain, a 263 residue catalytic domain (TCD) that is preceded by a furin cleavage site (residues 211-214), a disintegrin domain, an EGF-like domain, and a crambin-like domain, an apparent transmembrane helix and the intracellular C-terminal tail. Tumor necrosis factor-α converting enzyme (TACE), including a polynucleotide sequence, is described in detail in the published PCT application No. WO 96/41624.

As noted above, the over-production or unregulated production of TNFα presents serious physiological dangers. It has been implicated in various deleterious physiological diseases such as rheumatoid arthritis, cachexia and endotoxic shock. It also may eventually lead to organ failure and death. Thus, a way to control or block release of TNFα into the circulation is needed. Because of TACE's role in the conversion of TNFα, inhibition, modulation, or regulation of TACE would affect the release of TNFα into circulation. Inhibitors of metalloproteinases and structure based design thereof are described in Zask et al., "Inhibition of Matrix Metalloproteinases: Structure Based Design" Current Pharmaceutical Design, 2:624-661 (1996). Thus, compounds that associate with TACE, such as inhibitors, receptors or modulators will be useful to protect patients from adverse effects associated with the over-production or unregulated production of tumor necrosis factor-α.

### SUMMARY OF THE INVENTION

The invention relates to a method of identifying a compound that associates with a TNF-α-converting enzyme polypeptide. The TNF-α-converting enzyme polypeptide may comprise the TNF-α-converting enzyme catalytic domain. Further, the TNF-α-converting enzyme polypeptide may be the expression product of a polynucleotide encoding the pro and catalytic domains of TNF-α-converting enzyme. Alternatively, the TNF-α-converting enzyme polypeptide may be the expression product of a polynucleotide encoding the amino acid residues 1-477 of TNF-α-converting enzyme. The polynucleotide may be substituted such that amino acid residue Ser266 is changed to Ala and amino acid residue Asn452 is changed to Gln, and wherein a second polynucleotide encoding the sequence Gly-Ser-(His)₆ is fused to the C-terminus.

The compositions above may further comprise a binding partner suitable for co-crystallization with the TNF-α-converting enzyme polypeptide. The binding partner may be a hydroxamate-based binding partner. Further the binding partner may be N-{D,L-2-(hydroxyaminocarbonyl)methyl-4-methylpentanoyl}-L-3-amino-2-dimethylbutanoyl-L-alanine,2-(amino)ethyl amide.

The compositions above may have a crystal structure diffracting to 2.0 Å, are monoclinic, have a unit cell comprising four crystallographically independent TNF-α-converting enzyme catalytic domain (TCD) molecules, have the TCD molecules are in an asymmetric unit, and/or have monoclinic space group P2₁ and the cell has the constants a=61.38 A, b= 126.27 A, c=81.27 A, and β=107.41°.

Further, the polypeptides above may be characterized by the structure coordinates according to Table 1, or a substantial part thereof.

A method for crystallizing a TNF-α-converting enzyme polypeptide, comprises (A) mixing a solution comprising a TACE polypeptide and a binding partner with a crystallization buffer; and (B) crystallizing the mixture of step (A) by drop vapor diffusion to form a crystalline precipitate. The method further comprises (C) transferring seeds from the crystalline precipitate formed by the drop vapor diffusion and a crystallization promotor into a mixture of a concentrated solution comprising a TACE polypeptide and binding partner substrate, and a crystallization buffer; and (D) crystallizing the mixture of step (C) by drop vapor diffusion to form a crystal. In another embodiment, the crystallization buffer is 0.1M Na Citrate pH 5.4, 20%w/v PEG 4000, and 20% v/v Isopropanol. In still another embodiment, the binding partner is N-{D,L-2-(hydroxyaminocarbonyl)methyl-4-methylpentanoyl}-L-3-amino-2-dimethylbutanoyl-L-alanine, 2-(amino)ethyl amide. In yet another embodiment, crystallization is at a temperature ranging from 4 to 20 degrees Celsius. In another embodiment, the solution comprising the TACE polypeptide and the inhibitor is at a concentration of about 5 mg/mL to about 12 mg/mL in a buffer. In a further embodiment, the solution comprising a TACE polypeptide and the binding partner is mixed with the crystallization buffer in a 1:1 ratio.

A tumor necrosis factor-α (TNF-α)-converting enzyme crystal may be made by co-crystallizing a TNF-α-converting enzyme polypeptide with a co-crystallization substrate.

It is also possible to have a computer-readable medium having recorded thereon x-ray crystallographic coordinate data for the catalytic domain of TNF-α converting enzyme, or a portion thereof. The computer-readable medium may have recorded thereon the x-ray crystallographic coordinate data set forth in Table 1, or a portion thereof. The medium may be selected from the group consisting of a floppy disc, a hard disc, computer tape, RAM, ROM, CD, DVD, a magnetic disk, and an optical disk. The computer-readable medium may have recorded thereon machine-readable data, wherein the computer-readable medium, when used in conjunction with a machine programmed with instructions for using the data, is capable of generating image signals for depicting a graphical, three-dimensional representation of a TNF-α converting enzyme polypeptide, or portion thereof.

A system may be used for studying a TNF-α converting enzyme polypeptide, said system comprising (a) a memory capable of storing information representing at least a portion of a TNF-α, converting enzyme polypeptide, wherein said memory comprises at least one first-type storage region, including a set of spatial coordinates specifying a location in a three dimensional space, and at least one second-type storage region comprising information representing a characteristic of one of a plurality of amino acids, said second-type storage regions being logically associated with said first-type storage regions in said memory to represent a geometric arrangement of at least one characteristic of said at least a portion of said TNF-α converting enzyme peptide in said three dimensional space; (b) a processor coupled to said memory to access said first-type storage regions and said second-type storage regions, wherein the processor generates image signals for depicting a visual image representing three dimensional image of said at least one characteristic of said at least a portion of said TNF-α converting enzyme polypeptide in said three dimensional space based on data from said memory; and (c) a display coupled to said processor to receive said image signals, wherein the display depicts a visual three dimensional image of said at least one characteristic of said at least a portion of said TNF-α converting enzyme polypeptide in said three dimensional space based on said image signals. The image signals may include signals for depicting a visual three dimensional image of a ribbon structure of at least a portion of said TNF-α converting enzyme polypeptide in said three dimensional space. In another embodiment of the invention, the image signals include signals for depicting a visual image of a solid model representation of said at least a portion of said TNF-α converting enzyme polypeptide in said three dimensional space. In still another embodiment of the invention, the image signals include signals for depicting a visual three dimensional image of electrostatic surface potential of said at least a portion of said TNF-α converting enzyme polypeptide in said three dimensional space. In yet another embodiment of the invention, the image signals include signals for depicting a visual three dimensional stereo image of said at least a portion of said TNF-α converting enzyme polypeptide in said three dimensional space. In a further embodiment of the invention, the system further comprises a storage device capable of storing data representing a geometric arrangement of a characteristic of a composition other than said TNF-α converting enzyme polypeptide; and an operator interface for receiving instructions from a operator; and wherein said processor is coupled to said storage device and to said operator interface and generates additional image signals for depicting said geometric arrangement of said characteristic of said composition relative to said visual three dimensional image of said at least one characteristic of said at least a portion of said TNF-α converting enzyme polypeptide on said display based on instructions from the operator interface. In one embodiment, the storage device is part of said memory. In another embodiment, the system comprises a plurality of first-type and second-type storage regions.

According to another aspect of the invention, there is provided a video memory capable of storing information for generating a visual display of at least a portion of a TNF-α converting enzyme polypeptide, said video memory comprising (a) at least one first-type storage region, each of said first-type storage regions including a set of spatial coordinates specifying a location in a three dimensional space; and (b) at least one second-type storage region, each of said second-type storage regions containing information for visually depicting a characteristic of one of a plurality of amino acids; wherein said second-type storage regions are logically associated with said first-type storage regions in said video memory to represent a geometric arrangement of at least one characteristic of said at least a portion of said TNF-α converting enzyme polypeptide in said three dimensional space. The second-type storage regions may be logically associated with said first-type storage regions in said video memory to represent a geometric arrangement of at least one characteristic of a catalytic domain portion of said TNF-α converting enzyme polypeptide in said three dimensional space. The first-type storage regions and said second-type storage regions may be regions of a semiconductor memory. The first-type storage regions and said second-type storage regions may be regions of an optical disk. Alternatively, the first-type storage regions and said second-type storage regions are regions of a magnetic memory. The video memory comprises a plurality of first-type and second-type storage regions.

In an aspect of the invention, there is provided a method of identifying a compound that associates with TNF-α-converting enzyme, comprising the steps according to claim 1. In one embodiment, the associating compound is an inhibitor, mediator, or other compound that regulates TNF-α-converting enzyme activity. In another embodiment, the associating compound is a competitive inhibitor, un-competitive inhibitor, or non-competitive inhibitor. In still another embodiment, the coordinates are the coordinates of Table 1, or a substantial part thereof. The TNF-α-converting enzyme polypeptide crystal comprises the TNF-α-converting enzyme catalytic domain. In still another embodiment, the TNF-α-converting enzyme polypeptide is the expression product of a polynucleotide encoding the pro and catalytic domains of TNF-α-converting enzyme. In yet another embodiment, the TNF-α-converting enzyme polypeptide is the expression product of a polynucleotide encoding the amino acid residues 1-477 of TNF-α-converting enzyme. In another embodiment, the polynucleotide is substituted such that amino acid residue Ser266 is changed to Ala and amino acid residue Asn452 is changed to Gln, and wherein a second polynucleotide encoding the sequence Gly-Ser-(His)₆ is fused to the C-terminus. In a further embodiment, the TNF-α-converting enzyme polypeptide crystal is co-crystallized with a binding partner. In still another embodiment, the binding partner is a hydroxamate-based binding partner or N-{D,L-2-(hydroxyaminocarbonyl)methyl-4-methylpentanoyl}-L-3-amino-2-dimethylbutanoyl-L-alanine,2-(amino)ethyl amide. In yet other embodiments, the TNF-α-converting enzyme polypeptide crystal has a crystal structure diffracting to 2.0 Å, is monoclinic, has a unit cell comprising four crystallographically independent TNF-α-converting enzyme catalytic domain (TCD) molecules, has the TCD molecules are in an asymmetric unit, and/or is of monoclinic space group P2₁ and the cell has the constants a=61.38 Å, b=126.27 Å, c=81.27 Å, and β=107.41°. In still another embodiment, the invention the the associating compound is designed to associate with the S1' region of TNF-α-converting enzyme. In yet another embodiment, the associating compound is designed to associate with the S1'S3' pocket of TNF-α-converting enzyme. In still other embodiments of the invention, the associating compound is designed to (i) incorporate a moiety that chelates zinc, (ii) form a hydrogen bond with Leu348 or Gly349 of TNF-α-converting enzyme, (iii) introduce a non-polar group which occupies the S1' pocket of TNF-α-converting enzyme, (iv) introduce a group which lies within the channel joining S1' - S3' pockets of TNF-α-converting enzyme and which makes appropriate van der Waal contact with the channel, and/or (v) form a hydrogen bond with Leu348 or Gly349 on the backbone amide groups of TNF-α-converting enzyme.

These and other aspects of the invention will become apparent to the skilled artisan in view of the teachings contained herein.

### BRIEF DESCRIPTION OF THE FIGURES

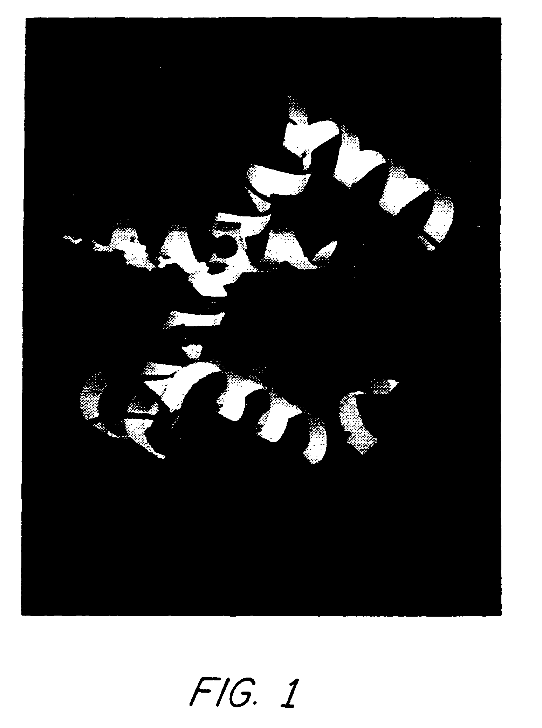

**Fig. 1**: Figure 1 is a ribbon diagram of the TACE catalytic domain (TCD). The chain starts on the lower left back side, runs through the structural elements sI, hAI, hA, sII, hB, hB2, sIII, IV, IVa, sIVb, sV, hC, Met-turn and hD, and ends in the upper left back. The three disulfides are shown as connections, with the sulphurs given as small spheres. The catalytic zinc (central sphere) is liganded by the three imidazoles of His4O5, His4O9 and His415, and by the hydroxyl and the carbonyl oxygen atoms of the inhibitor hydroxamic acid group. The inhibitor mimicking interaction of primed-site residues of a peptide substrate is shown in full. Figure 1 was made using SETOR. See Evans, S. "SETOR: Hardware Lighted Three-Dimensional Solid Model Representations of Macromolecules" J. Mol. Graph. 11:134-138 (1993).

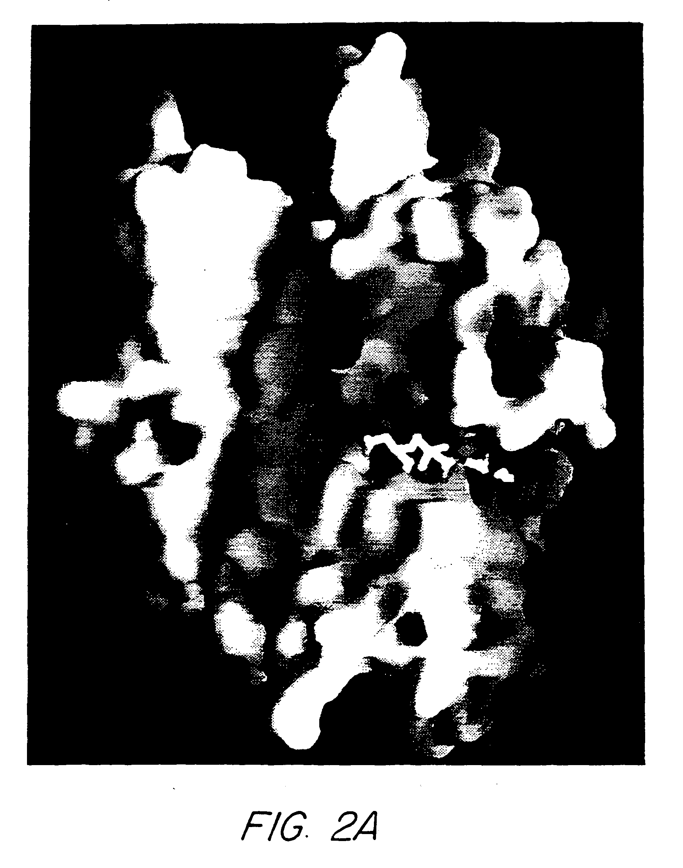

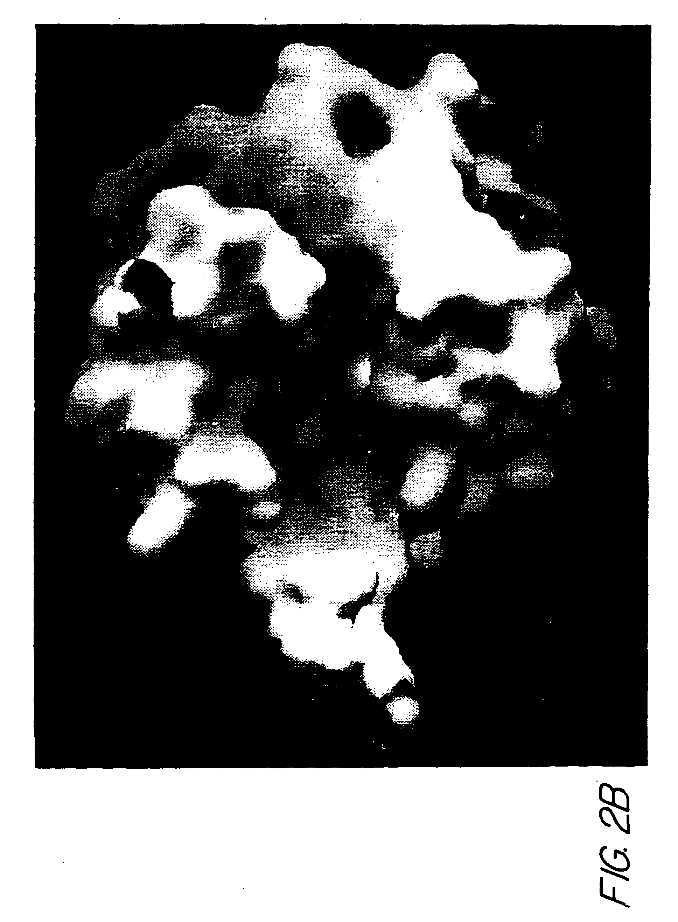

**Figs. 2a and 2b:** Figures 2a and 2b are solid surface representations of the catalytic domains of TACE (TCD) (Figure 2a) and MMP-3 (Figure 2b). The electrostatic surface potential is contoured from -15 (intense red) to 15 (intense blue) k₈T/e. Both active-site clefts run from left to right, with the catalytic zinc atoms (spheres) in the centers. In TACE, the bound inhibitor is shown in full structure, binding with its isobutyl (P1') and its Ala (P3') sidechains into the deep S1' and the novel S3' pockets. The orientation is similar to Fig. 1. Figures 2a and 2b were made using GRASP. Nicolls, A., Bharadwaj, R. and Houig, B., "Grasp - Graphical representation and analysis of surface properties," *Biophys.* 64, A166 (1993).

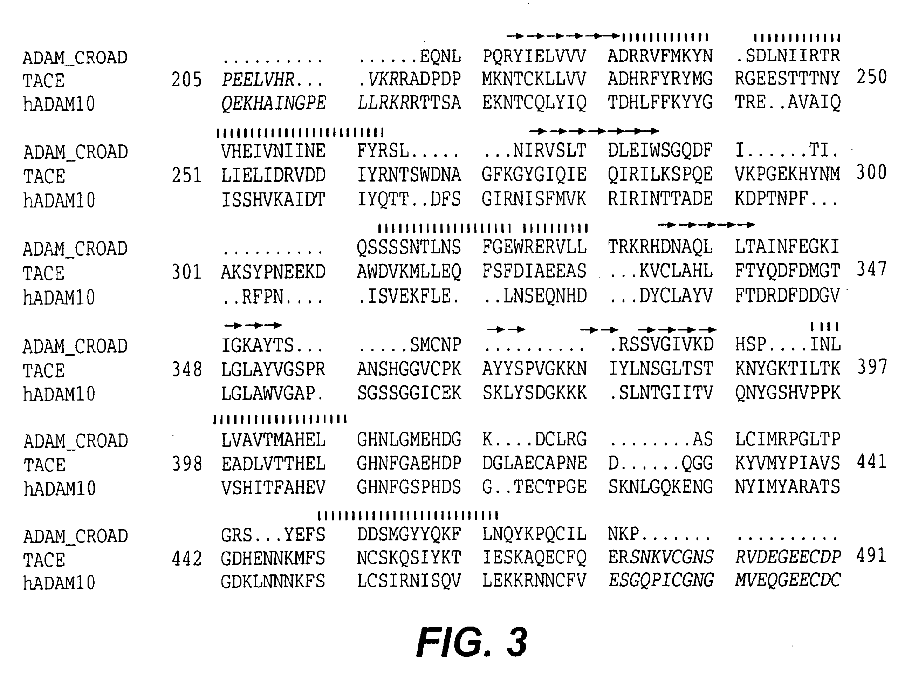

**Fig. 3**:Figure 3 aligns the catalytic domain sequences of adamalysin II (ADAM_CROAD), TACE and human ADAM 10 (hADAMIO), according to their topological equivalence and sequence similarity, respectively. The residue numbers are due to the generic TACE numbering. Arrows and braces represent β-strands and α-helices in TACE.

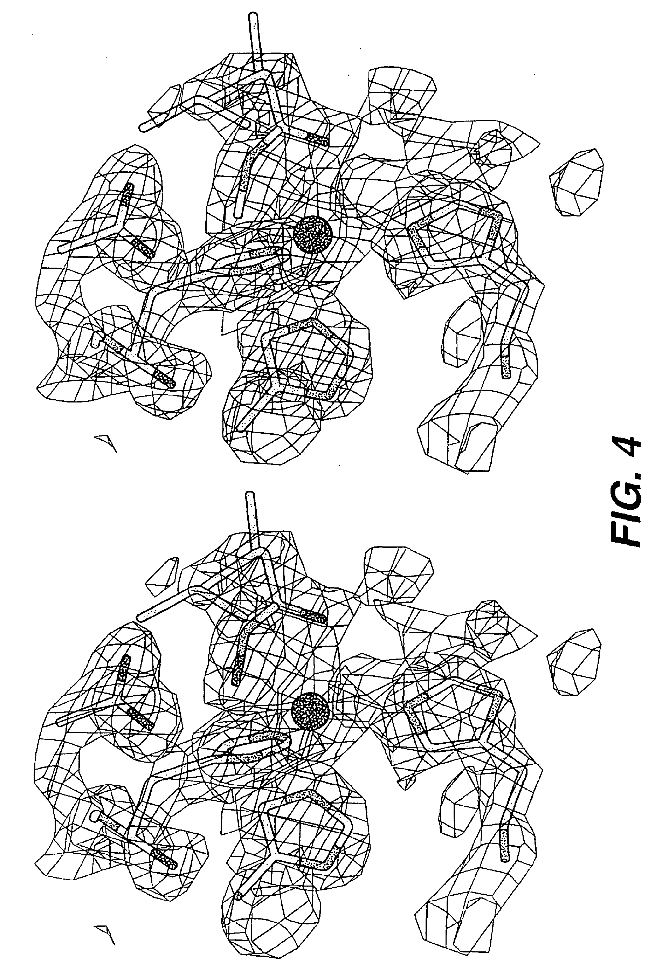

**Fig. 4:** Figure 4 is a stereo section of the final 2.0 Å electron density around the catalytic zinc (large, central sphere) superimposed with the final TACE model. Visible are the three zinc liganding imidazole rings of His4O5 (top), His4O9 (left) and His415 (bottom), the "catalytic" Glu406, and the hydroxamic acid moiety of the inhibitor. The orientation is similar to Fig. 1. Figure 4 was made using TURBO-FRODO. See Roussel, A. & Cambilleau, C., "Turbo-Frodo in Silicon Graphics Geometry," *Partners Directory,* Silicon Graphics, Mountain View, CA (1989).

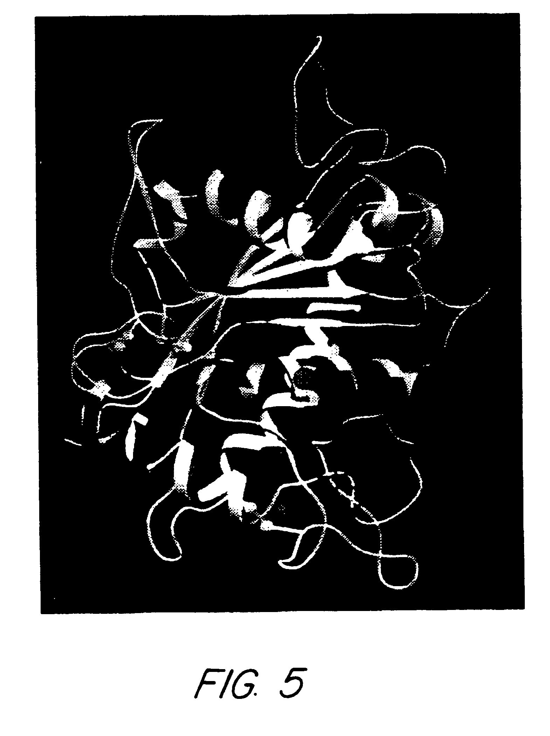

**Fig. 5:** Figure 5 is a superposition of the ribbon plots of the catalytic domain of TACE (light) and adamalysin (dark). Also shown is the catalytic zinc of TACE (sphere) and the three (TACE) and two (adamalysin) disulfide bridges. The orientation is similar to Fig. 1. Figure 5 was made using GRASP.

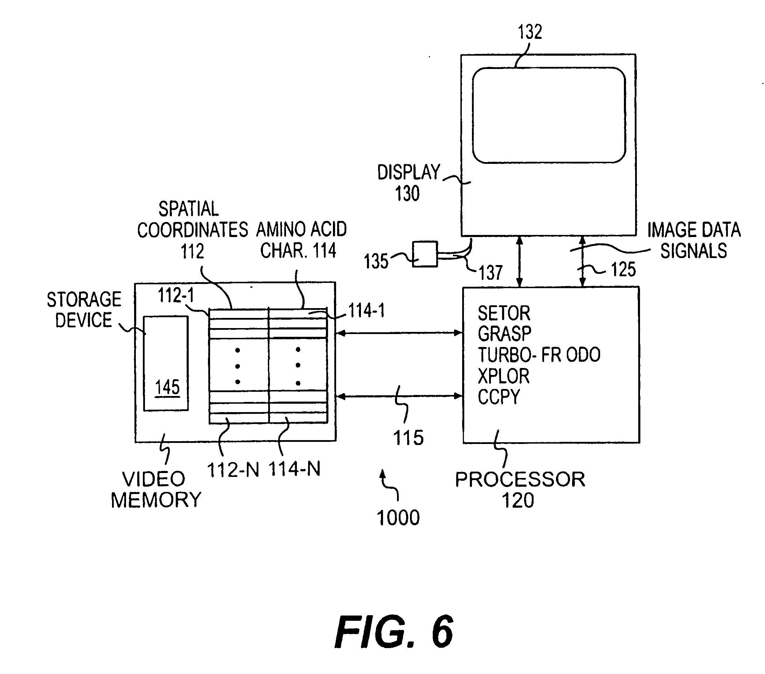

**Fig. 6:** Figure 6 illustrates a system for studying a TNF-α converting enzyme, including a video memory storing information for generating a visual display of at least a portion of a TNF-α converting enzyme.

### DETAILED DESCRIPTION OF THE INVENTION

The present invention relates using the structural coordinates of a TACE polypeptide to elucidate the three-dimensional structure of a TACE polypeptide and designing and developing compounds that associate with TACE. Knowledge of the three-dimensional structure and structure coordinates provided according to the invention permit the skilled person to make compounds that will interact with TACE. Such interacting compounds can be made by a variety of techniques and design criteria, including those disclosed in Protein Engineering (Oxender and Fox, eds.) (Alan R. Liss, Inc. 1987).

As used herein, TACE refers to a group of polypeptides that are capable of converting the 26 kD cell membrane-bound form of TNFα into the soluble 17 kD form that comprises the C-terminal 156 residues of the TNFα protein. TACE encompasses proteins having the amino acid sequence described in PCT application No. WO 96/41624, as well as any of those proteins having homology, preferably no less than 50%, more preferably at least 80% homology, still more preferably 90% homology to such sequence, at the amino acid level. Additionally. TACE further refers to the expression products of nucleotide sequences disclosed in PCT application No. WO 96/41624. TACE further encompasses the membrane-bound protein and soluble or truncated proteins comprising the extracellular portion of the protein and which retain biological activity and are capable of being secreted. Examples of such proteins are described in PCT application No. WO 96/41624.

The TACE amino acid sequence, or any part or residue thereof, can be found in Black et al., "A Metalloproteinase disintigrin that releases tumour-necrosis factor-α from cells," Nature 385: 729-733 (Feb. 1997). Variations in the amino acid sequence of TACE are within the present invention as well. All references to the TACE amino acid sequence contained herein refer to the sequence in Black *et al.,* supra.

As used herein, the TACE catalytic domain (TCD) refers to the portion of a TACE polypeptide between residues 215 and 477 and including the preceding furin cleavage site (residues 211-214), or any part thereof that is capable of cleaving the peptide PLAQAVRSSS.

### Expression. Isolation and Purification of TACE Polypeptides

Tumor necrosis factor-α converting enzyme (TACE) is described in the published PCT application No. WO 96/41624. The application describes isolated nucleic acids encoding TACE or portions of TACE, expression vectors comprising a cDNA encoding TACE or portions thereof, and host cells transformed or transfected with the expression vectors comprising a cDNA encoding TACE or portions of TACE. The application further describes processes for producing TACE and portions thereof, for example by culturing transfected cells engineered to express TACE, followed by purification of the recombinantly produced TACE or portion thereof. Methods of isolating, expressing, and purifying a TACE polypeptide are described in detail in published PCT application No. WO 96/41624.

According to the invention, cDNA encoding the signal peptide, pro and catalytic domains of TACE, *i.e.*, amino acid residues 1-477 is inserted into a suitable expression vector and expressed in a suitable cell line. The cDNA also may include other regions that facilitate expression or achieve other objects that otherwise that do not depart from the essence of the invention, such as flanking regions.

The cDNAs encoding the TACE polypeptide, or functional portions thereof, such as the TCD, may be altered by addition, substitution, deletion, or insertion. Such alterations may be made, for example, to prevent glycosolation, prevent formation of incorrect or undesired disulfide bridges, and/or enhance expression. Examples of such alterations are described in WO 96/41624 and can be carried out by the methods described therein and other conventional methods. TACE may also be conjugated. Such conjugates may comprise peptides added to facilitate purification and/or identification. Such peptides include, for example, poly-His peptides. Conjugation is described in U.S. Patent No. 5,011,912 and Hopp et al.*, Bio*/*Technology* 6:1204 (1988).

In one embodiment of the invention, the cDNA encodes a TNF-α converting enzyme polypeptide comprising the signal peptide, pro and catalytic domains of TACE (TCD), residues 1-477, with Ser266 changed to Ala and Asn452 changed to Gln. These substitutions are useful in preventing N-linked glycosolation. Additionally, the sequence Gly-Ser(His)₆ may be added to the C-terminus. The addition of the sequence Gly-Ser(His)₆ facilitates purification of the polypeptide using metal-chelate affinity resins, such as Ni-NTA resins.

Recombinant expression vectors containing the nucleotide sequence encoding TACE, or a portion thereof, may be prepared using well known methods. Suitable host cells for expression of TACE polypeptides include prokaryotic, yeast, and higher eukaryotic cells. Vectors and host cells suitable for use in the present invention are described in WO 96/41624. Further examples of suitable expression systems that can be employed to express recombinant TACE according to the present invention include mammalian or insect host cell culture expression systems, including baculovirus systems in insect cells (See Luckow and Summers, Bio/Technology 6:47 (1988))and mammalian cell lines such as COS-7 cells (Gluzman et al., Cell 23:175 (1981)). Additional examples are known in the art and include those described in WO 96/41624. In one embodiment of the invention, the TACE polypeptide is expressed in CHO cells. In this embodiment, the cells secrete a mixture of TACE polypeptide beginning with Val212 and Arg215.

In one embodiment, stable expressing cells may be selected by culturing the cells in a drug that kills those cells that do not incorporate the vector. Examples of suitable selection methods are described in, for example, Kaufman, R.J., "Selection and coamplification of heterologous genes in mammalian cells," Methods in Enzymology, 185:537-566 (1990).

Purification of the expressed TACE polypeptide may be carried out by any suitable means, such as those described in WO 96/41624. According to one aspect of the invention, it is preferable to obtain a TACE polypeptide that is suitable for crystallization. In obtaining a TACE polypeptide suitable for crystallization, it is important that the process for purifying the TACE polypeptide is sufficient to yield a polypeptide pure enough to properly crystallize.

A preferred method of purification starts with a suitable amount of medium from the culture of TACE-secreting cells. This medium is generally a supernate of the culture. The medium contains the TACE polypeptide to be purified. Preferably, the TACE polypeptide is recombinantly produced using DNA coding for the TACE polypeptide with the sequence altered to encode a conjugate or conjugates that facilitate purification. For example, the sequence encoding Gly-Ser-(His)₆ may be added to the C-terminus to facilitate purification using metal-chelate resins.

The medium is concentrated, for example, by diafiltration. Suitable diafiltration units include a Millipore 10K cut-off, 1 ft² TFF diafiltration unit. A suitable buffer solution is then added to the concentrated medium. Any suitable buffer may be used. One such suitable buffer contains 20 mM Tris (pH 7.5) and 300 mM NaCl.

The sample is reconcentrated and diluted numerous times. For example, the sample may be reconcentrated and diluted a second time with the buffer, reconcentrated again, diluted a third time with the buffer, and reconcentrated a final time. The sample retained in the diafiltration unit is recovered by a suitable method, such as by a back-flush method. The recovered material may then be filtered through a suitable membrane. Suitable membranes include, for example, 0.45 or 0.22 micron pore-size membranes. Azide is then added. The filtered sample may then be stored overnight at a low temperature, such as about 2-9 °C. After overnight storage, imidazole from a stock solution in water and ZnCl₂ from a stock solution in water are added to the filtered sample. The sample then is pumped over a suitable column. One suitable column, particularly when the TACE polypeptide is conjugated with the sequence Gly-Ser-(His)₆, is a metal-chelate resin, such as a Ni-NTA resin.

The column is washed with a buffer, such as a buffer of 20 mM Tris pH 7.5, 300 mM NaCl, 5 mM imidazole, and 5 uM ZnCl₂. The TACE polypeptide is then eluted with an increasing gradient of imidazole. Fractions are collected in tubes containing glycerol in water Tris pH 8. Preferably, the glycerol solution is prepared the day of the column run.

An aliquot from each fraction is spotted on a membrane which is stained with amido black to determine which fractions contain a significant amount of protein. Alternatively, a small amount, for example 5 µl, from each fraction may be used for gel analysis using Coomassie staining. The fractions with a significant amount of protein are pooled, and the pool is then concentrated with, for example, a diafiltration unit.

In some cases, aggregation of polypeptide may occur. In order to eliminate aggregates and further facilitate purification, an inhibitor of TACE, such as a hydroxamate-based inhibitor, may be added to the concentrated sample from a stock solution in water, and octylglucoside (commercially available from Boehringer Mannheim) is added from a stock solution in water. The sample is then incubated at room temperature for 15-24 hours.

Following incubation, the sample is applied to a size exclusion column. The column is first equilibrated with a suitable buffer, such as a buffer of 10 mM Tris pH 7.5, 100 mM NaCl, 10% glycerol. Suitable size exclusion columns include, for example, LKB 2135-365, packed with TSK-G3000 SWG or the like such as Superdex-200. The buffer is then pumped through the column. The highly purified TACE polypeptide can be detected by absorption at 280 nm.

A gel analysis of all fractions with significant protein is carried out to determine which fractions should be pooled. The size exclusion chromatography pool is concentrated using, for example, a diafiltration unit.

A binding partner, such as an inhibitor, may then be added to the purified sample. The binding partner is particularly useful in stabilizing the TACE polypeptide. The binding partner may be any suitable compound. Suitable binding partners include, for example, hydroxamate-based inhibitors. One suitable inhibitor is N-{D,L-2-(hydroxyaminocarbonyl)methyl-4-methylpentanoyl}-L-3-amino-2-dimethylbutanoyl-L-alanine, 2-(amino)ethyl amide. This inhibitor, as well as other inhibitors, are described in US patent No. 5,594,106 (Black et al.).

The protein complex can be stored at low temperature, for example, at about 4 °C.

### TACE Crystal and Methods of Crystallization of TACE Polypeptides

One aspect of the invention relates to a method of crystallizing a TACE polypeptide. A preferred method comprises co-crystallizing a TACE polypeptide with a binding partner described above. Exemplary means for obtaining the TACE polypeptide, as well as purification of the polypeptide are described above.

Crystals may be grown or formed by any suitable method, including drop vapor diffusion, batch, liquid bridge, and dialysis, and under any suitable conditions. Crystallization by drop vapor diffusion is often preferable. In addition, those of skill in the art will appreciate that the crystallization conditions may be varied. Various methods of crystallizing polypeptides are generally known in the art. See, for example, WO 95/35367, WO 97/15588, EP 646 599 A2, GB 2 306 961 A, and WO 97/08300.

In one embodiment of the invention, a DNA construct comprising TACE residues 1-477, with Ser266 changed to Ala, Asn452 changed to Gln, and the sequence Gly-Ser-(His)₆ added to the C-terminus, may be expressed in CHO cells. These cells primarily secrete a processed mixture of TACE, about half beginning with Val212 and about half with Arg215. The mixture is purified as described above. The purified TACE polypeptide, with the added binding partner, is stored in a buffer as described above.

The TACE polypeptide and binding partner are co-crystallized. The TACE/binding partner solution, at a polypeptide concentration of about 5 mg/mL to about 12 mg/mL in a TACE buffer described above, is mixed with a suitable crystallization buffer and crystallized using a suitable crystallization technique, for example drop vapor diffusion. Suitable crystallization buffers, for example, include: 0.1 M Na Acetate pH 5.3, 0.2 M CaCl₂, 30% v/v Ethanol; 0.1 M Na Citrate pH 5.0, 40% v/v Ethanol; 0.1 M Na Citrate pH 8.7, 20% w/v PEG 4000, 20% v/v Isopropanol; and 0.1 M Na Citrate pH 5.4, 20% w/v PEG 4000, 20% v/v Isopropanol. The sample is incubated at a temperature ranging from about 4 to 20 degrees Celsius. A crystalline precipitate is formed.

Seeds from the crystalline precipitate obtained, as whole crystals or crushed crystal suspensions, are transferred, along with a suitable crystallization promoter, such as hair of rabbit, to a solution of concentrated TACE/substrate in a crystallization buffer. Crystals suitable for X-ray data collection are formed.

One TACE polypeptide crystal comprises a TNF-α converting enzyme catalytic domain (TCD) polypeptide co-crystallized with an inhibitor. The crystal diffracts to about 2 A and belongs to the monoclinic space group P2₁. The crystal's unit cell comprises four crystallographically independent TCD molecules. The TCD molecules are in an asymmetric unit and are not clustered into separate tetrameres, but are integrated into the infinite periodic structure. The crystal has the cell constants: a=61.38 Å (angstrom), b= 126.27 Å, C=81.27 Å and β=107.41°.

### X-Ray Diffraction

Another aspect of the invention relates to the structure of TACE, particularly the structure of the TACE catalytic domain (TCD). The structure of TACE can be determined utilizing a crystal comprising a TACE polypeptide as described above. According to the present invention, the structure of TACE, and particularly the TCD, is determined using X-ray crystallography. Any suitable X-ray diffraction method for obtaining three-dimensional structural coordinates of a polypeptide may be used. The three-dimensional structure coordinates, or any part thereof that characterizes the part of the TACE polypeptide of interest, such as the TACE catalytic domain or part thereof that is capable of cleaving the peptide PLAQAVRSSS. can be used as described herein.

### Methods of Using TACE X-Ray Diffraction Coordinates

The invention also relates to use of the structure coordinates obtained from the above described X-ray diffraction studies of the TACE catalytic domain. The coordinates may be utilized, by direct analysis, with the aide of computers, or combinations thereof, to determine the structure, including secondary and tertiary structure, of the TACE catalytic domain. The TACE catalytic domain structure coordinates also may be used to develop, design, and/or screen compounds that associate with TACE. As used herein, "associate" means that the compound may bind to or interact with TACE ionically, covalently, by hydrogen bond, van der Waals interaction, salt bridges, steric interaction, hydophilic interactions and hydrophobic interaction. Moreover, the term "associate" encompasses associations with any portion of the TACE catalytic domain. For example, compounds that associate with TACE may be compounds that act as competitive inhibitors, un-competitive inhibitors, and non-competitive inhibitors. Compounds that associate with TACE also may be compounds that act as mediators or other regulatory compounds. Compounds that associate with TACE also may be compounds that isomerize to short-lived reaction intermediates in the chemical reaction of substrate with TACE. In particular, compounds designed to associate with TACE may be used therapeutically as inhibitors, mediators and other regulatory compounds.

The use of X-ray coordinates for structure determination, molecular design and selection and synthesis of compounds that associate with other polypeptides is known in the art. Published PCT application WO 95/35367 describes the use of X-ray structure coordinates to design, evaluate, synthesize and use compounds that associate with the active site of an enzyme. UK Patent Application 2306961A describes the use of X-ray coordinates in rational drug design. Published PCT application, WO 97/15588 describes the structural determination of a polypeptide using x-ray diffraction patterns as well as use of the coordinates and three-dimensional structure in finding compounds that associate with the polypeptide of interest. This invention, however, for the first time allows the use of X-ray coordinates for a TACE polypeptide for structural determination, molecular design, and selection and synthesis of compounds that associate with TACE.

In one aspect of the invention, the structure coordinates obtained by the foregoing methods may be displayed as, or converted to, a graphical representation, including three-dimensional shape representations. This may be accomplished using commercially available computer programs capable of generating graphical representations of molecules, or parts thereof, from a set of structural coordinates. Examples of computer programs capable of generating graphical representations of molecules, or parts thereof, from a set of structural coordinates are described in published PCT application WO 97/08300.

The structure coordinates and structure may be compared to, or superimposed over, other similar molecules, such as other metalloproteinases. For example, the TACE structure coordinates and structure may be compared to or superimposed over the structure coordinates or structure of snake venom metalloproteinases, such as, for example, adamalysin II. The TACE structure coordinates and structure also may be compared to or superimposed over the structure coordinates or structure of matrix metalloproteinases, such as ADAM 10, including human ADAM 10. Comparison of TACE and other molecules for which a graphical structure or three-dimensional structural coordinates are available may be carried out with the aide of available software applications, such as the Molecular Similarity application of QUANTA (Molecular Simulations, Inc., Waltham, MA).

Compounds that associate with TACE also may be computationally evaluated and designed by screening and selecting chemical entities or fragments for their ability to associate with TACE, and specifically the TACE catalytic domain. Several methods may be used to accomplish this aspect of the invention. One may visually inspect a computer-generated model of TACE, and specifically the catalytic domain, based on the structure coordinates described herein. Computer generated models of chemical entities or specific chemical moieties can then be positioned in or around the catalytic domain and evaluated based on energy minimization and molecular dynamics, using, for example, available programs such as CHARMM or AMBER. Positioning of the chemical entity or fragment can be accomplished, for example with docking software such as Quanta and Sybyl. Additionally, known and commercially available computer programs may be used in selecting chemical entities or fragments. Once suitable chemical entities or fragments are selected, they may be assembled into a single compound, such as an inhibitor, mediator, or other regulatory compound. Known and commercially available model building software may assist in assembly.

Compounds that associate with TACE and specifically the TACE catalytic domain may be designed as a whole, rather than by assembly of specific chemical moieties or chemical entities. This embodiment may be carried out using computer programs such as LUDI (Biosym Technologies, San Diego, CA), LEGEND (Molecular Simulations, Burlington, MA), and Leap Frog (Tripos Associates, St. Louis, MO).

A candidate compound may be chosen based upon the desired sites of interaction with TACE and the candidate compound in light of the sites of interaction identified previously. Once the specific candidate compound-TACE interactions are determined, docking studies, using commercially available docking software, are performed to provide preliminary "modeled" complexes of selected candidate compound with TACE.

Constrained conformational analysis is performed using, for example, molecular dynamics (MD) to check the integrity of the modeled TACE-inhibitor complex. Once the complex reaches its most favorable conformational state, the structure as proposed by the MD study is analyzed visually to ensure that the modeled complex complies with known experimental SAR/QSAR (structure-activity relationship/quantitative structure-activity relationship) based on measured binding affinities.

Other modeling techniques also may be used in accordance with the invention. Examples of these techniques are disclosed in Cohen et al., "Molecular Modeling Software and Methods for Medicinal Chemistry," J. Med. Chem., 33:883-894 (1990) and Navia et al., "The Use of Structural Information in Drug Design," Current Opinions in Structural Biology, 2:202-210 (1992).

Compounds developed or designed to associate with TACE may be optimized or the efficiency of association can be tested using a number of methods known in the art. For example, the deformation energy and electrostatic interactions may be determined and optimized. Known and commercially available software and hardware systems may be used. Examples of such software are disclosed in WO 95/07619. Structure-based analoging for optimization of the inhibitor potency, selectivity and physical drug-like properties in an iterative manner also may be performed by one skilled in the art of drug design.

Substitutions also may be made to selected or designed compounds. These substitutions can be made to improve or modify the association properties of the compound. Such substitutions may be made, for example, in side groups or particular atoms of the compounds. Generally, one should begin with conservative substitutions that have approximately the same size, shape, charge and other characteristics of the original group or atom. Substituted compounds may be further analyzed and optimized as described above.

In a further aspect of the invention, the potential inhibitory, mediatory, regulatory, or other binding effect of a compound may be analyzed and evaluated, using, for example, commercially available computer software, prior to actual synthesis and testing of such compound. In this way, one can evaluate the probability of synthesizing and testing of inoperative compounds.

Procedures for measuring inhibition generally are known in the art and are disclosed, for example, in PCT 96/41624. Such methods include assays based on reaction with a peptide substrate.

### TACE Catalytic Domain Structure

The physical features of the TCD, determined based on the X-ray diffraction data obtained using the methods described and its use in creating molecular models of the TCD, are further described, with reference to the Figures.

The domain depicted in Fig. 1 has the shape of an oblate ellipsoid, notched at its flat side to give a relatively small active-site cleft separating the small "lower" subdomain from the "upper" main molecular body (Fig. 2a). The TCD polypeptide chain starts on the molecular surface (in the lower back, Fig. 1), with the chain becoming well defined between Asp217 and Met221 (see Fig. 3). Central to the molecule is the five-stranded β-pleated sheet, with the β-strands arranged in the order (from back to front, see Fig 1) sII, sI, sIII, sV and sIV (see Fig. 3), with sIV, the "edge" strand, running antiparallel to the others. This β-sheet is highly twisted flanked by two α-helices (hB and hB2) on its convex and two helices (hA and hC) on its concave side. The β-strands sI and sII are connected by the short α-helix hA1 and the long α-helix hA (the obliquely running helix on the backside, Fig. 1). The β-strands sII and sIII are linked by the large "multiple-turn loop", the long "intermediate" α-helix hB and the adjacent short α-helix hB2, all of them arranged on "top" of the β-sheet thus fully shielding its central part from bulk water (Fig. 1). The multiple-turn loop is bulged out at two sites giving rise to a "spur-like" and a quite acidic protuberance, respectively (visible in Fig. 2a on top of the molecule). The sIII-sIV linker terminates in a short "bulge", before it enters the edge strand sIV. The sIV-sV connecting segment is dissected into two large "ear-like" surface-located loops, a first one nestling to the main molecular body (giving rise to the "blue" surface, center left, in Fig. 2a), and a long β-hairpin loop (sIIa-sIIb) projecting from the molecular surface (top left in Figs. 1 and 2). A bulged-out loop links sV with the "active-site helix" hC, which is located in the center of the molecule and stops abruptly at the strictly conserved Gly412, where the chain kinks down to build the lower subdomain.

The C-terminal chain comprising the last 61 TCD residues (Fig. 3) first forms three short straight almost perpendicularly arranged segments linked by two "narrow" supertwisted loops, returns via the tight "Met-turn" Tyr433-Val434-Met435-Tyr436 back to the surface where it kinks at Pro437 to form the Pro437-Ile438- Ala439 outer "wall" of the S1' crevice, approaches in a wide loop the C-terminal α-helix hD and runs through it, and ends up on the molecular "back" surface close to the N-terminus, with the last defined residues Arg473-Ser474 fixed via hydrogen bonds to the main molecular body. Via Cys423-Cys453, the first of the two "narrow" loops is disulfide-linked with the N-terminus of helix hD, whose C-terminal end in turn is clamped to the "ear-like" sIV-sV linker peptide through Cys365-Cys469. Spatially adjacent, the third disulfide bridge of TCD, Cys225-Cys333, connects the N-terminal parts of β-strands sI and sIII. In the intact TACE molecule, four residues downstream of Ser474 would reside Cys478, which is already integral part of the compact elongated disintegrin domain (Saudek et al. , "Three-dimensional structure of echistatin, the smallest active RGD protein" Biochem. 30, 7369-7372 (1991)). Considering Ser474 and this Cys478 as pivot points of their respective domains, the three residue linker would allow relatively unconstrained docking of the disintegrin domain to the "left" surface side of the catalytic domain.

The active-site cleft of TACE (Fig. 2a) is relatively flat on the left hand (non-primed) side, but becomes notched towards the right. The catalytic zinc residing in its center is penta-coordinated by the three imidazole N∈2 atoms of His4O5, His4O9 and His415 (provided by the active-site helix and the following "descending" chain comprising the conserved zinc binding consensus motif HEXXHXXGXXH), and by the carbonyl and the hydroxyl oxygen of the hydroxamic acid moiety of the inhibiter (see Figs. 1, 2a and 4). This zinc-imidazole ensemble is based on the distal ε-methyl-sulphur moiety of the strictly conserved Met435, harbored in the Met-turn characteristic for the metzincin clan (Bode et al., "Astacins. serralysins, snake venom and matrix metalloproteinases exhibit identical zinc binding environments (HEXXHXXGXXH and Met-turn) and topologies and should be grouped into a common family, the 'metzincins"' FEBS Lett. 331, 134-140 (1993); Stöcker et al., "The metzincins: Topological and sequential relations between the astacins, adamalysins, serralysins, and matrixins (collagenases) define a superfamily of zinc-peptidases" Protein Sci. 4, 823-840 (1995)). Both carboxylate oxygens of the "catalytic" Glu4O6 (which acts as a general base during catalysis (Grams et al., "X-ray structures of human neutrophil collagenase complexed with peptide hydroxamate and peptide thiol inhibitors: Implications for substrate binding and rational drug design" Eur. J. Biochem. 228, 830-841 (1995)) squeezed between the zinc-liganding imidazole of His4O5 and the edge strand, are hydrogen bonded to the hydroxyl and the N-H group of the hydroxamic acid (see Fig.4). To the right of the catalytic zinc opens the deep S1' pocket, which, besides the S1' wall-forming segment (bottom, Figs. 1 and 2a), is bordered by the side chains of His4O5 and Glu4O6 (left), the sIV main chain and the Leu345 side chain (top), and the side chains of Val44O (back) and Ala439 (right). To the right of Ala439 opens a second (S3') pocket, which inside the molecule merges with the S1' pocket, leaving a small bridge made of the opposing side chains of Ala439 and Leu348 (Fig. 2a).

The (pseudo)peptidic part of the inhibitor binds in an extended geometry to the notched right-hand side of the active-site cleft, mimicking the interaction of the primed residues of a productively bound peptide substrate (Fig. 2a). It runs antiparallel to the upper short bulge Gly346-Thr347-Leu348 and parallel to the S1' wall-forming segment Pro437-Ile438-Ala439, making two and two inter-main chain hydrogen bonds, respectively. The dominant intermolecular interactions are made by the P1' isobutyl (pseudo-leucyl) side chain of the inhibitor and the essentially hydrophobic S1' pocket, however, is large and accommodates three partially ordered solvent molecules in addition. The P2' t-butyl side chain extends away from the enzyme, but nestles to the hydrophobic canopy above formed by the enzyme's bulge. The P3' Ala side chain points into the large negatively charged S3' pocket, but is too short to make favorable contacts. The C-terminal diaminoethyl group has different conformations in the four molecules.

The P1' to P3' segment Val77-Arg78-Ser79 of a bound pro-TNFα probably binds in a similar manner, possibly under better matching with the underlying cleft surface; the preceding P3 to P1 residues Ala74-Gln75-Ala76 certainly will align antiparallel to the edge strand, with their side chains extending into the (partially charged) S3 pocket and the (negatively charged) shallow S2 depression, and projecting out of the central cleft, respectively. The primed subsites and surrounding molecular surfaces of TACE are dominated by negative charges, while the non-primed subsites are essentially hydrophobic in nature (Fig. 2a). More distant interactions may be involved in the specificity of TACE for processing pro-TNFα. The 12 residue substrate comprising the pro-TNFα cleavage site can also be split by some of the MMPs, although with less specificity and efficacy (Black et al., "Relaxed specificity of matrix metalloproteinases (MMPs) and TIMP intensity of tumor necrosis factor-α (TNF-α) production suggest the major TNF-α converting enzyme is not an MMP" Biochem. Biophys. Res. Commun. 225, 400-405 (1996)). Thus, the preferential processing of the (probably trimeric) (Tang et al., "Human pro-tumor necrosis factor is a homotrimer" Biochem. 35, 8216-8225 (1996a); Tang et al., "Length of the linking domain of human pro-tumor necrosis factor determines the cleavage processing" Biochem. 35, 8226-8233 (1996b)) membrane-bound pro-TNFα in *vivo* might in part be due to correct assembling, i.e. suitable presentation of the pro-TNFα cleavage segment to the TACE active site in a distinct distance from the anchoring membrane. Some experimental evidence (Tang et al., Biochem. 35, 8216-8225 (1996a); Tang et al., Biochem. 35, 8226-8233 (1996b)) suggests that the cleavage site might not be determined by the cleavage sequence alone, but that also the distance to the base of the compact cone formed by the associated C-terminal segments of three TNFα molecules (Jones et al., "Structure of tumor necrosis factor" Nature 338, 225-228 (1989)) plays a role. In a productive TACE-proTNFα complex, the base of this TNFα-trimer cone (into which the disordered N-termini run up) may be recognized by the "right" side of the TACE catalytic domain (Fig. 2a), with the about 10 residues long spacer favoring the correct placement of the proTNFα Ala76-Val77 scissile peptide bond in the active site of TACE.

The polypeptide topology and in particular the surface presentation of the catalytic zinc prove the catalytic domain of TACE to be a typical metzincin. (Bode et al., "Astacins, serralysins, snake venom and matmrix metalloproteinases exhibit identical zinc binding environments (HEXXHXXGXXH and Met-turn) and topologies and should be grouped into a common family, the 'metzincins'" FEBS lett. 331, 134-140 (1993); Stöcker et al., "The metzincins: Topological and sequential relations between the astacins, adamalysins, serralysins, and matrixins (collagenases) define a superfamily of zinc-peptidases" Protein Sci. 4, 823-840 (1995)) A superposition with the other metzincins shows, however, that its topology is most similar to that of the catalytic domain of snake venom metalloproteinases such as adamalysin II (Fig. 5). (Gomis-Rüth et al., "First structure of a snake venom metalloproteinase: prototype for matrix metalloproteinases/collagenases" EMBO J. 12, 4151-4157 (1993); Zhang et al., "Structural interaction of natural and synthetic inhibitors with the venom metalloproteinase, atrolysin C (form d)" Proc. Natl. Acad. Sci. USA 91, 8447-8451 (1994); Kumasaka et al., "Crystal structure of H2-proteinase from the venom of Trimeresurus flavoviridis" J. Biochem. 119, 49-57 (1996)) This close homology is reflected by the much better simultaneous superposition of the central sheet and the large helices, but in particularly also by a couple of structural features, which TACE shares exclusively with the adamalysins such as: the long helix hB and the preceding multiple-turn loop arranged on top of the β-sheet; the typically arranged and shaped C-terminal helix hC; and the extended C-terminus placed on the backside surface. About 175 of the 263 TACE and 201 adamalysin α-atoms are topologically equivalent (with an rms deviation of 1.3 Å, 39 of which have identical side chains (Fig. 3). These numbers are close to those obtained from a comparison of members within the different metzincin families. (Stocker *et al., supra*) In addition, detailed structural features prove the close relationship of TACE to the adamalysins: a more conserved core structure; the loosely arranged N-terminus; the characteristic Asp416 (directly following the zinc binding consensus motif, Fig. 3) involved in identical intramolecular hydrogen bond interactions; the adjacent disulfide bridge Cys423-Cys453 linking the first narrow loop to the C-terminal helix hD (which TACE does not share with adamalysin II, but with the H2-proteinase from the snake venom of T. flavoviridis) (Kumasaka *et al., supra*); disulfide bridge Cys365-Cys469 connecting the sIV-sV linker with the C-terminal helix hD; a similarly shaped active-site cleft, with particularly strong similarities in the SI' pocket and other primed subsites.

The catalytic domain of TACE (TCD) also differs from adamalysin II in several respects: with 263 residues, its chain is much longer; most of the additional residues of TACE are clustered giving rise to a more projecting hA-sII turn, to the two surface protuberances of the multiple-turn loop, to the two "ears" of the sIV-sV linker, and to a more bulged-out sV-hC connector (see Figs. 3 and 5); lack of a calcium binding site but presence of a third disulfide bridge Cys225-Cys333 in TACE, both elements serving, however, for the same function namely to clamp the N- terminal chain to strand sill; the quite deep S3' pocket of TACE which merges with its S1' pocket; an almost inverted charge pattern in and around the primed subsites with an absolute predominance of positive charges in adamalysin.

According to its sequence, and probably with respect to its three-dimensional structure, the TACE catalytic domain is thus not a typical member of the mammalian ADAMs proper (a family of membrane-anchored cell-surface proteins, with the catalytic domain quite homologous to adamalysin (Wolfsberg et al., "ADAMs in Fertilization and Development" Developm. Biol. 18C, 389-401 (1996))) TACE presumably shares this "outsider" role with (bovine) ADAM 10 (Fig. 3), which does also possess some TACE-like activity (Lunn et al., "Purification of ADAM 10 from bovine spleen as a TNFα convertase," FEBS Lett. 400, 333-335 (1997)), and whose *Drosophila* version *(kuz)* has recently been shown to process the Notch receptor (Rooke et al., Science 273, 1227-1231 (1996)). Also ADAM 10 probably exhibits an elongated hA-sII loop and the two "ears" typical for TACE, but might have a multiple-turn intermediate in size between TACE and adamalysin (see Fig. 3). Ninety of the ADAM 10 catalytic domain residues are identical to TACE further underlining the close homology (see Fig. 3), whereas the other mammalian ADAMs probably resemble much more adamalysin II (Gomis-Ruth et al., "Refined 2.0 A crystal structure of snake venom zinc endopeptidase adamalysin II" J. Mol. Biol. 239, 513-544 (1994)).

The structural homology of TACE to the MMPs is significantly lower. The relative arrangement of the common secondary structural elements differs more (reflected by the significantly larger rms deviation of 1.6 Å of the about 120 topologically equivalent Cα-atoms), and the MMPs lack characteristic TACE/adamalysin structural elements (such as the intermediate helix hB and the multiple-turn loop, the Asp residue behind the third zinc-binding histidine), or exhibit typical determinants (such as the structural zinc and the integrated calcium ions) not seen in TACE. Notwithstanding the differences in secondary structure, the active-site cleft of TACE bears some similarity with that of the MMPs, with the flat nonprimed (left-hand) side, and the narrow primed side centering in the deep S1' pocket (Fig. 2b). This subsite similarity to the MMPs explains the observed partial sensitivity of TNFα-convertase activity towards synthetic hydroxamic acid inhibitors originally designed for inhibition of various MMPs (DiMartino et al., "Anti-arthritic activity of hydroxamic acid-based pseudopeptide inhibitors of matrix metalloproteinases and TNFα processing" Inflamm. Res. 46, 211-215 (1997)). Model building experiments with TIMP-1 structure (Gomis-Rüth et al., "Mechanism of inhibition of the human matrix metalloproteinase stromelysin-1 by TIMP-1" Nature 389, 77-81(1997)) show no obvious obstacles in the active-site region of TACE that would easily explain its resistance to blockage by the TIMPs.

This TCD crystal structure thus gives evidence for a topological similarity of the catalytic domain of TACE with that of the adamalysins/ADAMs, and for a share of its substrate binding site to that of the MMPs. TACE exhibits, however, several structural peculiarities regarding surface contour, charge and shape, which facilitates the design of potent selective synthetic inhibitors.

In designing and developing compounds, such as inhibitors, mediators and other compounds having activities with biological significance, that associate with TACE, it is desirable to select compounds with a view toward the particular surface contour, charge, shape, and other physical characteristics of the TACE catalytic domain. Generally, the compounds should be capable of physically and structurally associating with TACE, as well as be able to assume a conformation that allows it to associate with TACE. The features described above will direct the skilled artisan in this regard. In particular, compounds with a linear functionality should be particularly suitable. Such compounds will be particularly suitable in light of the deep pockets of the TACE catalytic domain.

The compounds that associate with TACE, for example, may be designed to associate with the S1' region or the S1'S3' pocket of TACE. Compounds that associate with TACE also may be designed to (i) incorporate a moiety that chelates zinc. Further exemplary compounds include compounds are designed to form a hydrogen bond with Leu348 or Gly349 of TACE. (ii) introduce a non-polar group which occupies the S1' pocket of TACE. (iii) introduce a group which lies within the channel joining S1' - S3' pockets of TACE and which makes appropriate van der Waal contact with the channel, and (iv) form a hydrogen bond with Leu348 or Gly349 on the backbone amide groups of TNF-α-converting enzyme, or (v) any combination of the above.

### Computer-Readable Medium

A computer-readable medium may have recorded thereon the x-ray diffraction structure coordinates of a crystalline TACE polypeptide. The computer-readable media of the invention are useful for storage, transfer, and use with software of the TACE structural coordinates. The computer readable medium may be any suitable data storage material, including, but not limited to, a floppy disc, a hard disc, computer-type Random Access Memory, Read-Only Memory flash memory, CD-ROM, recordable and rewritable CDs, recordable and rewritable DVDs, magnetic-optical disk, ZIP drive, JAZ drive, Syquist drive, digital tape drive, or the like. Other suitable media will be known to those of skill in the art.

The computer readable medium may comprise the coordinates of Table 1 or a substantial portion thereof. The computer-readable medium may be used in conjunction with a machine programmed with instructions for using the data recorded on the medium, such as a computer loaded with one or more programs identified throughout the specification, to display a graphical, three-dimensional representation of a TACE polypeptide, or any part thereof.

### Computer Based System

Figure 6 illustrates a system 1000 for studying a TACE polypeptide. The system includes a video memory 110 that stores information representing at least a portion of a TACE polypeptide. The memory has at least one first-type storage region 112, having recorded thereon a set of spatial coordinates specifying a location in a three dimensional space, and at least one second-type storage region 114, having recording thereon information representing a characteristic of one of a plurality of amino acids. The second-type storage regions are logically associated with the first-type storage regions in the video memory 110 to represent a geometric arrangement of at least one characteristic of at least a portion of the TACE polypeptide in the three dimensional space. Memory, 112 and 114 can comprise, for example, the data shown in Table 1. The system 1000 also includes a processor, coupled to the memory to access the first-type storage regions 112 and the second-type storage regions 114, to generate image signals for depicting a visual three dimensional image of at least one characteristic of at least a portion of the TACE polypeptide in the three dimensional space based on data from the memory 110. The processor can be any general purpose processor with a CPU, register, memory and the like. A display 130 coupled to the processor 120 via lines 125 to receive the image signals, for depicting a visual three dimensional image of at least one characteristic of at least a portion of the TACE polypeptide in the three dimensional space based on the image data on a screen 132.

The image data may include data for depicting a visual three dimensional image of a ribbon structure of at least a portion of a TACE polypeptide in three dimensional space, such as shown in Fig. 1. Further, the image data may include data for depicting a visual three dimensional image of a solid model representation of at least a portion of said TACE polypeptide in three dimensional space, such as shown in Fig. 2. Still further, the image data may include data for depicting a visual three dimensional image of electrostatic surface potential of at least a portion of TACE polypeptide in three dimensional space, such as shown in Fig. 2. Alternatively, the image data may include data for depicting a visual three dimensional stereo image of at least a portion of a TACE polypeptide in three dimensional space, such as shown in fig. 4.

The system 1000 of the present invention may further comprise a storage device 145 that stores data representing a geometric arrangement of a characteristic of a composition other than the TACE polypeptide and an operator interface, such as a mouse 135, for receiving instructions from a operator. Storage device 145 can include, for example, the three-dimensional X-ray coordinate data for other chemical entities. The processor 120 is coupled to the storage device 145 and to said operator interface 135 and generates additional image data for depicting the geometric arrangement of the characteristic of the composition relative to said visual three dimensional image of said at least one characteristic of said at least a portion of TACE polypeptide on the screen 132 based on instructions from the operator interface. In the Fig. 6 embodiment, the storage device 145 is part of the memory 110.

The first-type storage regions 112 and said second-type storage regions 114 are regions of, for example, a semiconductor memory, regions of an optical disk, or regions of a magnetic memory.

In one embodiment, processor 120 and video memory 110 are in the form of a UNIX or VAX computer, such as those available from Silicon Graphics, Sun Microsystems, and IBM. However, the invention is not limited to use of this particular hardware and software.

The invention is described in more detail in the following illustrative examples. Although the examples may represent only selected embodiments of the invention, it should be understood that the following examples are illustrative and not limiting.

### Example 1 - TACE Polypeptide Expression, Isolation, and Purification

A cDNA encoding the signal peptide, pro and catalytic domains of TACE, amino acid residues 1-477, as disclosed in Black et al., "A Metalloproteinase disintigrin that releases tumour-necrosis factor-α from cells," Nature 385: 729-733 (Feb. 1997), with Ser266 changed to Ala, Asn452 changed to Gln and the sequence Gly-Ser-(His)₆ added to the C-terminus, was inserted into an expression vector for CHO cells. The TACE polypeptide was expressed in CHO cells and a mixture of the TACE polypeptide beginning either with Val212 or Arg215 was secreted. The cells were cultured in the drug, methotrexate, which kills those cells that did not incorporate the vector.

The expressed TACE polypeptide was then purified. Purification started with 5 liters of the medium containing the expressed TACE polypeptide. The medium was concentrated to about 200 mL with a Millipore 10K cut-off, 1 ft² TFF diafiltration unit. The pumping rate was 50-100 mL/min. Two liters of a buffer solution of 20 mM Tris (pH 7.5) and 300 mM NaCl (Buffer E) was then added to the sample.

The sample was reconcentrated as described above and diluted a second time with 2 liters of Buffer E, reconcentrated again, diluted a third time with 2 liters of Buffer E, and reconcentrated to about 100 mL. The sample retained in the diafiltration unit was recovered by a back-flush. This material was then filtered through a 0.45 µm and was azide added to 0.05%. The filtered sample was stored overnight at 4 °C.

After overnight storage, imidazole was added to the filtered sample to 5 mM from a 200 mM stock in water and ZnCl₂ was added to 5 uM from a 1 M stock in water. The sample then was pumped over 2.2 mL of Qiagen Ni-NTA Superflow resin (Cat. # 30430) at 3 mL/min (column size 7.5 x 50 mm).

The column was washed at 5 mL/min with 100 mL of a buffer of 20 mM Tris pH 7.5, 300 mM NaCl, 5 mM imidazole, and 5 uM ZnCl₂ (Buffer A). The protein was then eluted with an increasing gradient of imidazole, going up to 200 mM in 1 minute (5 mL total volume), followed by 35 mL of 200 mM imidazole in Buffer A. Two mL fractions were collected, TACE generally coming off about 6 mL into the elution. The fractions were collected in tubes containing 500 ul of 50% glycerol in water and 200 ul of 1 M Tris pH 8. The glycerol in water was prepared the day of the column run.

A dot blot, with 3 µl from each fraction, was stained with amido black to determine which fractions contained a significant amount of protein. The fractions with a significant amount of protein were pooled. The pool was then concentrated to 1-2 mL with a 10 K cut-off Amicon Centriprep concentrator.

The inhibitor N-{D,L-2-(hydroxyaminocarbonyl)methyl-4-methylpentanoyl}-L-3-amino-2-dimethylbutanoyl-L-alanine, 2-(amino)ethyl amide was added to the concentrated sample to 1 mM from a 50 mM stock in water, and octylglucoside was added to 1% from a 10 % stock in water. The sample was then incubated at room temperature for 15-24 hours.

Following incubation, the sample was applied to a 21.5 x 600 mm size exclusion column, LKB 2135-365, packed with TSK-G3000 SWG, and equilibrated with 10 mM Tris pH 7.5, 100 mM NaCl, 10% glycerol. This buffer was then pumped through the column at 2.5 mL/min for 100 minutes. The TACE polypeptide in the effluent was detected by absorption at 280 nm. Excluded material generally eluted at about 38 minutes. The pure TACE generally eluted at about 78 minutes or longer.

A gel analysis, with 15 µl of all fractions with significant protein was then carried out to determine which fractions should be pooled. The size-exclusion chromatography pool was concentrated to about 1 mL with a 10 K cut-off Amicon Centriprep concentrator.

The inhibitor N-{D,L-2-(hydroxyaminocarbonyl)methyl-4-methylpentanoyl}-L-3-amino-2-dimethylbutanoyl-L-alanine, 2-(amino)ethyl amide was then added to the purified sample to a concentration of 1 mM. The protein can be stored at 4 °C.

### Example 2 - Protein Crystallization

A DNA construct comprising the prodomain and the catalytic domain of human TACE (resides 1-477) was fused to the sequence Gly-Ser-(His)₆ to facilitate purification of the protein on a Ni-NTA affinity column. Chinese Hamster Ovary (CHO) were cells used for protein expression. The cells secreted a mixture of mature TACE beginning with either Val212 or Arg215. TACE-containing fractions from the Ni-NTA column were incubated in a buffer containing octylglucoside and the binding partner N-[D,L-[2-(hydroxyaminocarnbonyl)methyl]-4-methyl-pentanoyl)-L-3-(tert-butyl)-glycyl-L-alanine. The final purification step was performed on a gel filtration column. Purified TACE was stored in a buffer containing 10 mM Tris/HCL pH 7.5, 100 mM NaCl, 10% glycerol and 1 mM of inhibitor (TACE buffer).

Crystallization experiments were set up at a TACE concentration of approximately 5 mg/mL by mixing TACE (in TACE buffer) in a 1:1 ratio with the crystallization buffers listed below and using the sitting drop vapor diffusion technique. The experiments were performed in duplicate and incubated either at about 4°C or at 20°C. Crystalline precipitate was obtained at 20°C in the following crystallization buffers:

| | |
|---|---|
| Buffer A) | 0.1 M Na Acetate pH 5.3, 0.2 M CaCl₂, 30% v/v Ethanol |
| Buffer B) | 0.1 M Na Citrate pH 5.0, 40% v/v Ethanol |
| Buffer C) | 0.1 M Na Citrate pH 8.7, 20% w/v PEG 4000, 20% v/v Isopropanol |

Small crystals were obtained upon transferring seeds from the crystalline precipitate with a hair of a rabbit into a 1:1 mixture of a concentrated sample of TACE (12 mg/mL in TACE buffer) with either buffer B or C. Further refinement of buffer C resulted in buffer D, which allowed the production of crystals suitable for X-ray data collection.

| | |
|---|---|
| Buffer D) | 0.1 M Na Citrate pH 5.4, 20% w/v PEG 4000, 20% v/v Isopropanol |

The first data set was measured to a reduction of 2.5 Å on a MAR300 imaging plate scanner attached to a Rigaku-Denki totaling Cu-anode generator operated at 5.4 kW providing graphite-monochromatized CuKα radiation. The data were processed with MOSFLM v. 5.23 program and routines of the CCP4 suite. All attempts to solve the structure by molecular replacement methods using either adamalysin II, an all-alanine model of adamalysin II and models generated failed to produce useful starting points for phasing. Thus the locations of four independent zinc atoms were determined with the help of an anomalous difference Patterson synthesis. In order to measure MAD data, the crystals were deep-frozen in liquid nitrogen. Therefore, crystals were transferred into a cryo buffer (80% v/v buffer D containing 17 % v/v glycerol) with the help of a silk loop of appropriate size, soaked for about 10 seconds and then immediately deep-frozen at 90 degrees K.

The crystals obtained belong to the monoclinic space group P2₁, have cell constants a = 61.38 Å (angstrom), b = 126.27 Å, c = 81.27 Å, β = 107.41°, and contain four molecules in the asymmetric unit.

### Example 3 - X-ray Diffraction

Using the crystals described in Example 2, a first data set was measured to a resolution of 2.5 Å on a MAR300 imaging plate scanner attached to a Rigaku-Denki rotating Cu-anode generator operated at 5.4kW providing graphite-monochromatized CuKα radiation. The data were processed at with the MOSFLM v. 5.23 program and routines of the CCP4 suite.

All attempts to solve the structure by molecular replacement methods using either adamalysin II, an all-alanine model of adamalysin II, and other models failed to produce useful starting points for phasing.

Thus, the locations of the four independent zinc atoms were determined with the help of an anomalous difference Patterson synthesis. In order to measure MAD data, the crystals were deep-frozen in a nitrogen gas stream cooled down to the temperature of liquid nitrogen. The crystals were first transferred into a cryo-buffer of 80% v/v Buffer D (0.1 M Na Citrate pH 5.4, 20% w/v PEG 4000, 20% v/v Isopropanol) containing 17 % v/v glycerol. Transfer to the cryo-buffer was performed with the help of a silk loop of appropriate size. The crystals were soaked in the cryo-buffer for about 10 seconds and then immediately deep-frozen at 90 K.

Anomalous diffraction data to 2.0 Å were collected with MAR345 imaging plate scanner at 90 K on the BW6 wiggler beamline of DORIS (DESY, Hamburg, Germany), using monochromatic X-ray radiation at the wavelengths of maximal f" (1.2769 Å) and minimal f" (1.2776 Å) at the K absorption edge of zinc and at a remote wavelength (1.060 Å). The data were scanned and evaluated using DENZO/SCALEPACK, yielding 77653 independent reflections from 1051836 measurements (96.9% completeness, R-merge 0.031 in intensities).

MAD phases were refined and calculated with MLPHARE including all measured data to 2.0 Å resolution. Their initial mean-figure-of-merit of 0.53 was increased to 0.76 by solvent flattening/histogram matching methods applying DM. This density allowed building of the complete chains of the four independent TACE catalytic domains and the bound hydroxamic acid substrates on an SGI system using TURBO-FRODO. This model was crystallographically refined with XPLOR and with CCP4 routines to a crystallographic R factor of 18.6% (R_{free} 27.4%) using 79400 independent reflections from, 12.0 to 2A. resolution.

Four independent TACE molecules form the periodic arrangement.

Molecules 1 and 2, and 3 and 4 are defined from Asp219 and Met221, respectively, to Ser474.

### Example 4 - X-ray Diffraction

Anomalous dispersion diffraction data to 2.0 Å were collected with a MAR345 imaging plate scanner at 100 K on the wiggler beamline of DORIS (DESY, Hamburg, Germany), using monochromatic X-ray radiation of maximal f' (1.2797 Å) and minimal f (1.2804 Å) at the K absorption edge of zinc and at a remote wavelength (1.060 Å). These data were evaluated and scanned using DENZO/SCALEPACK, yielding 77,653 independent reflections (96.9% completeness, R-merge 0.031).

The structure coordinates obtained are reproduced in Table 1.

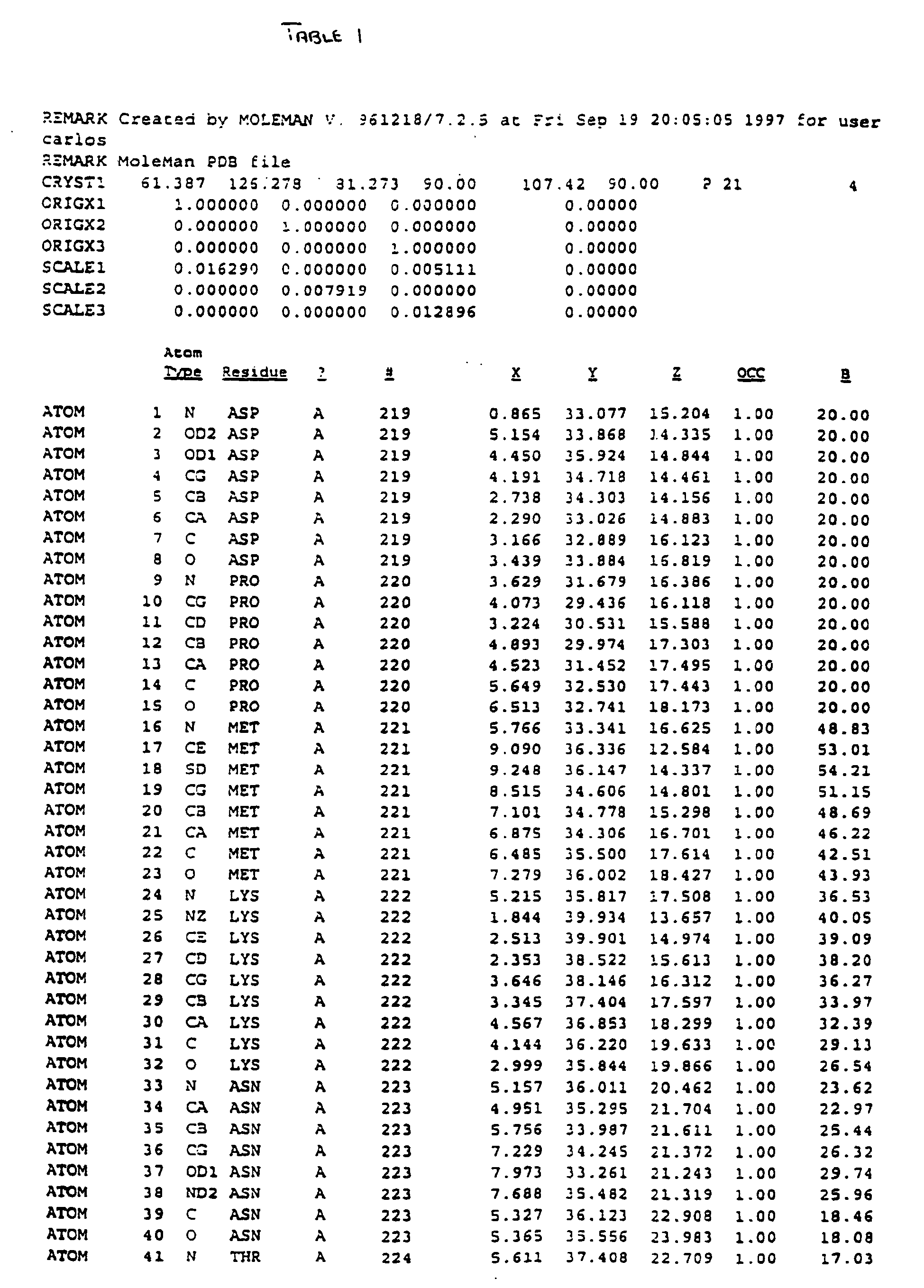

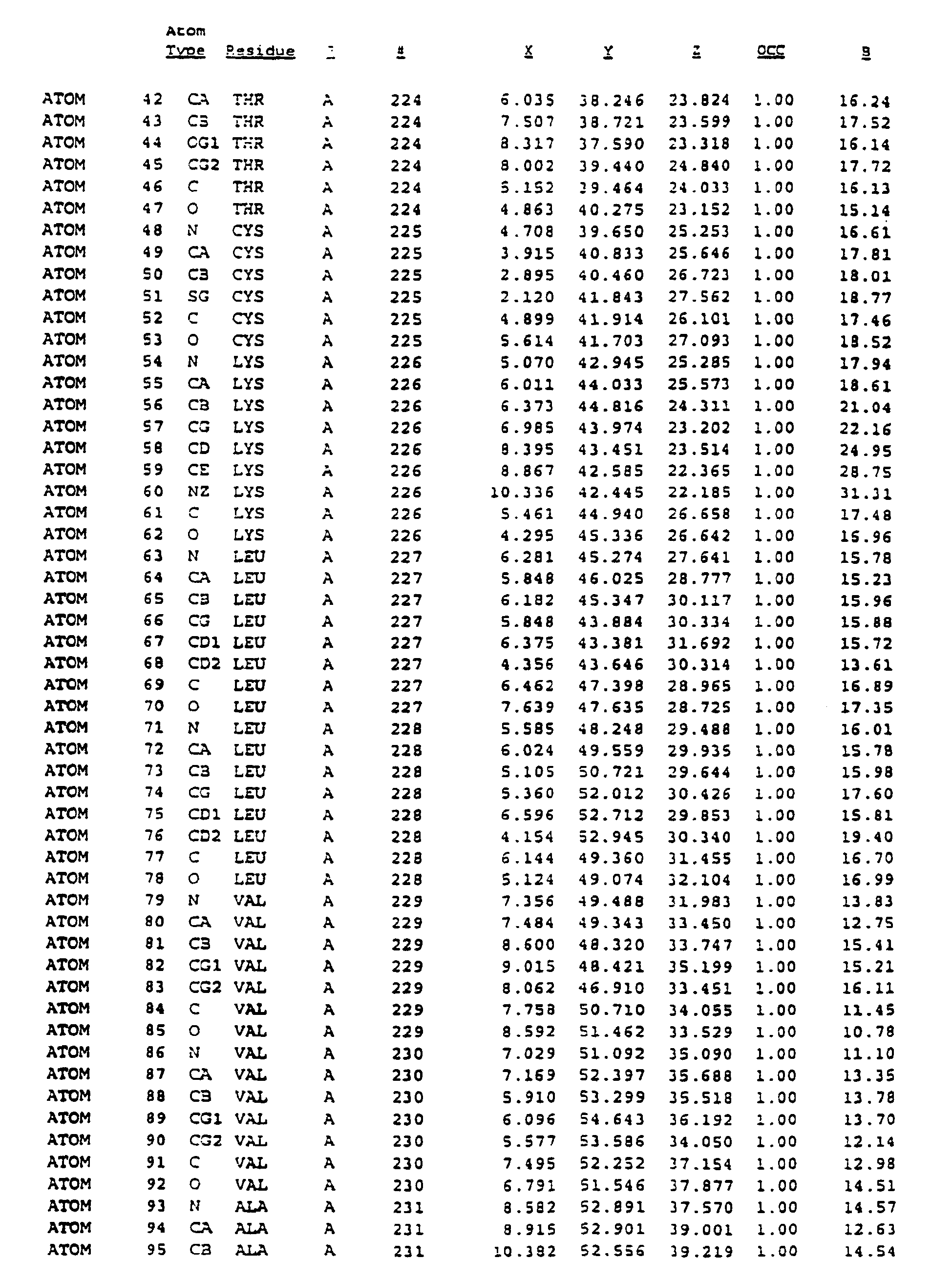

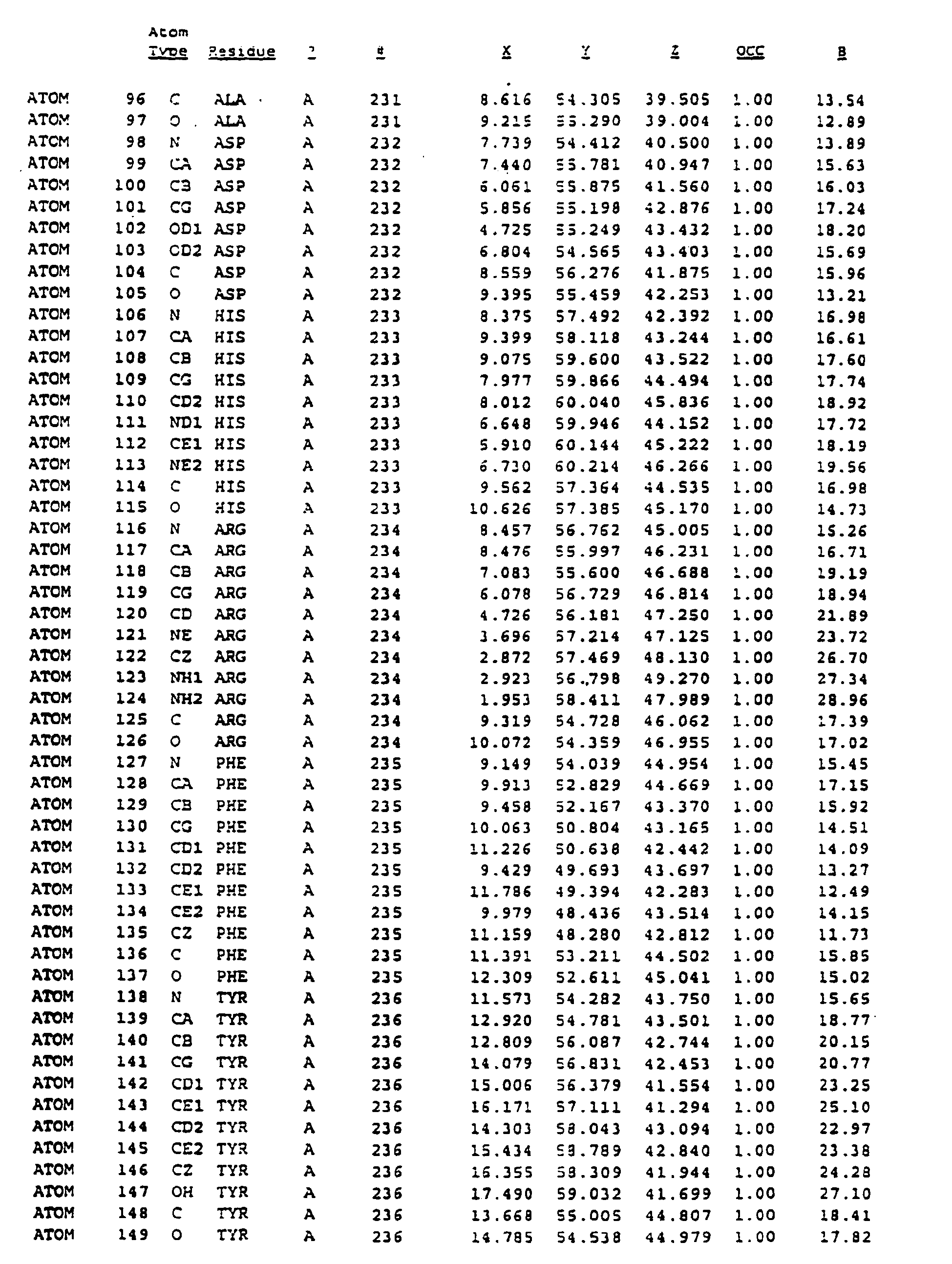

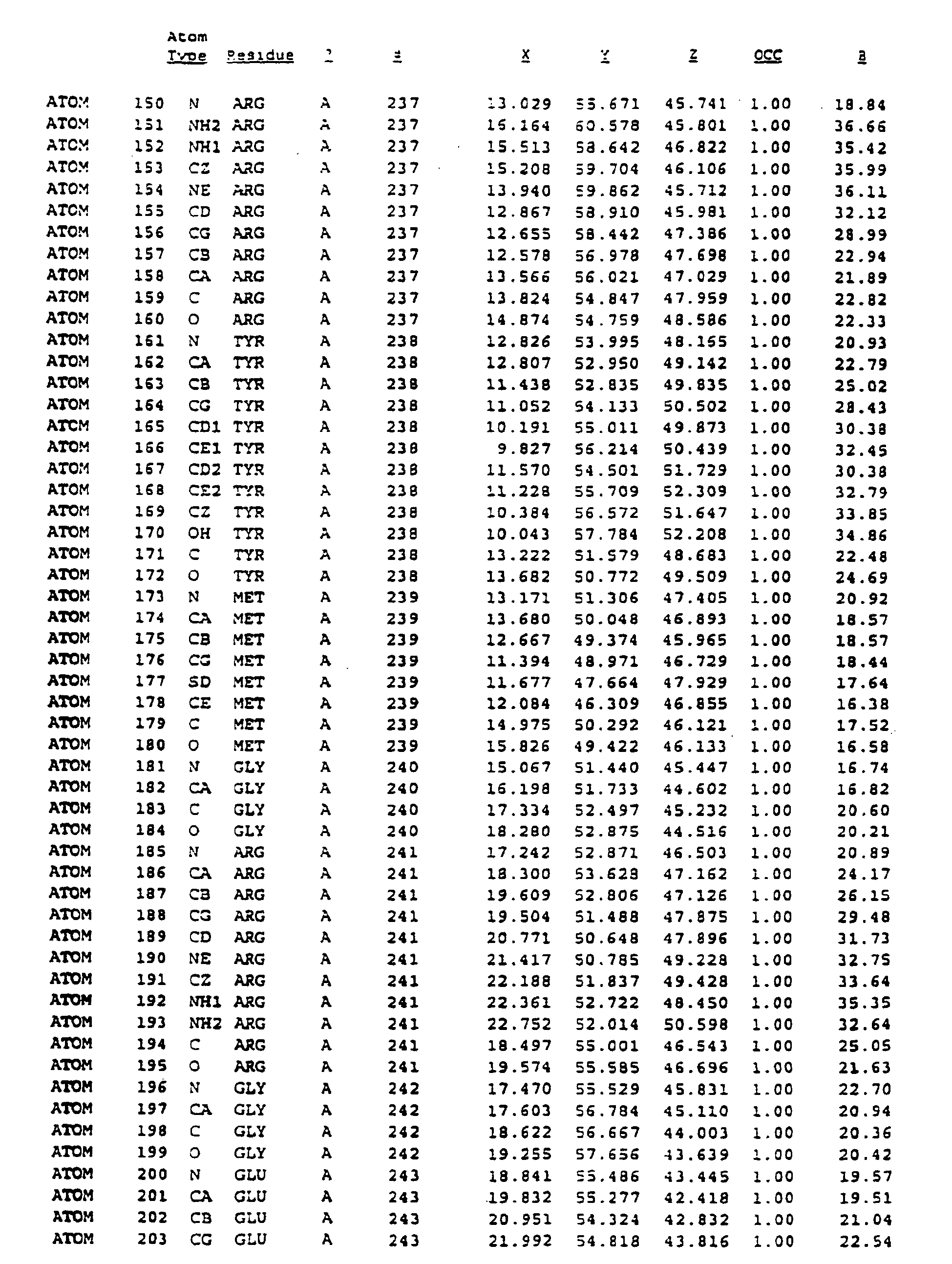

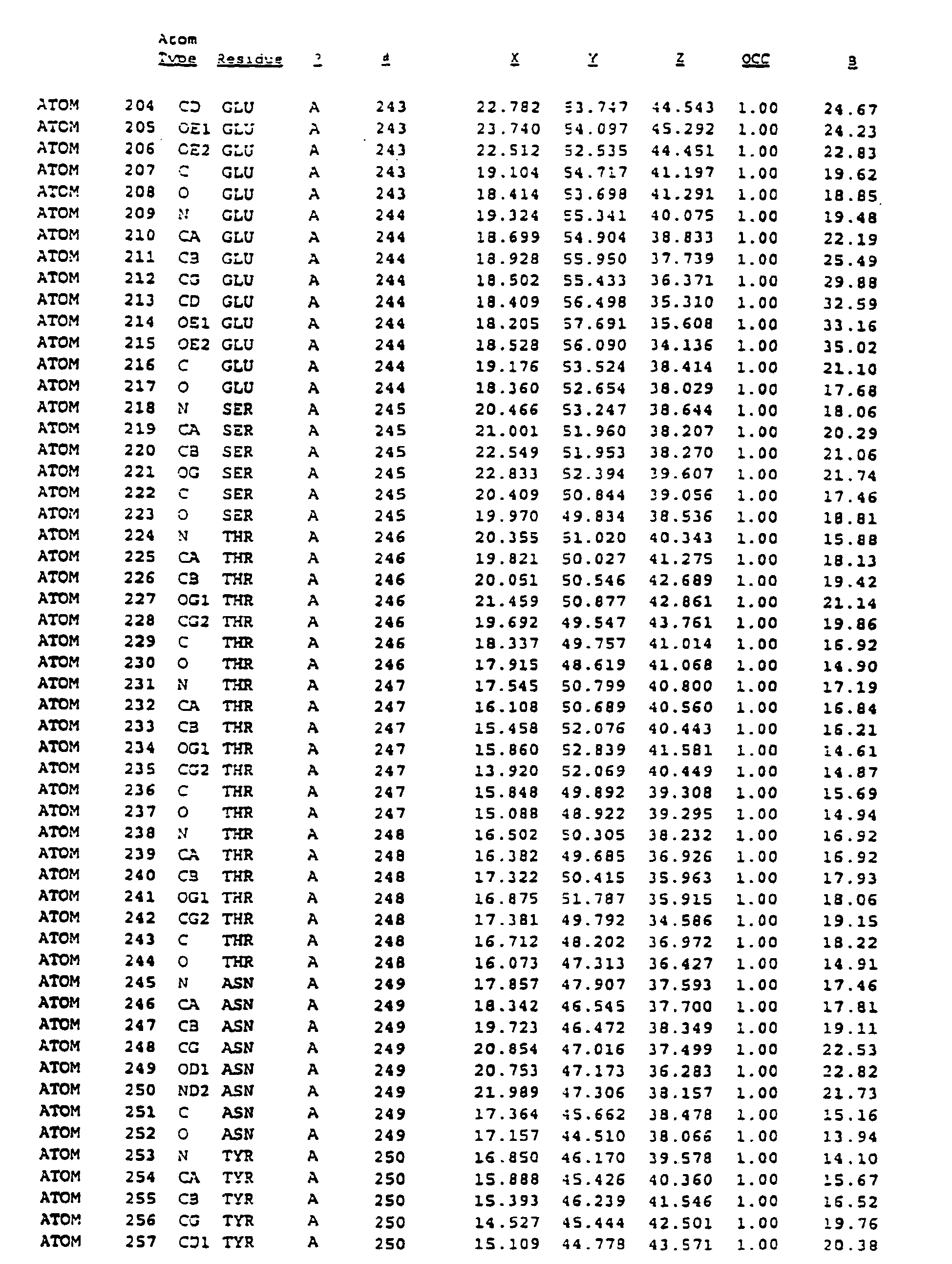

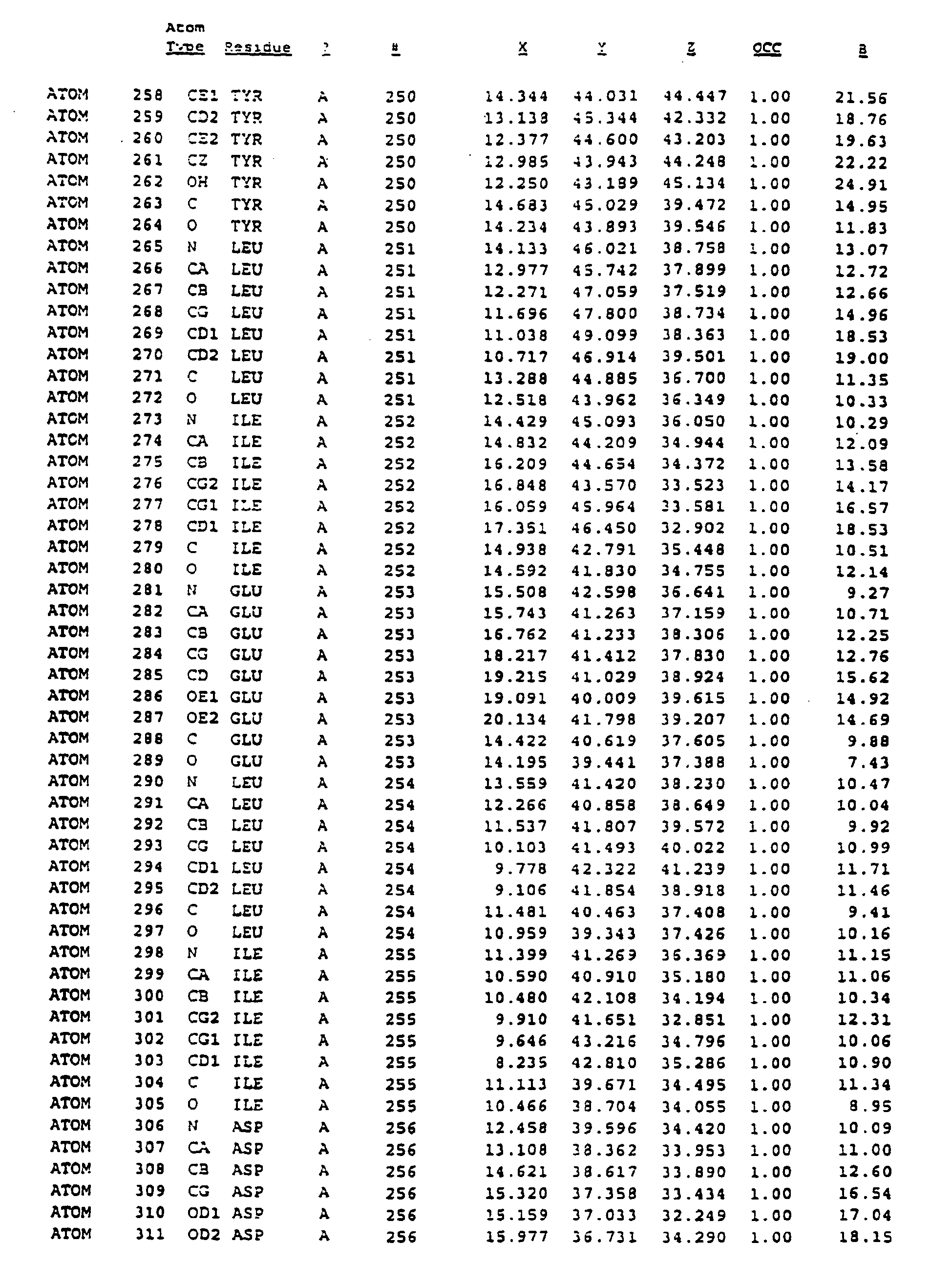

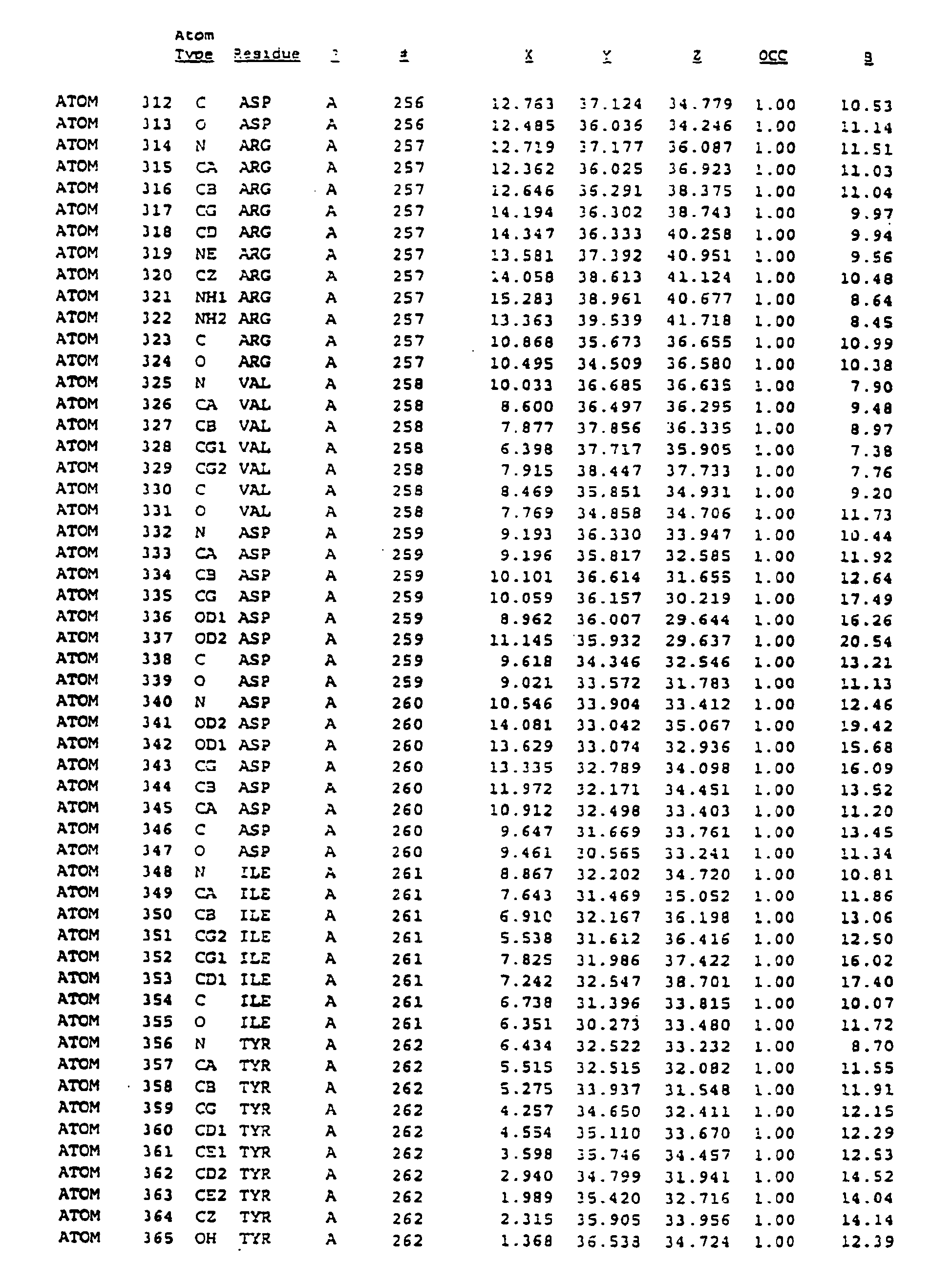

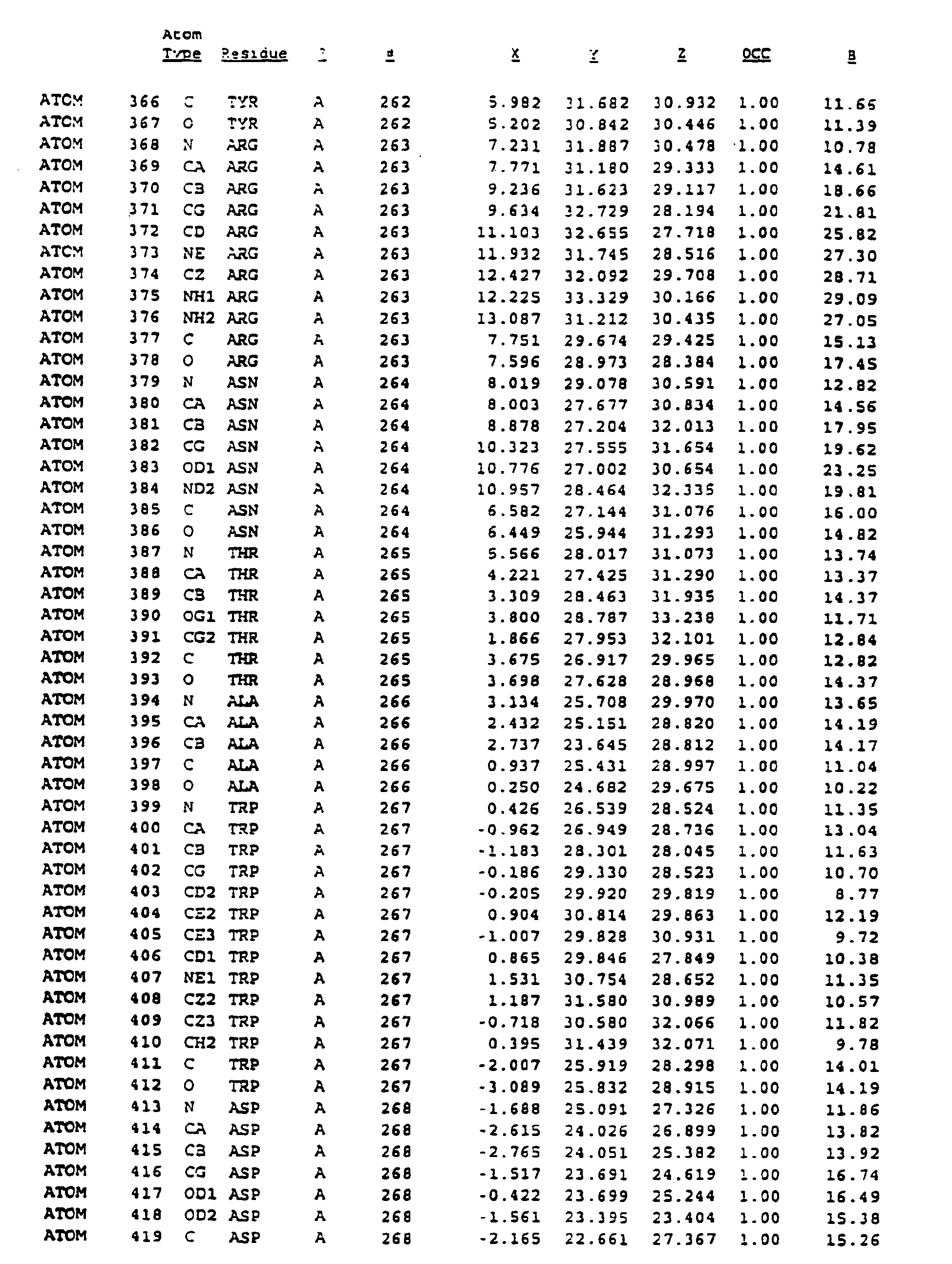

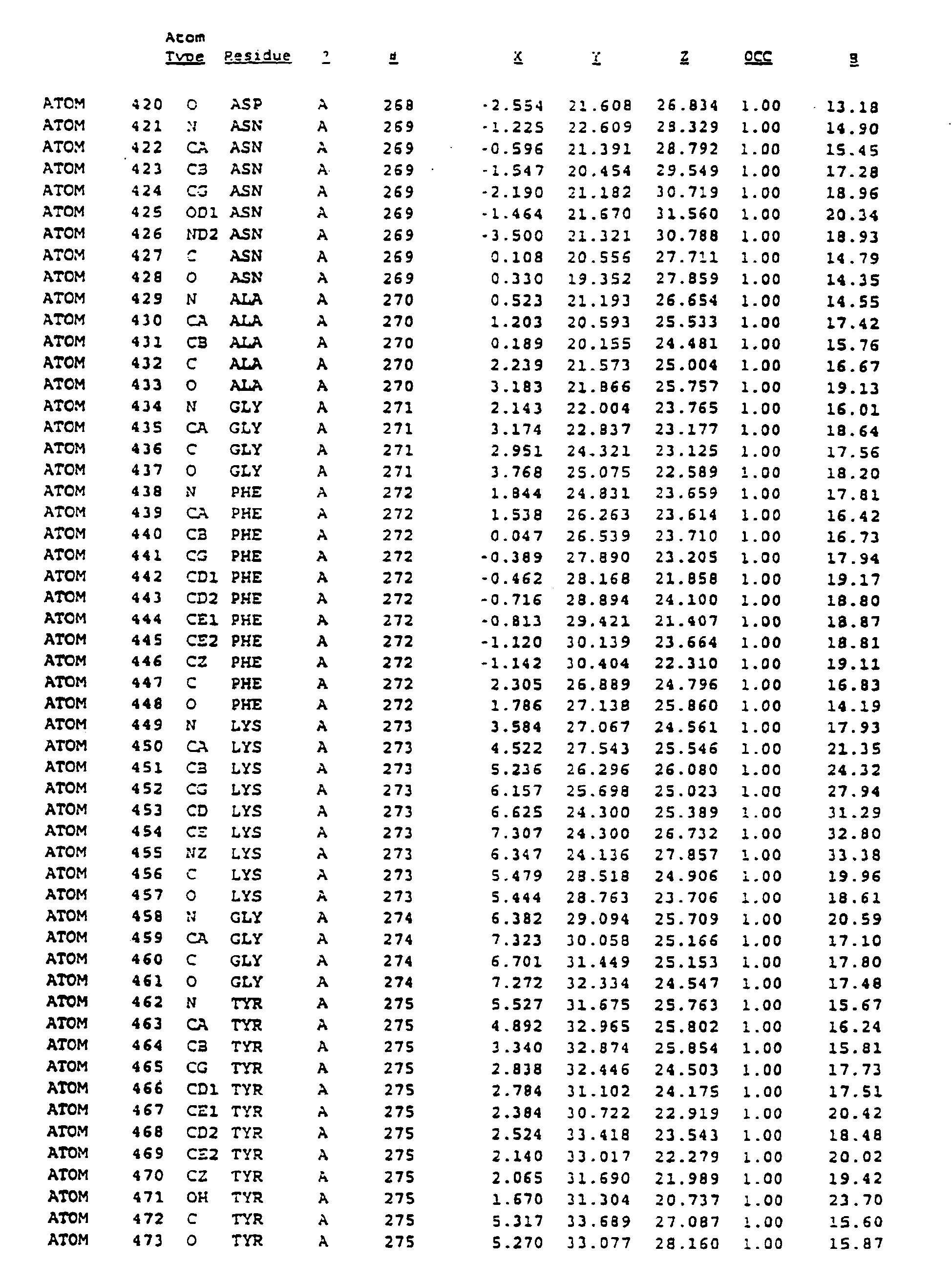

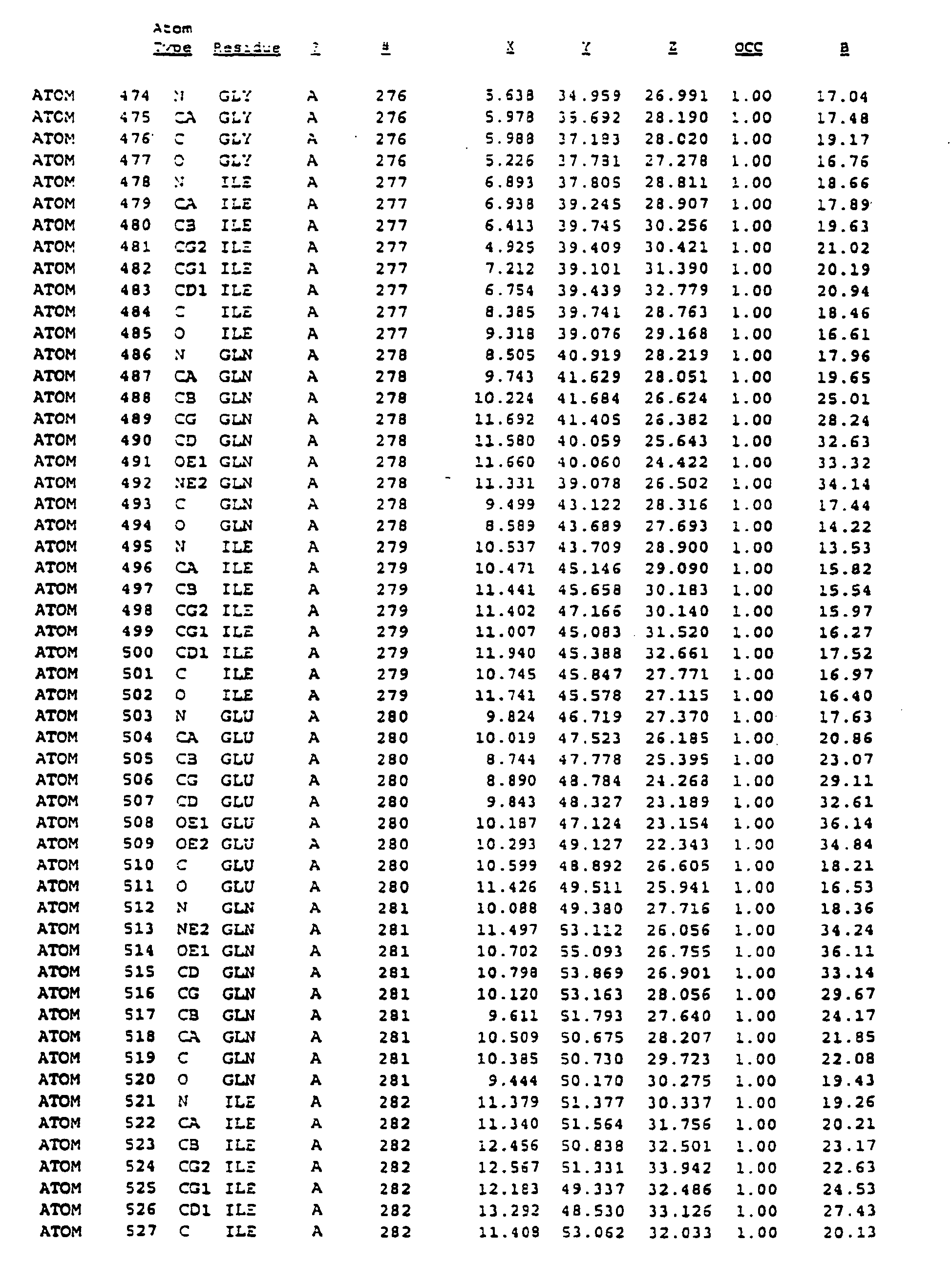

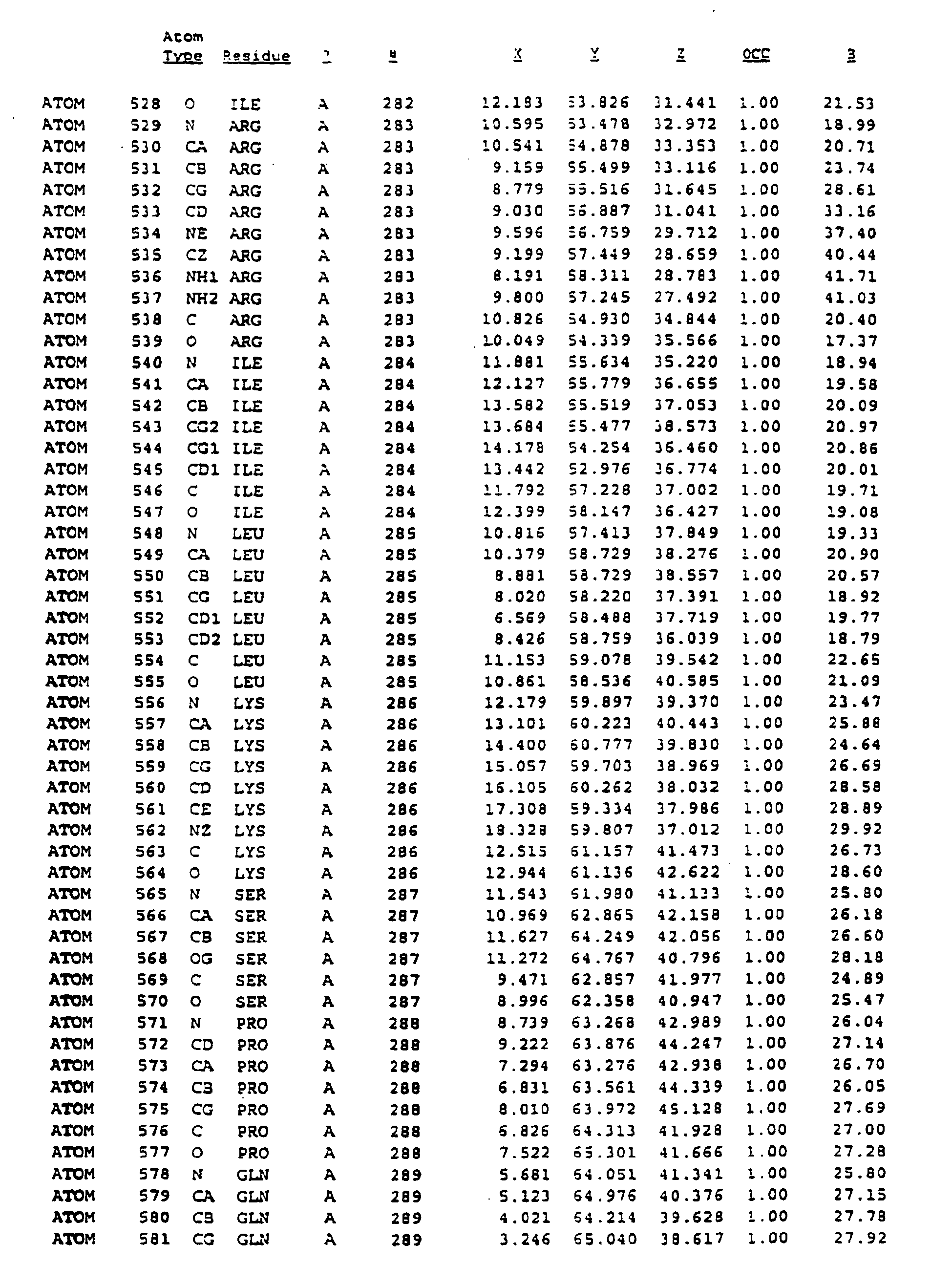

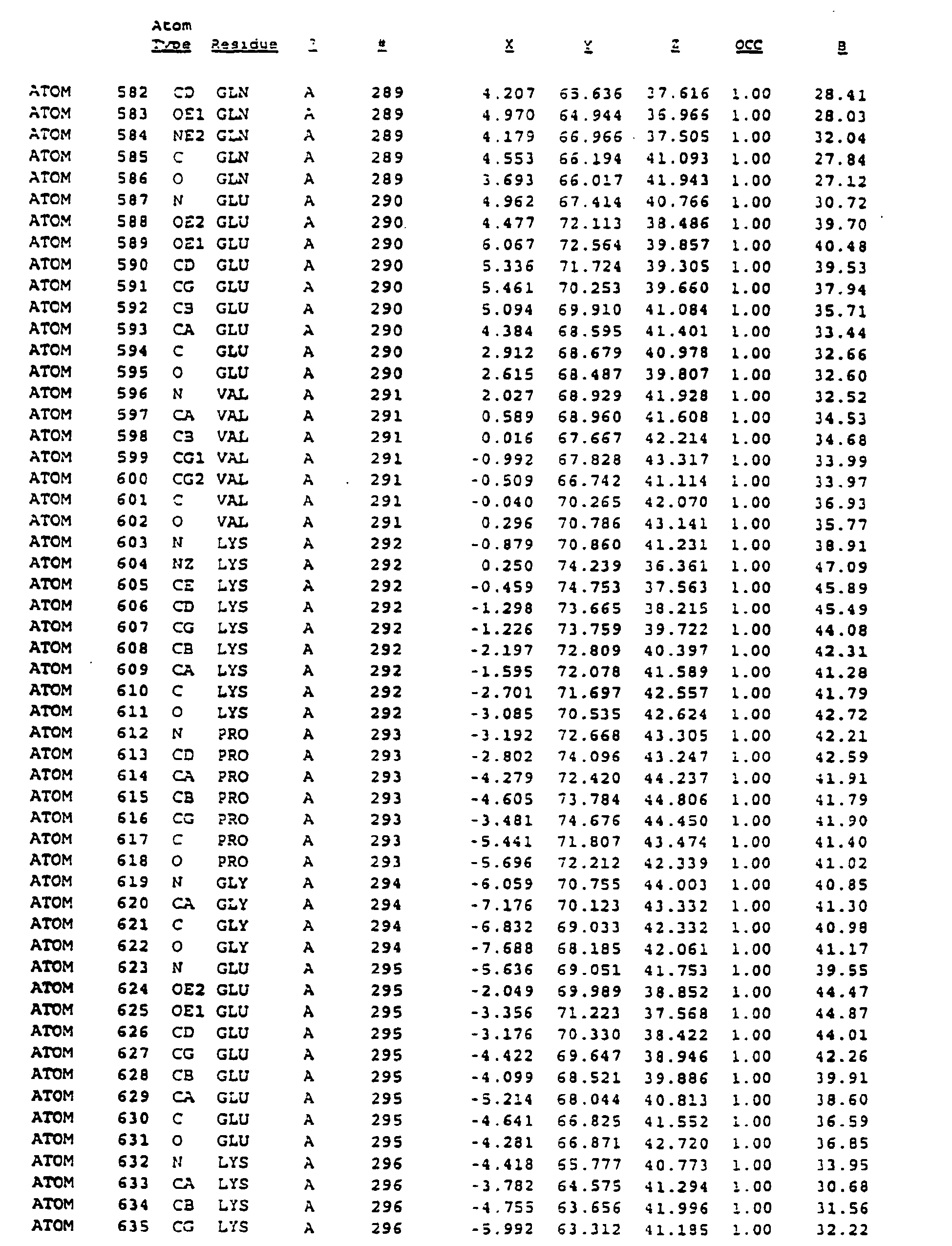

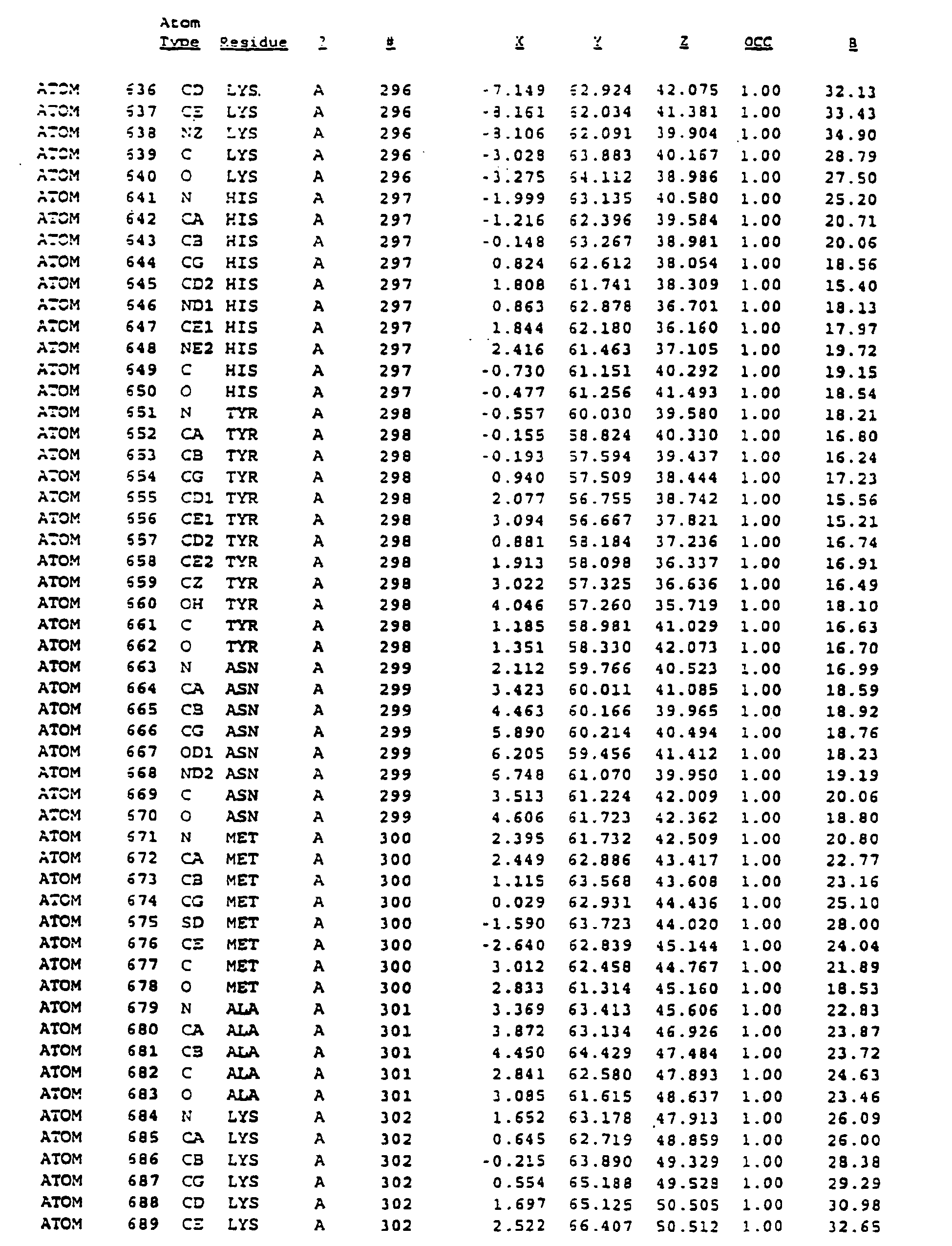

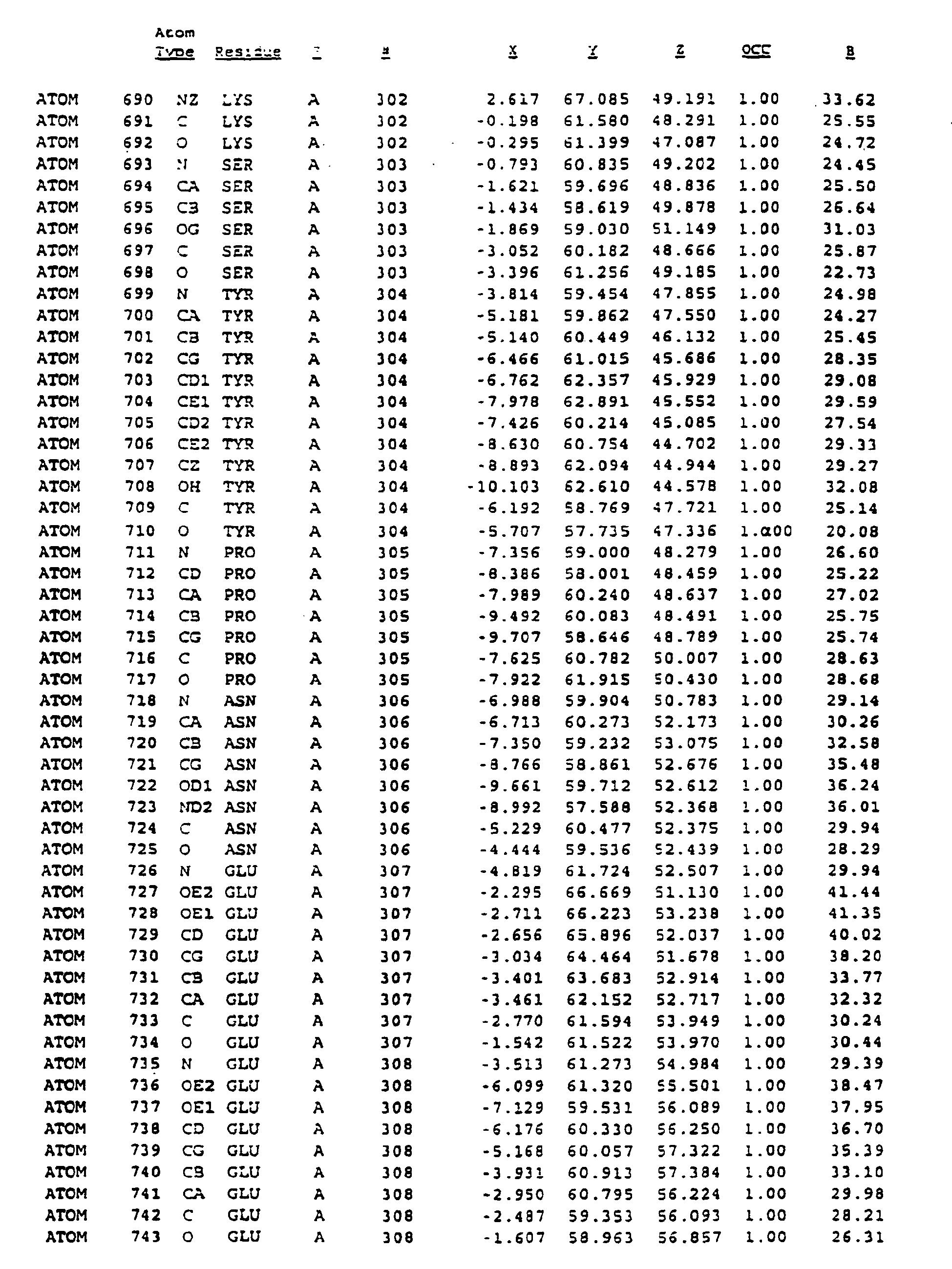

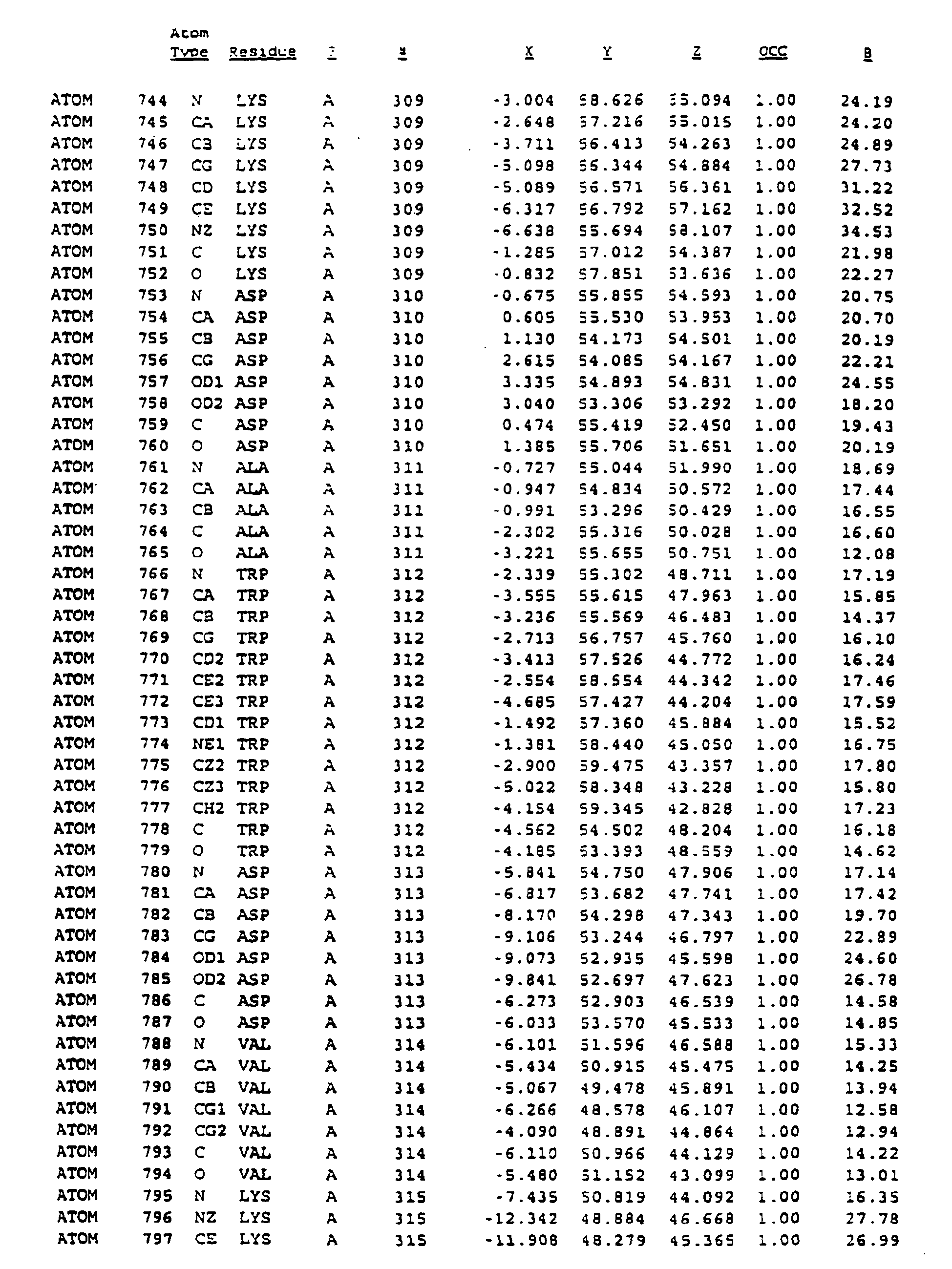

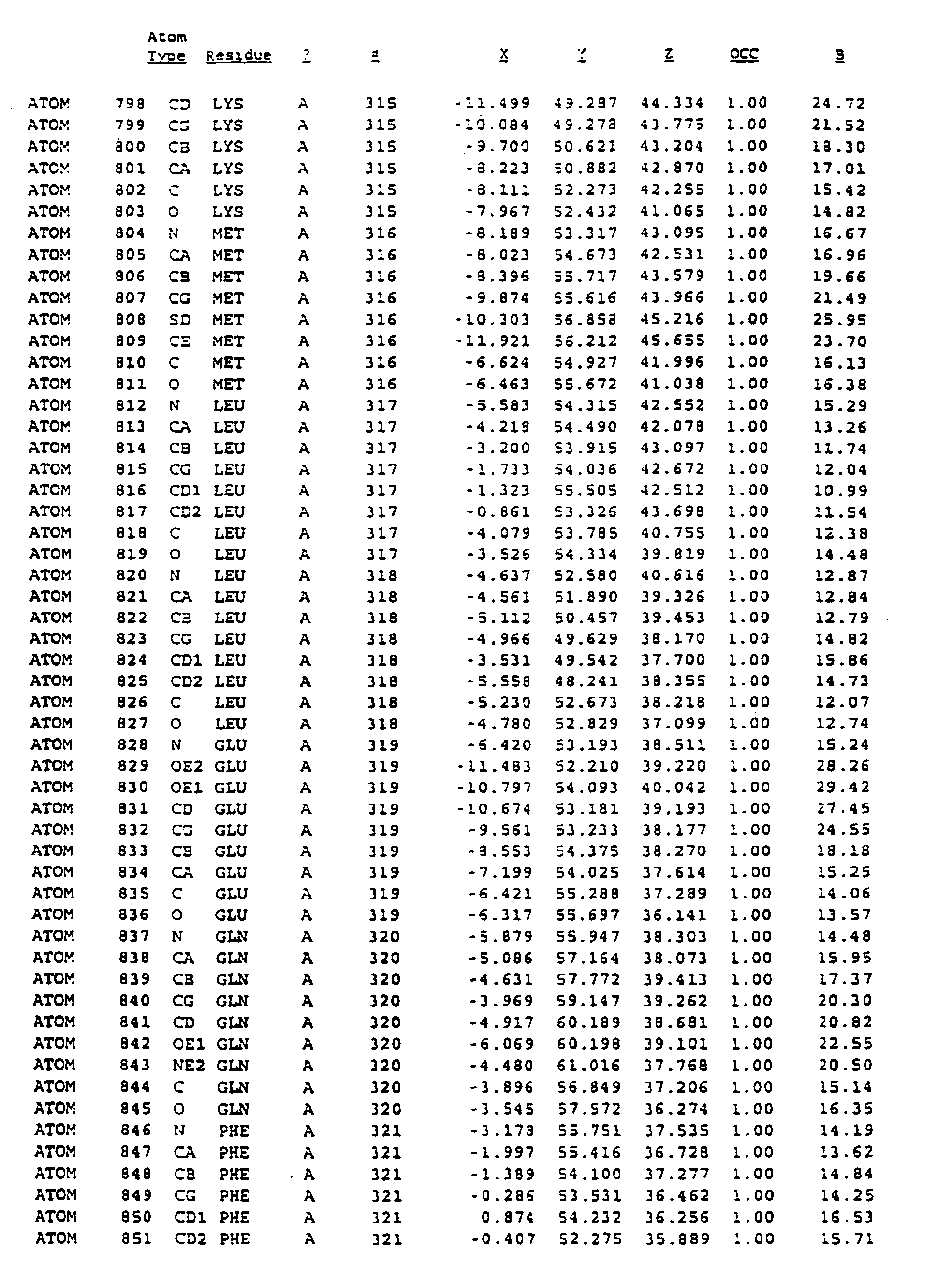

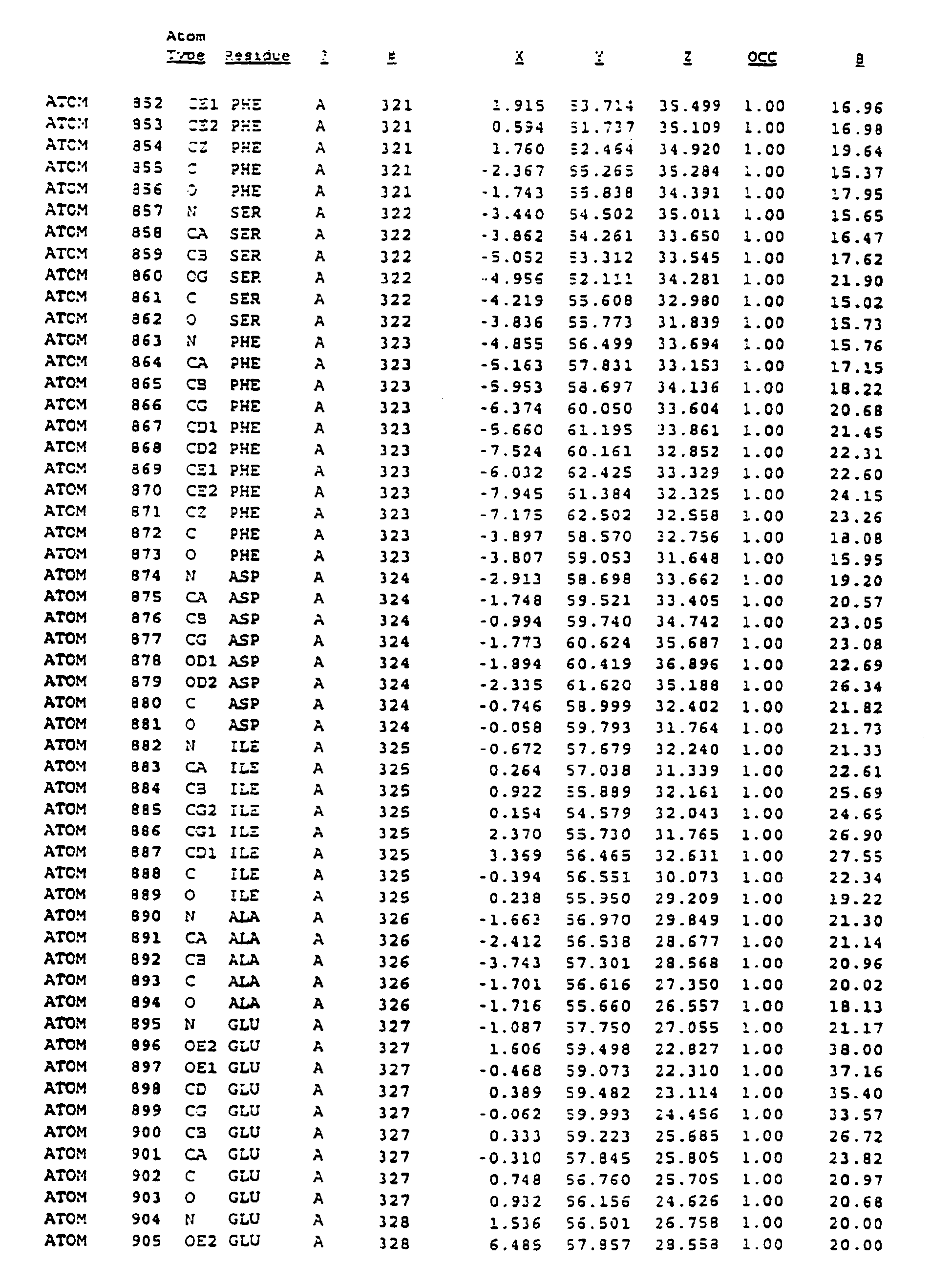

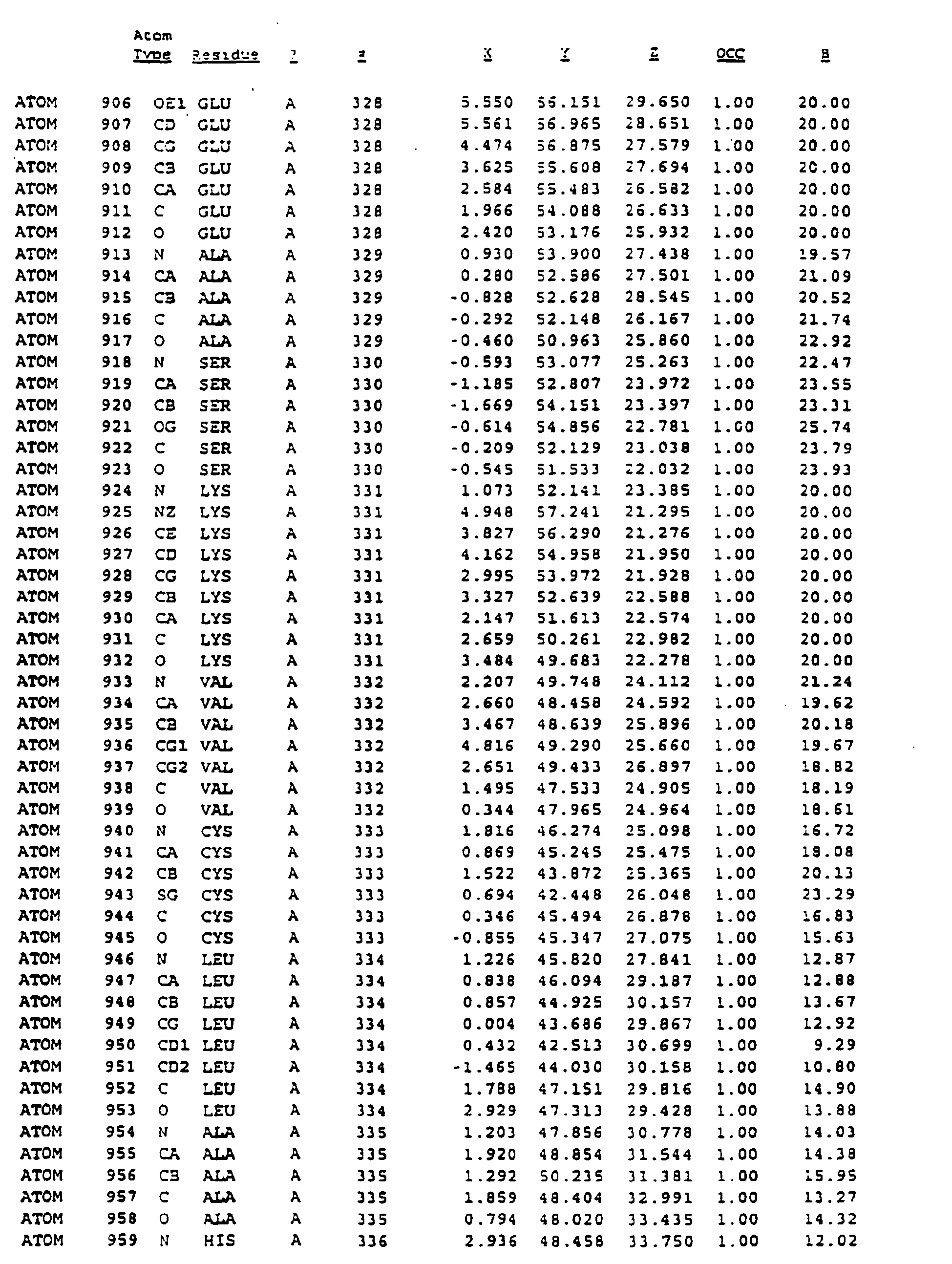

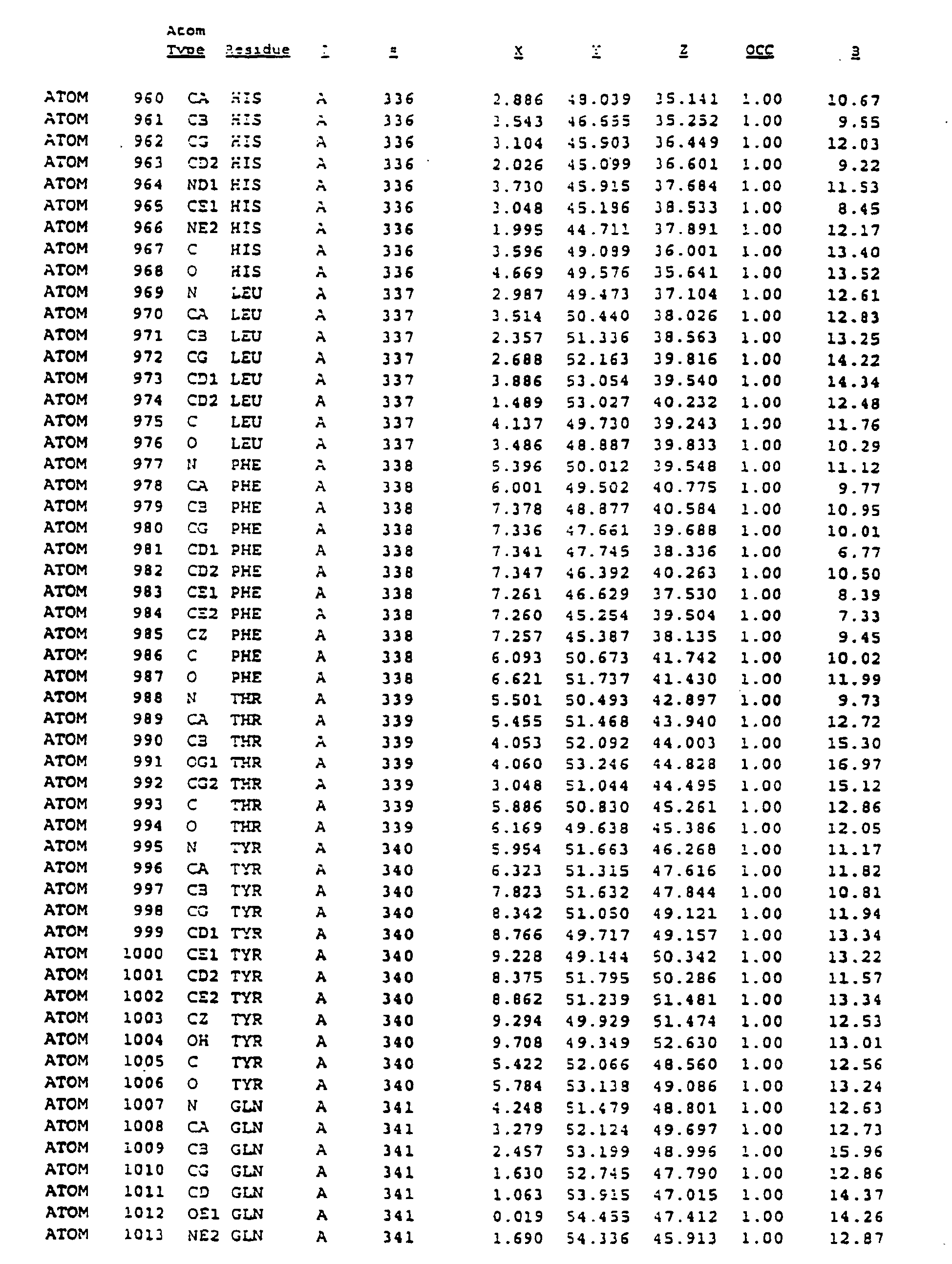

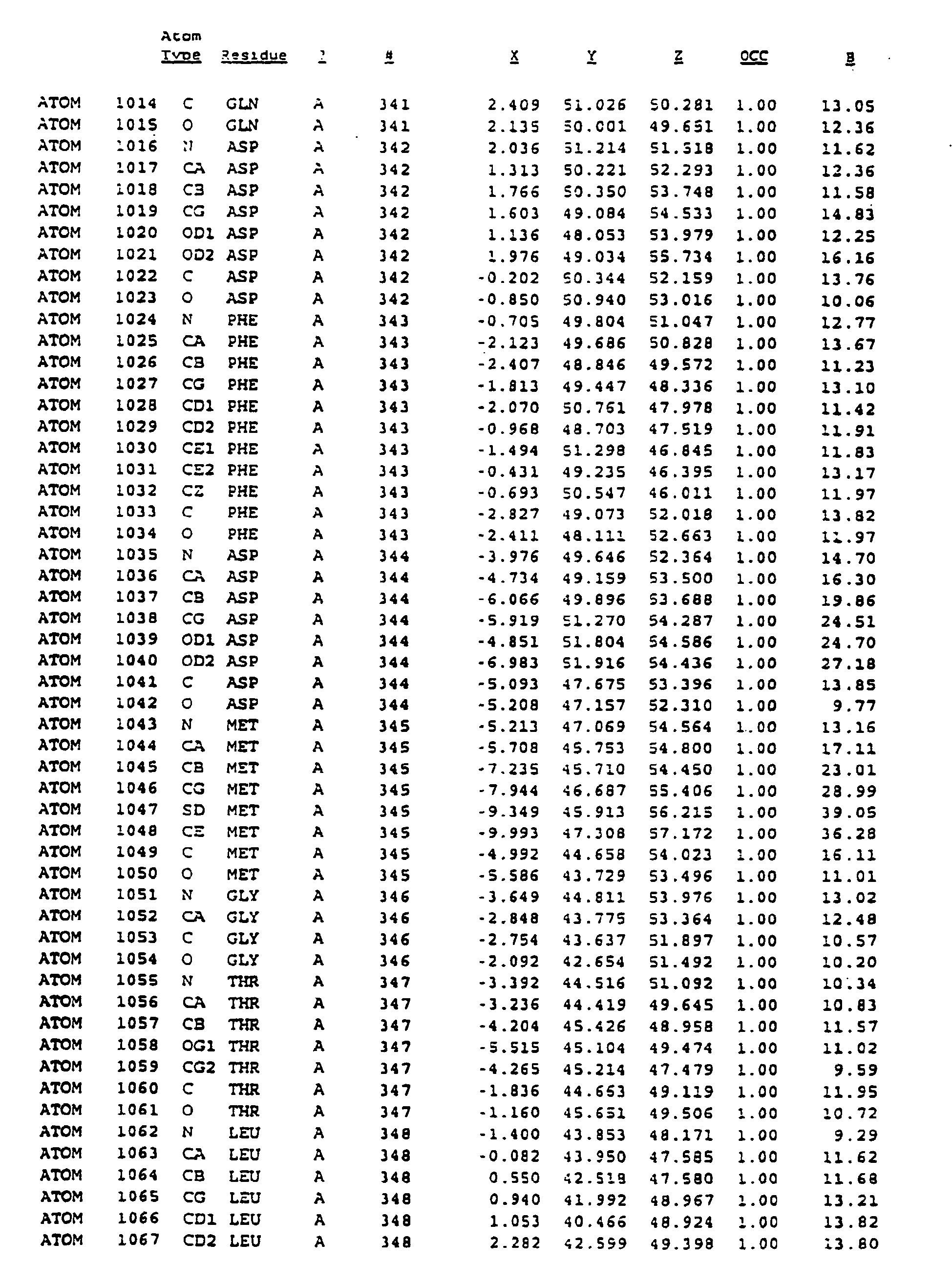

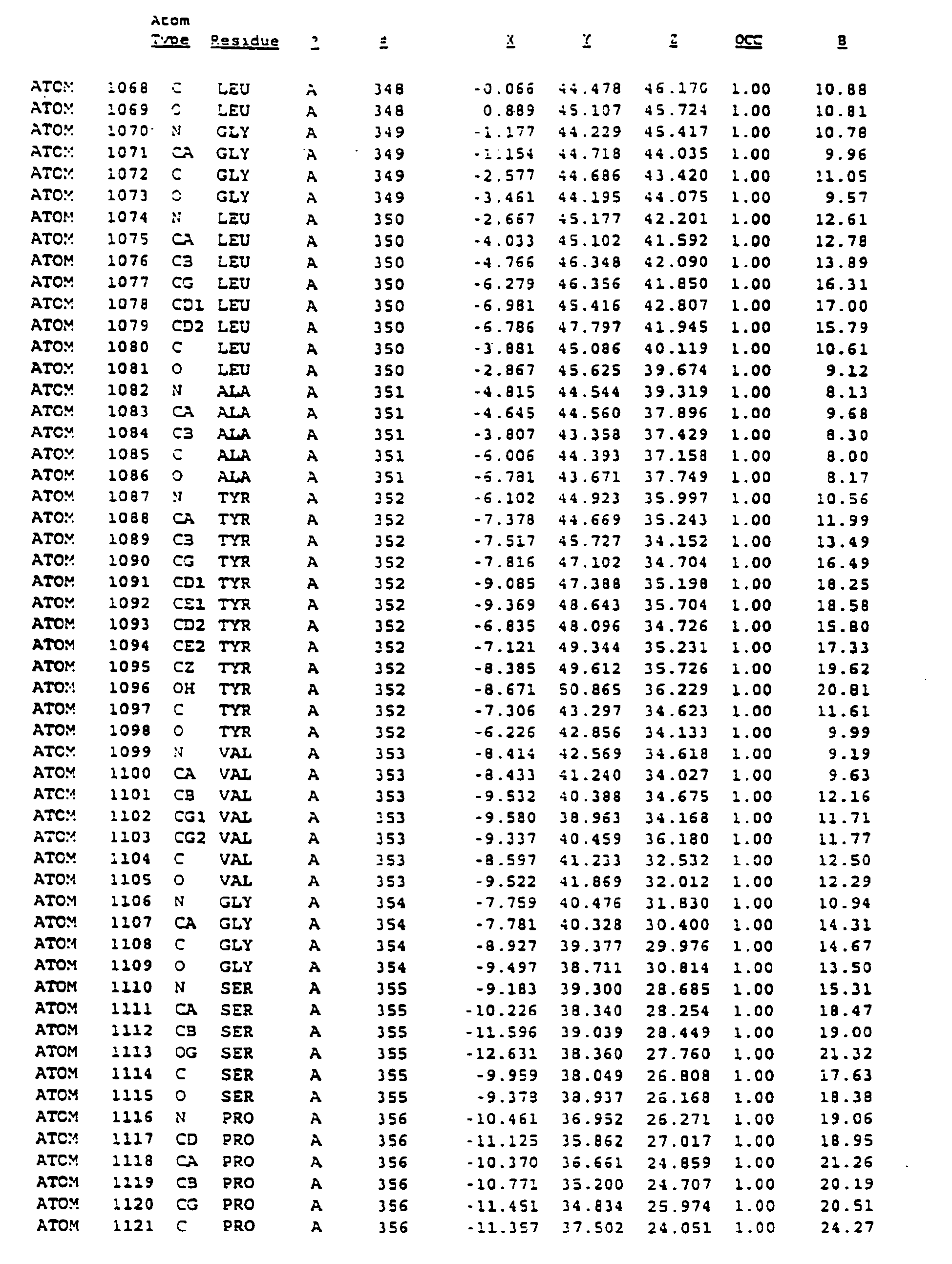

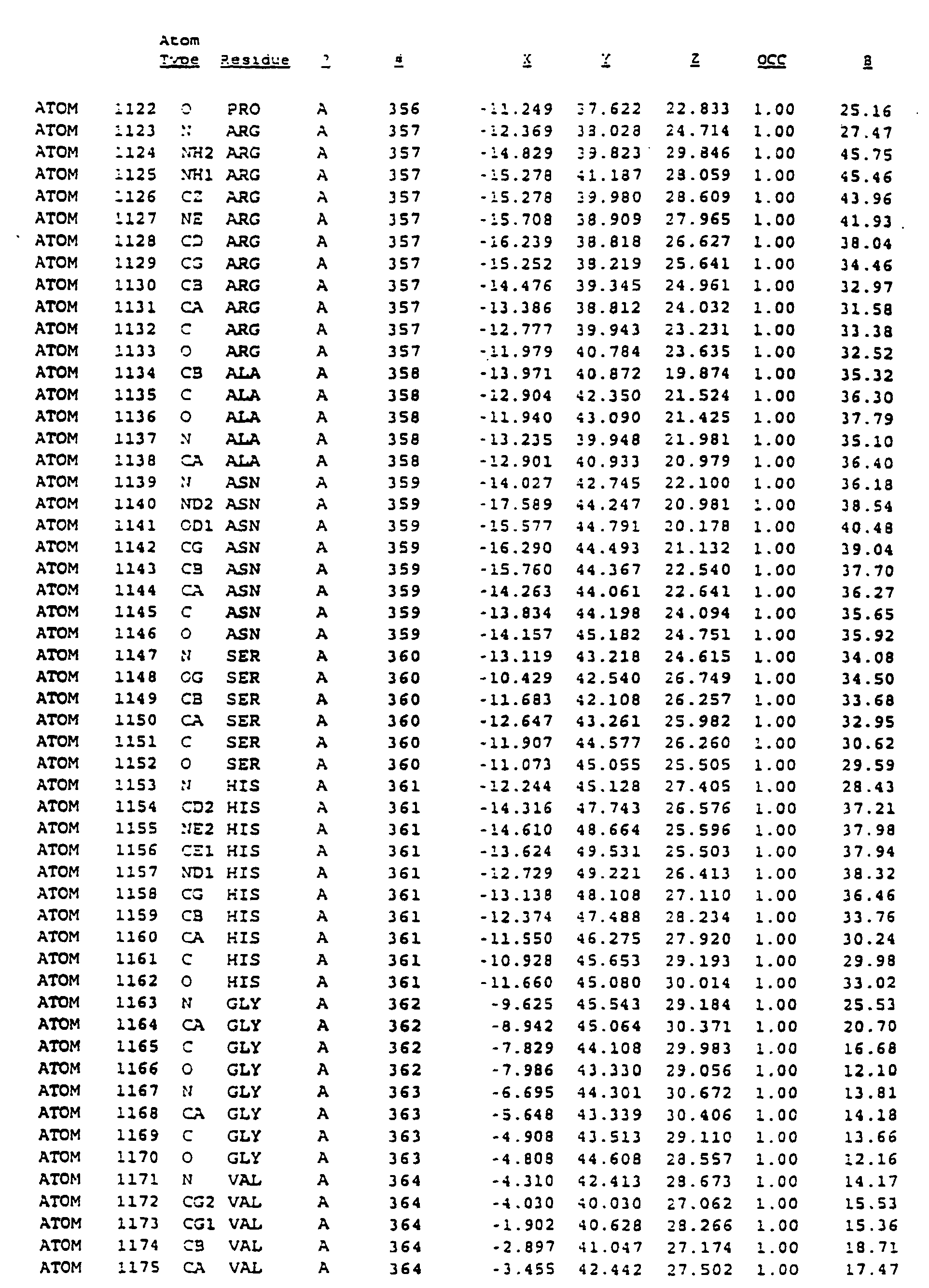

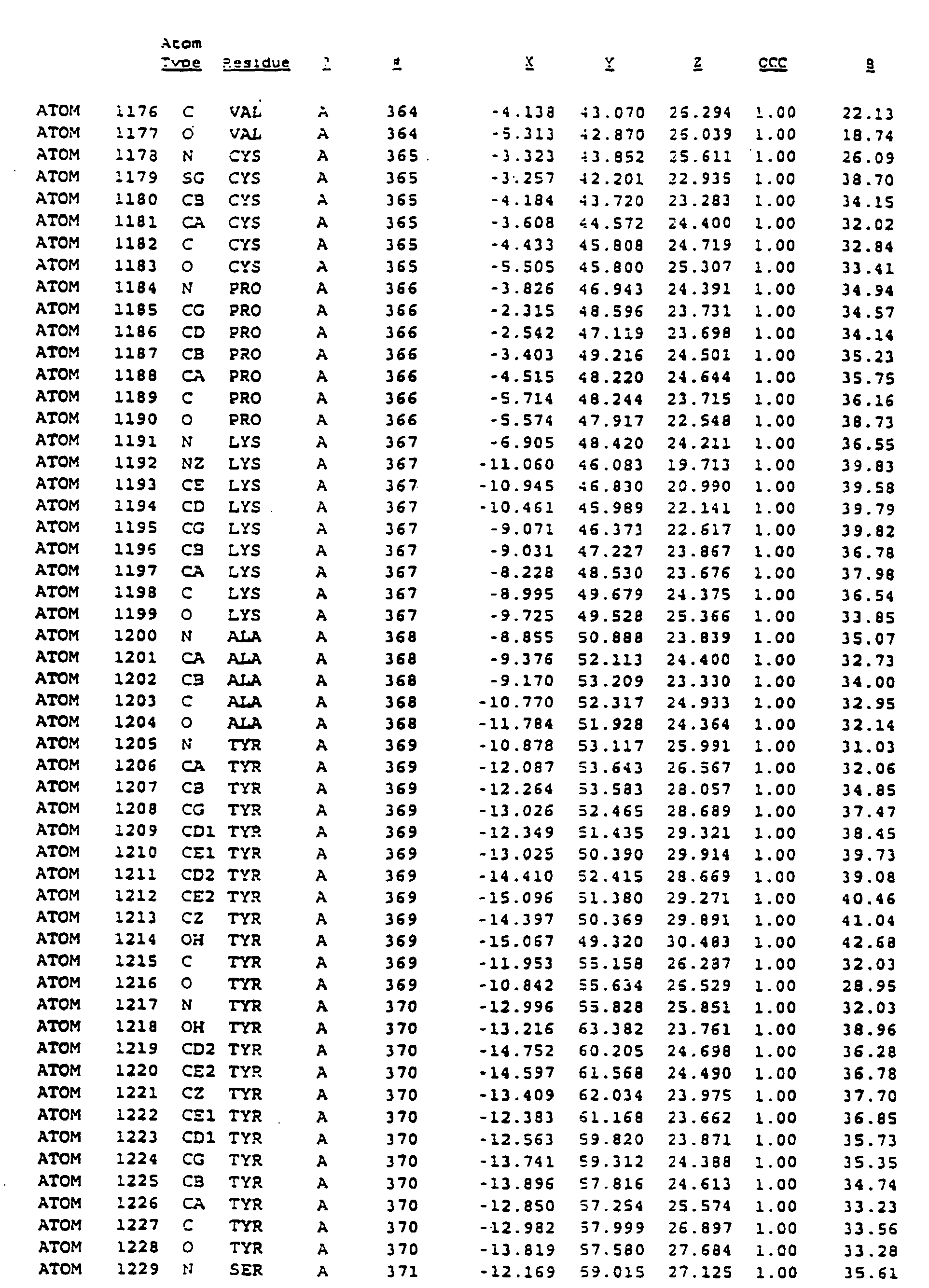

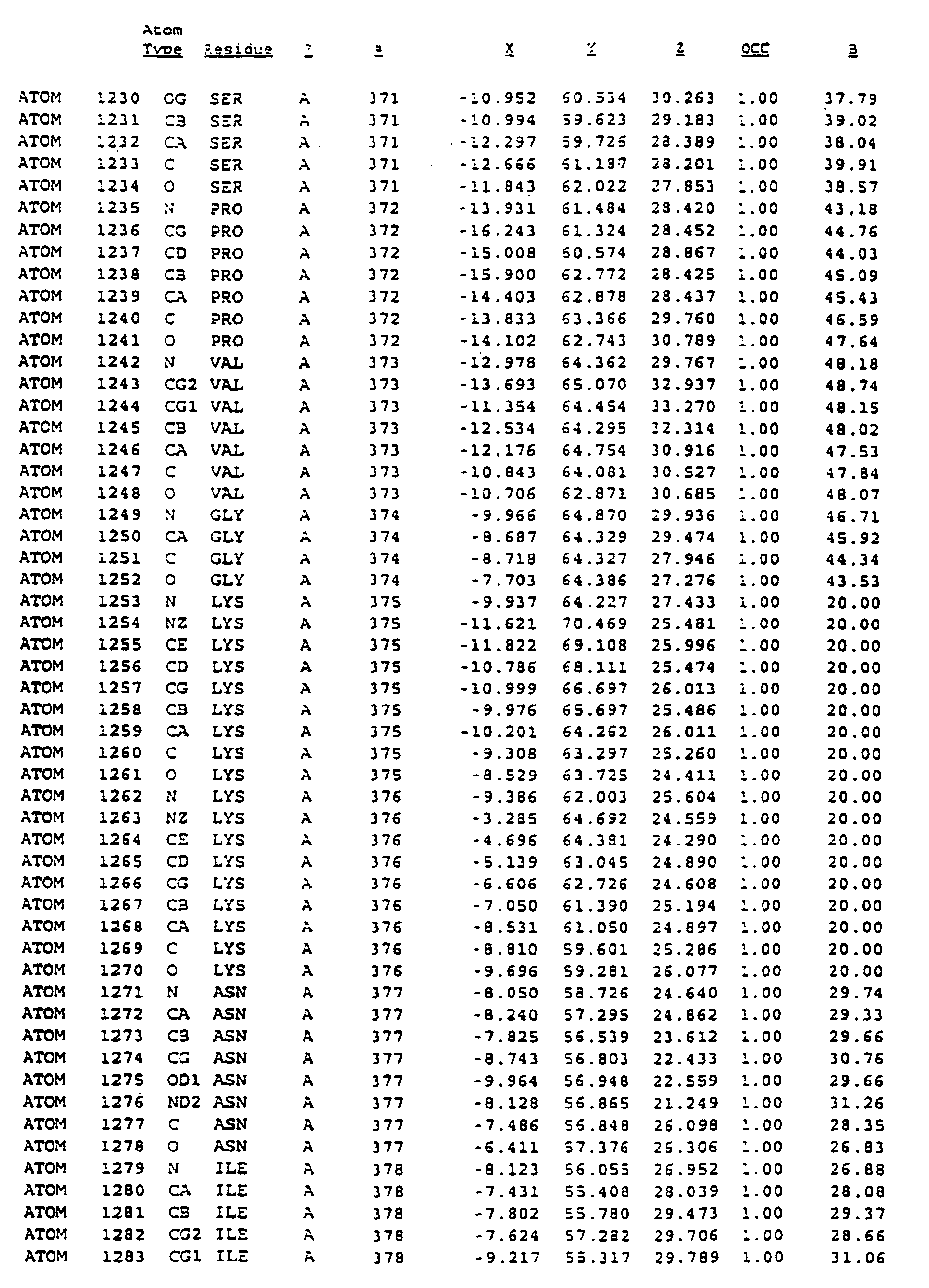

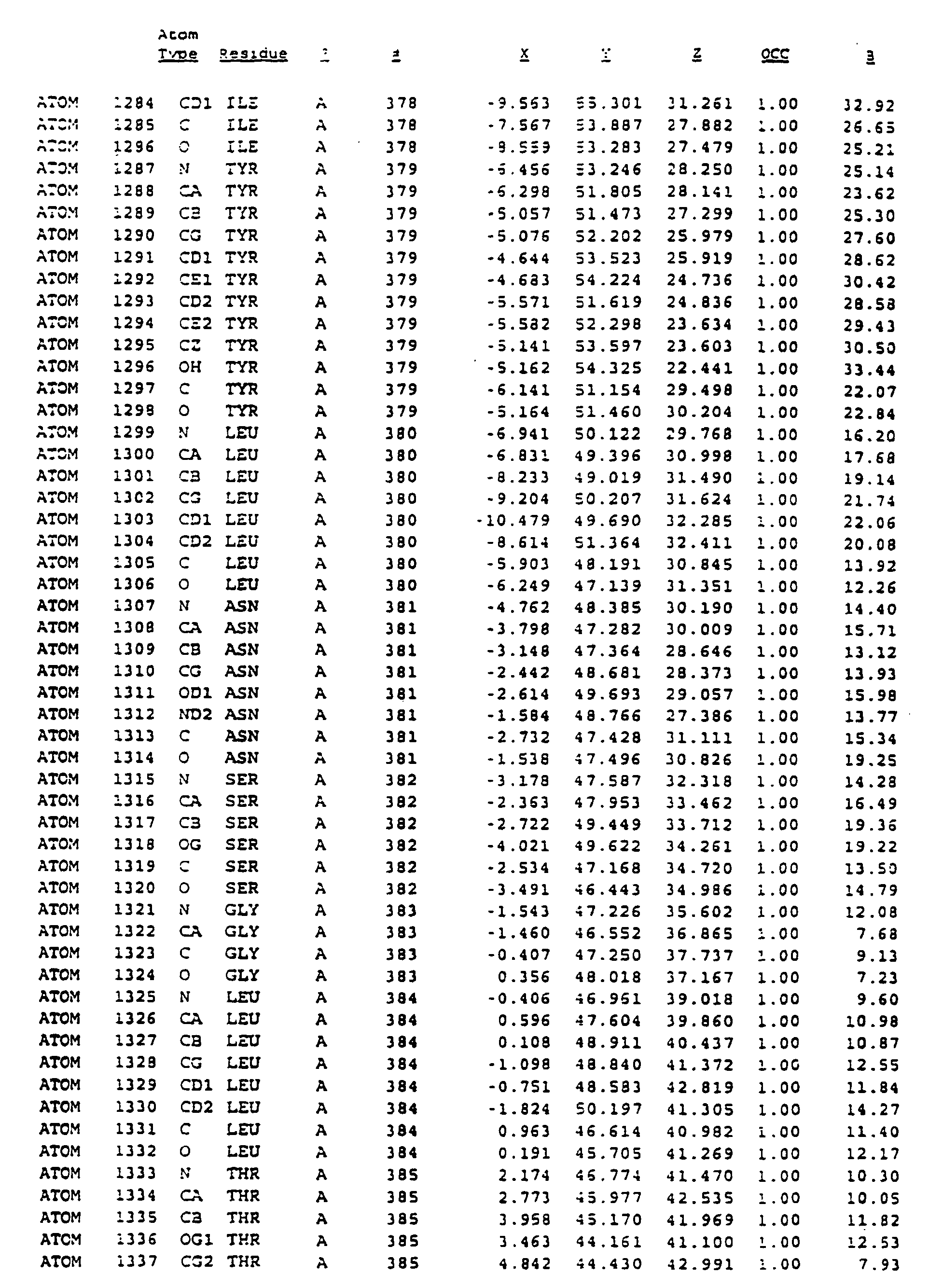

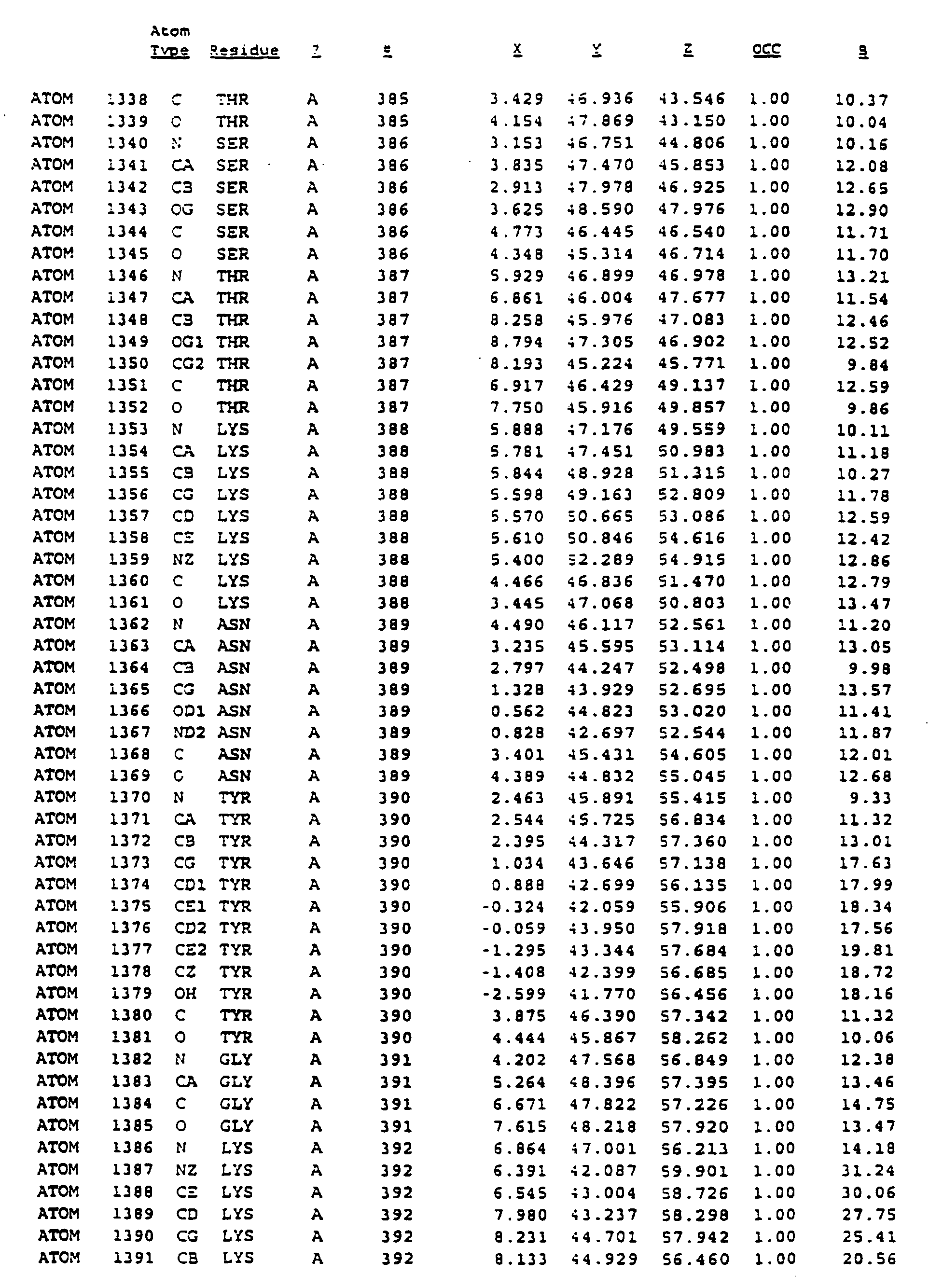

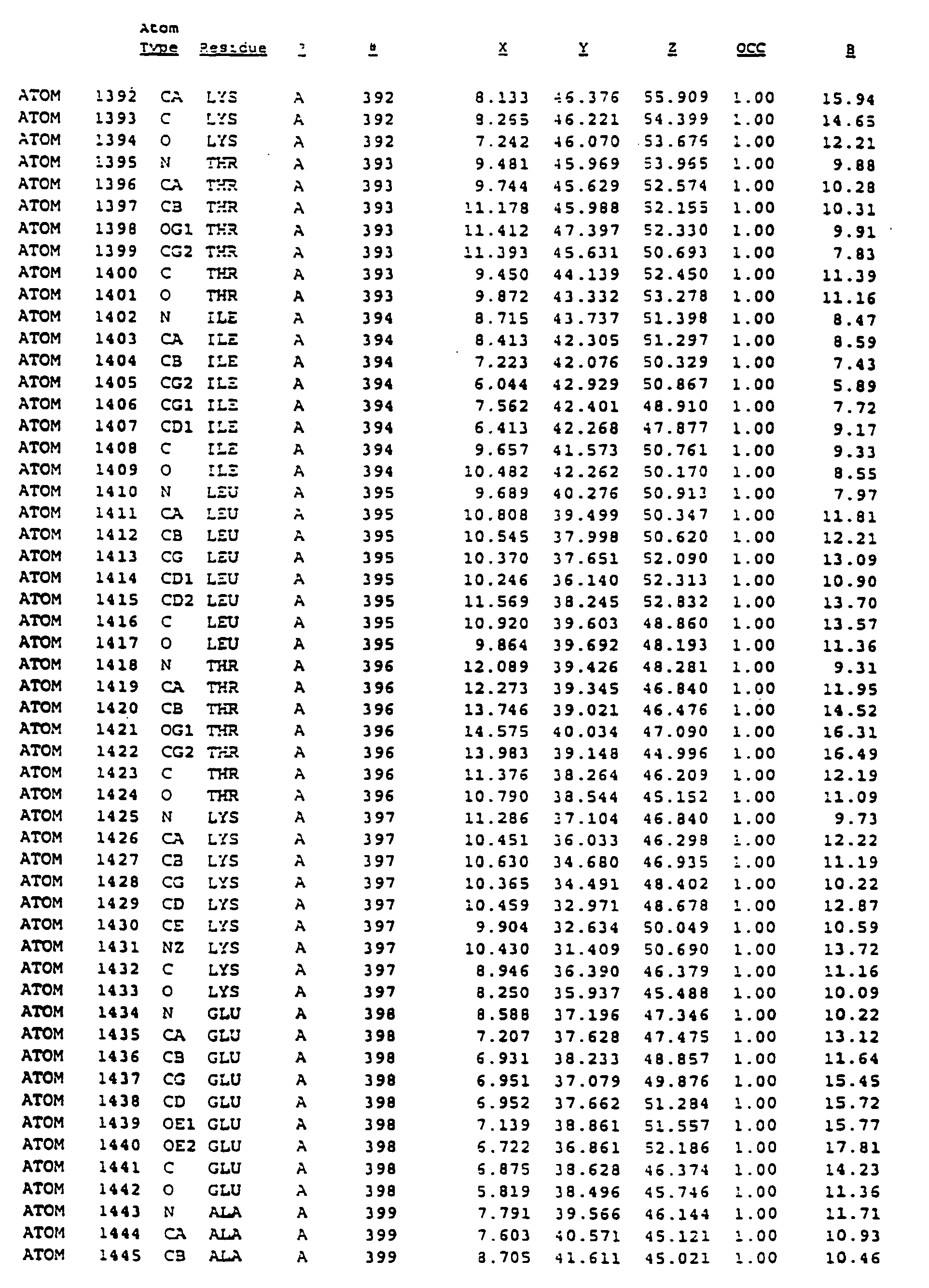

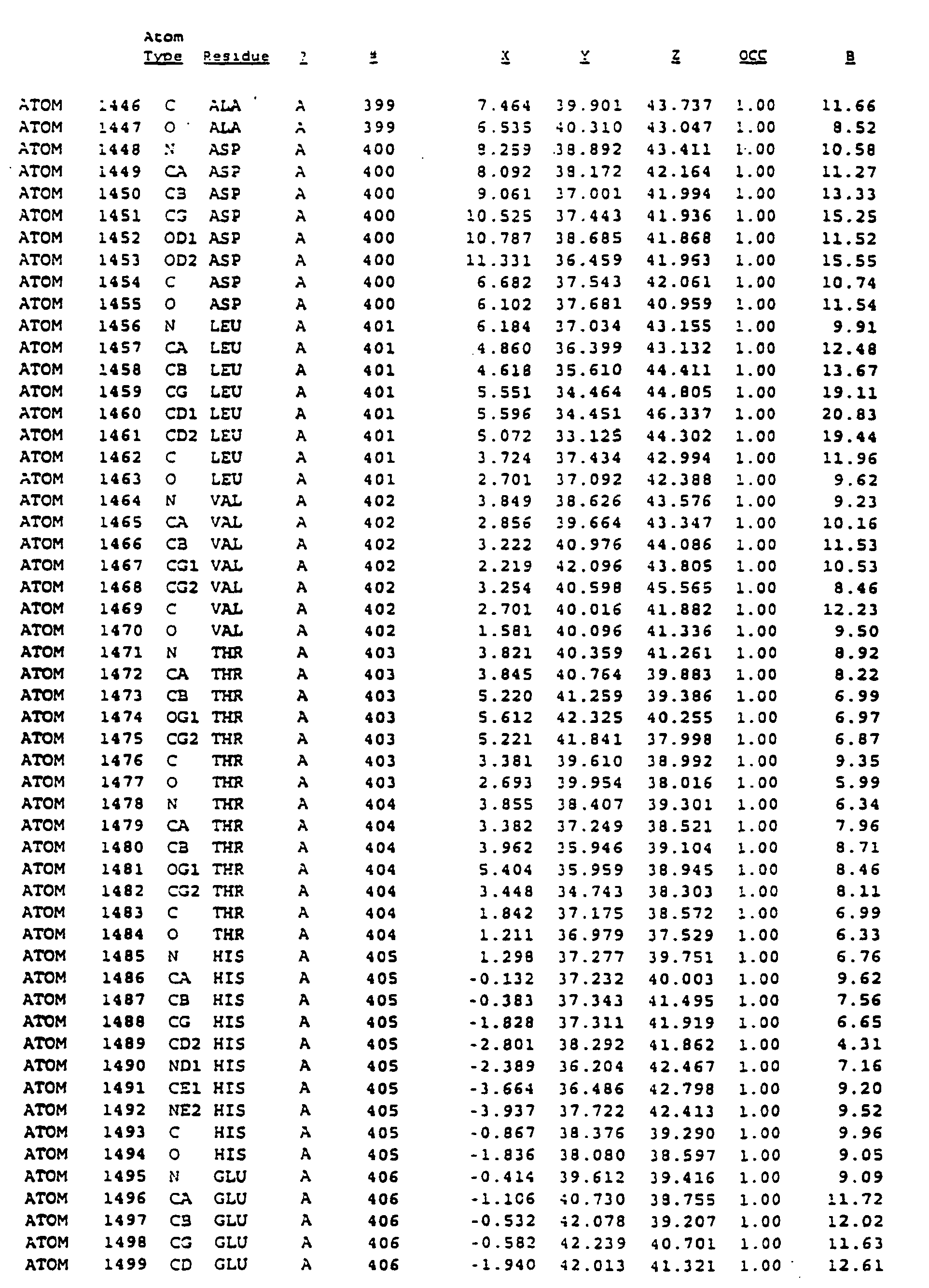

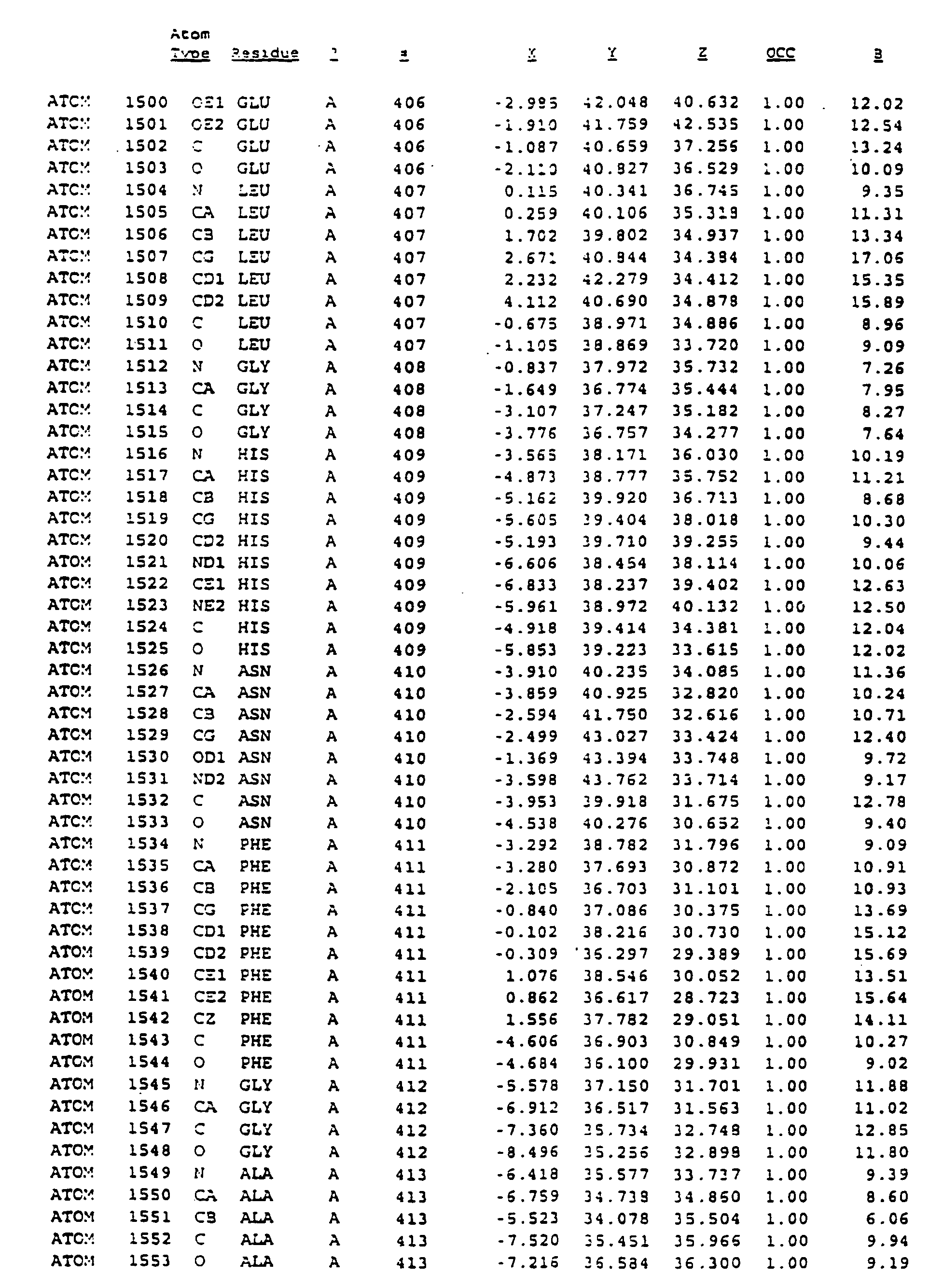

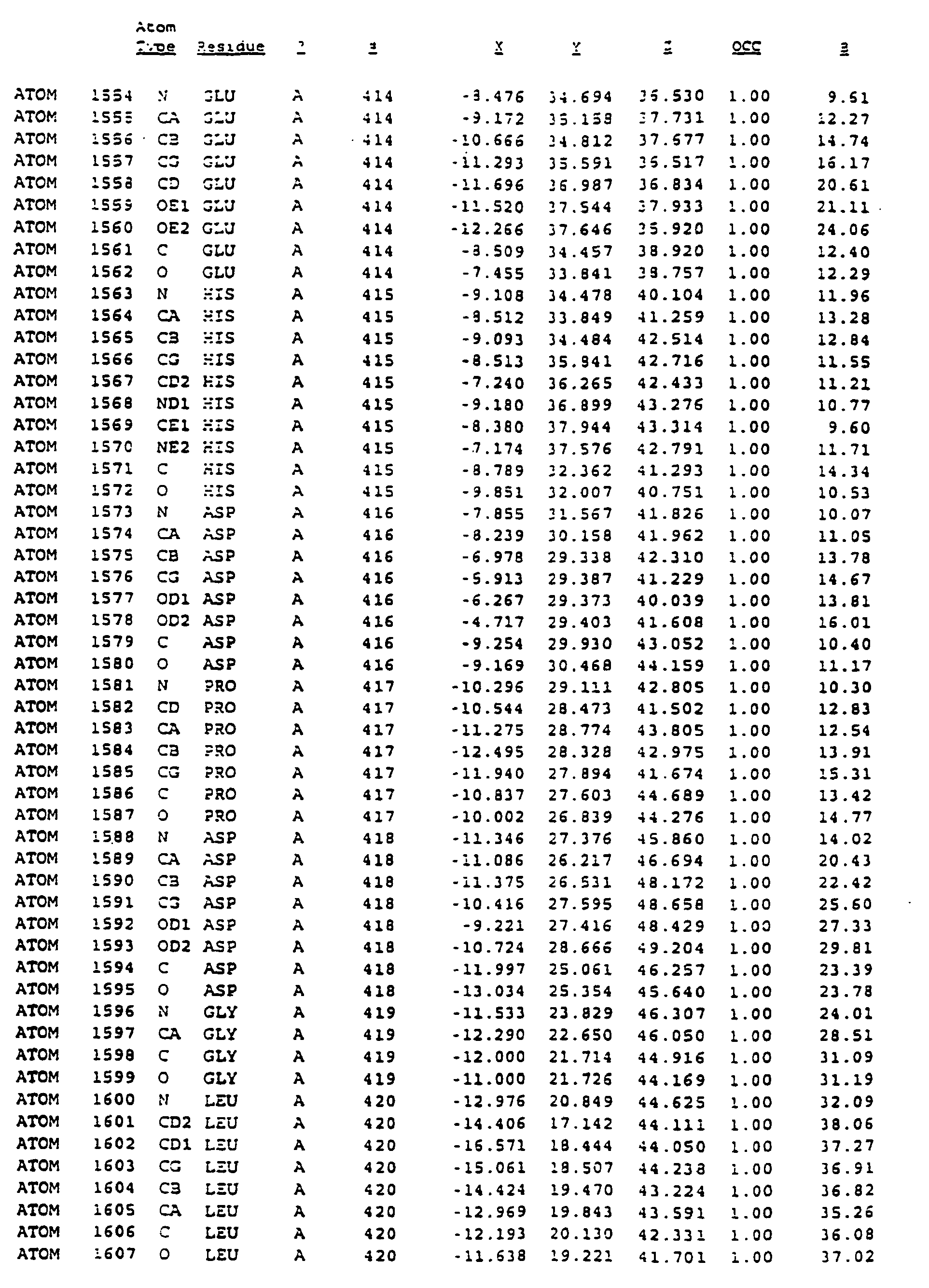

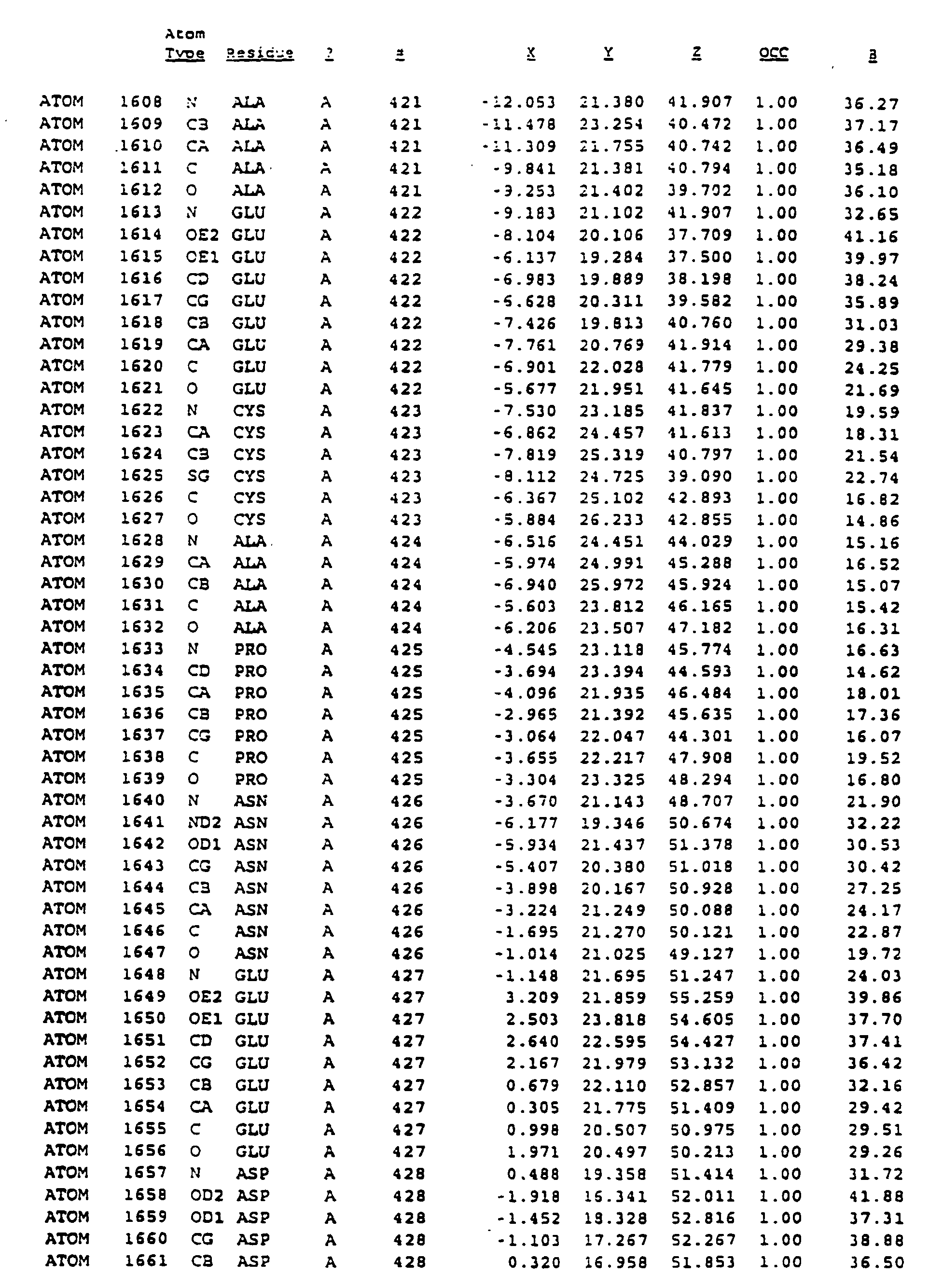

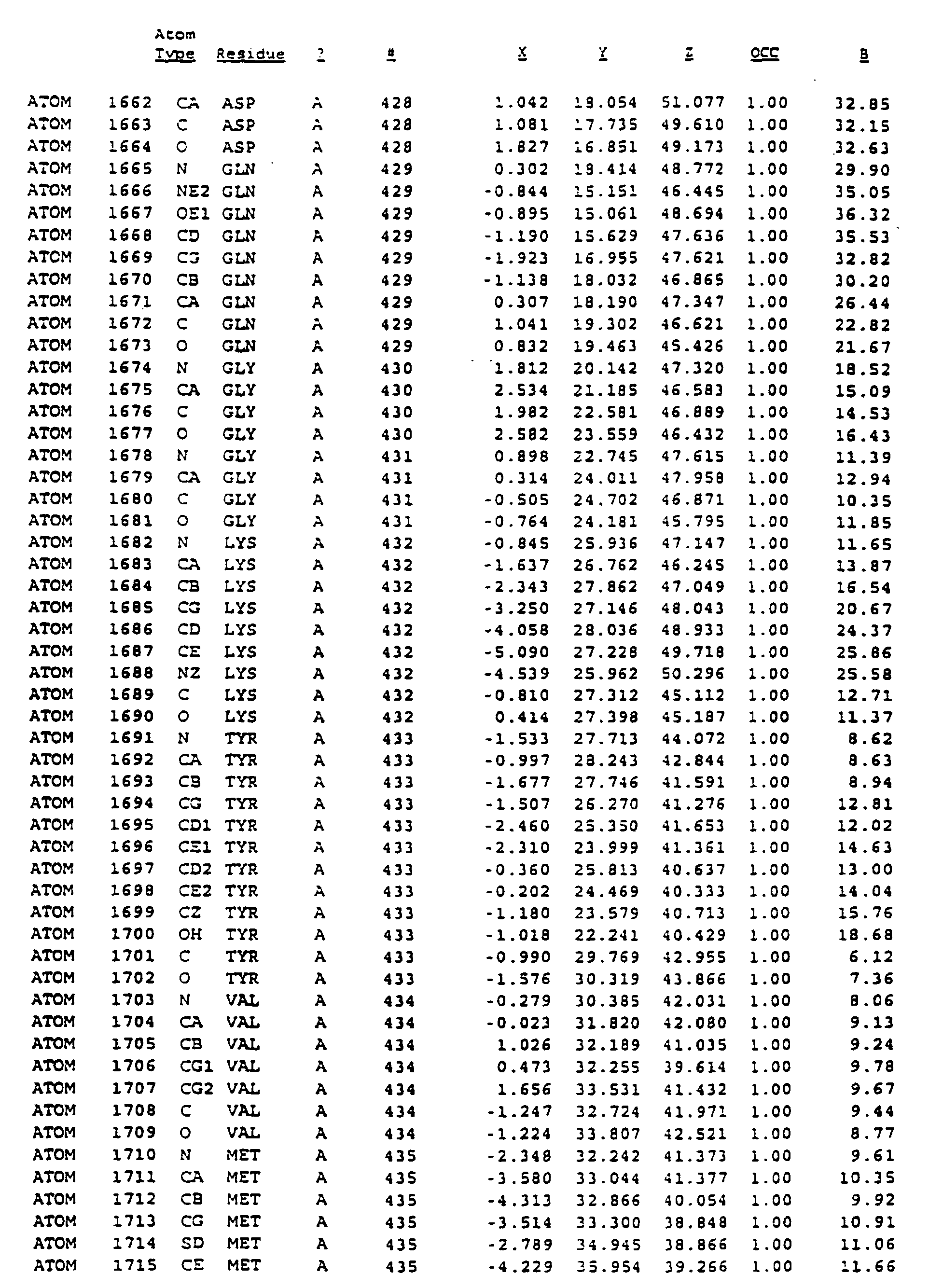

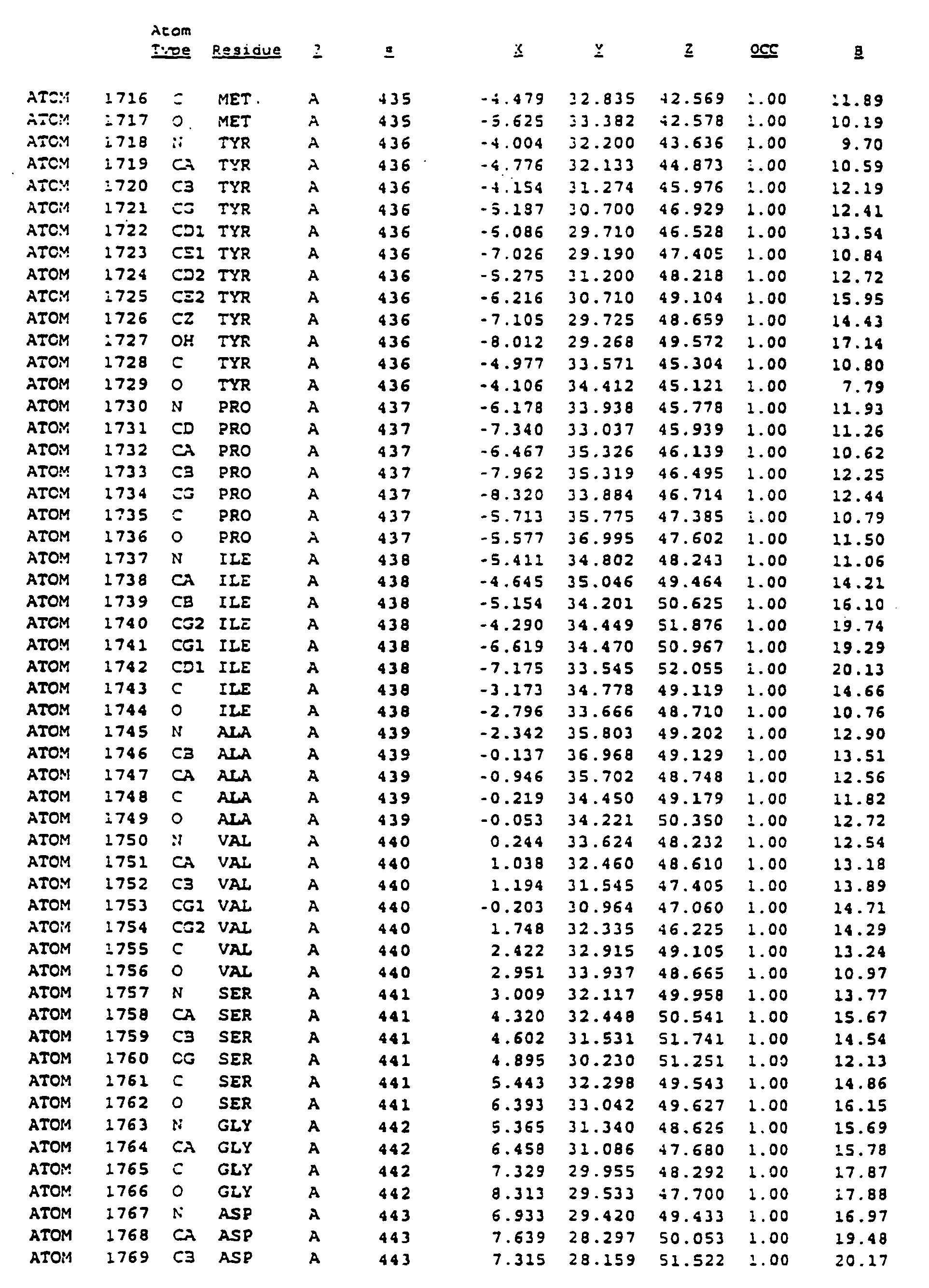

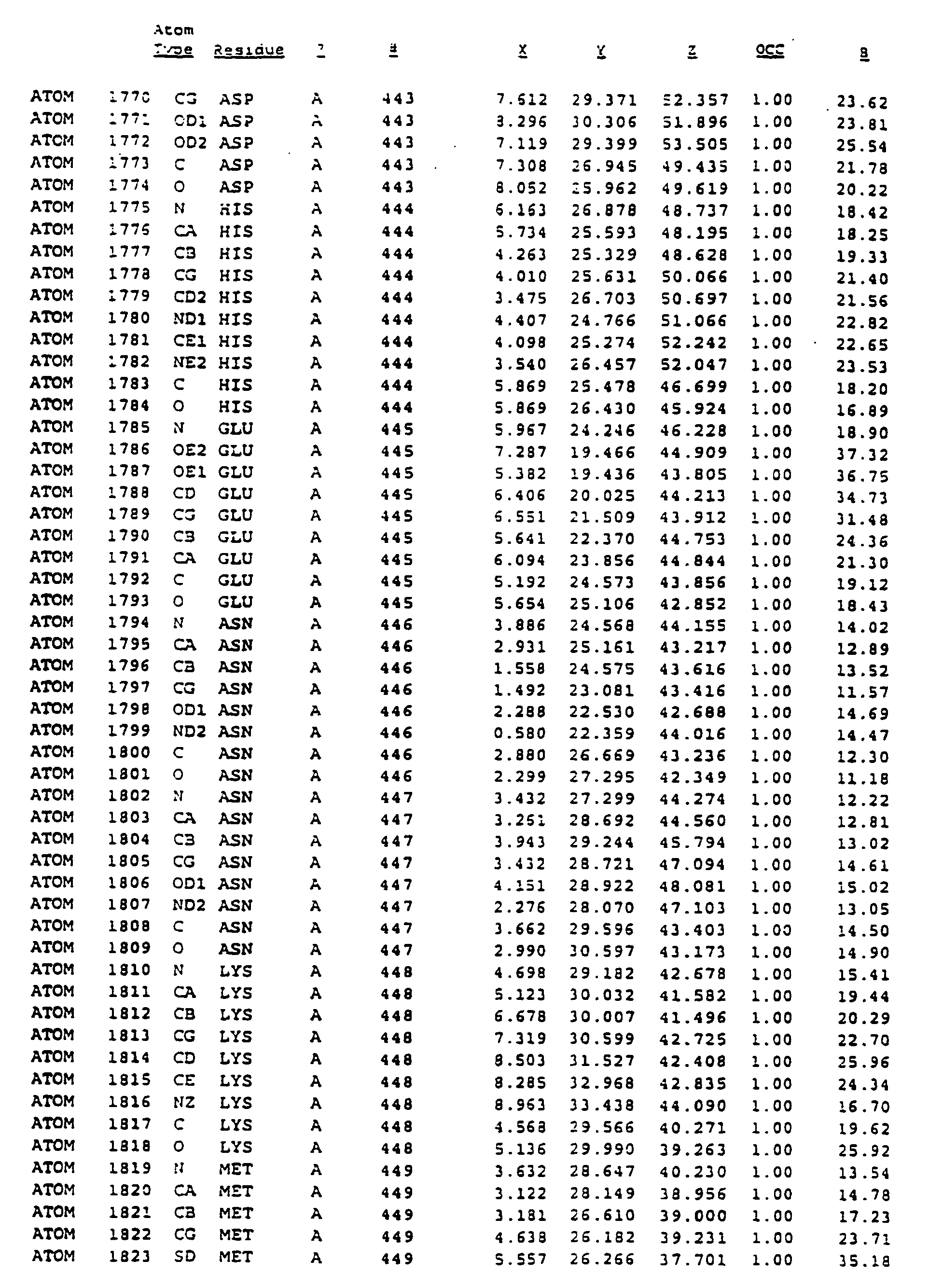

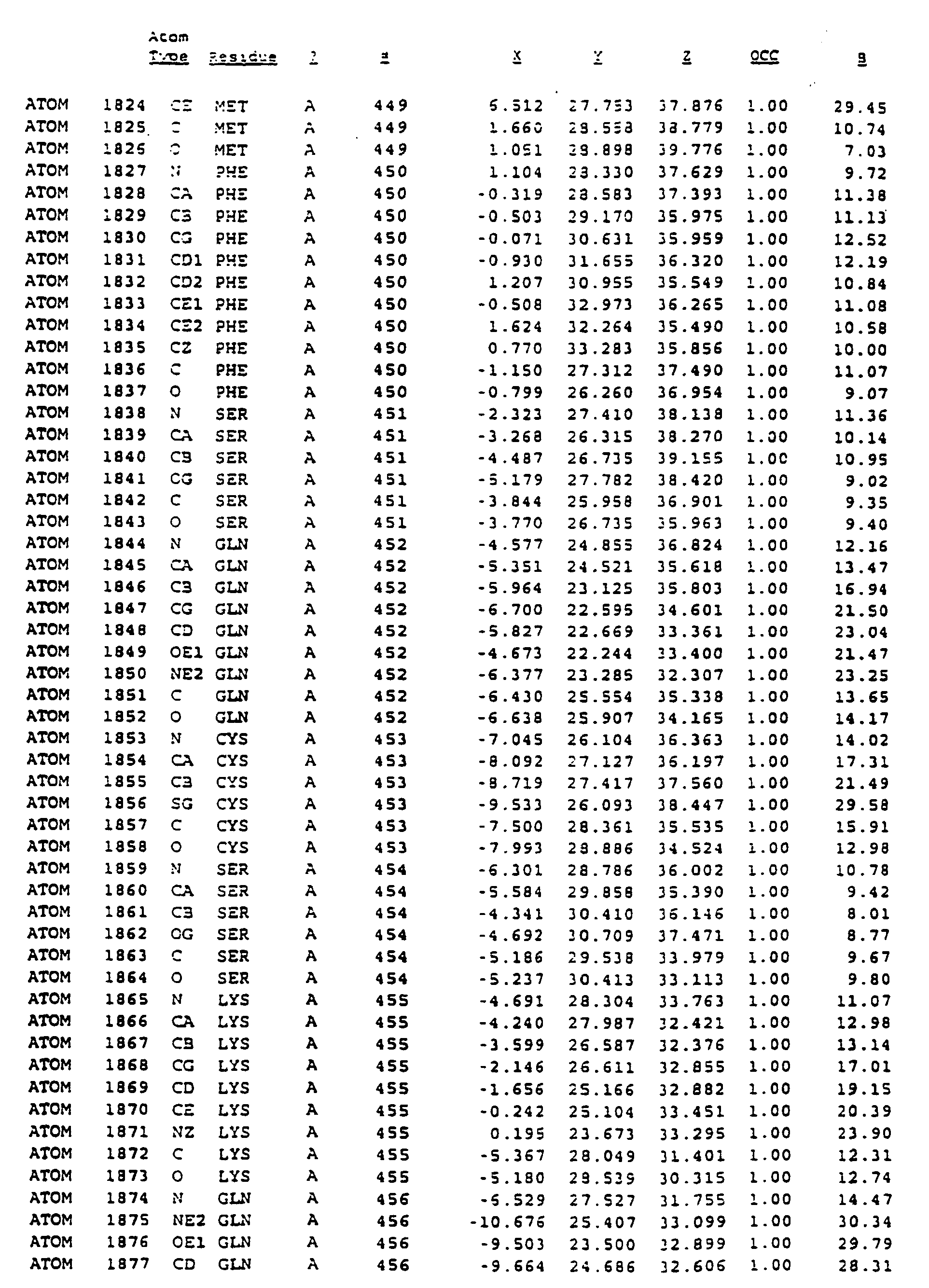

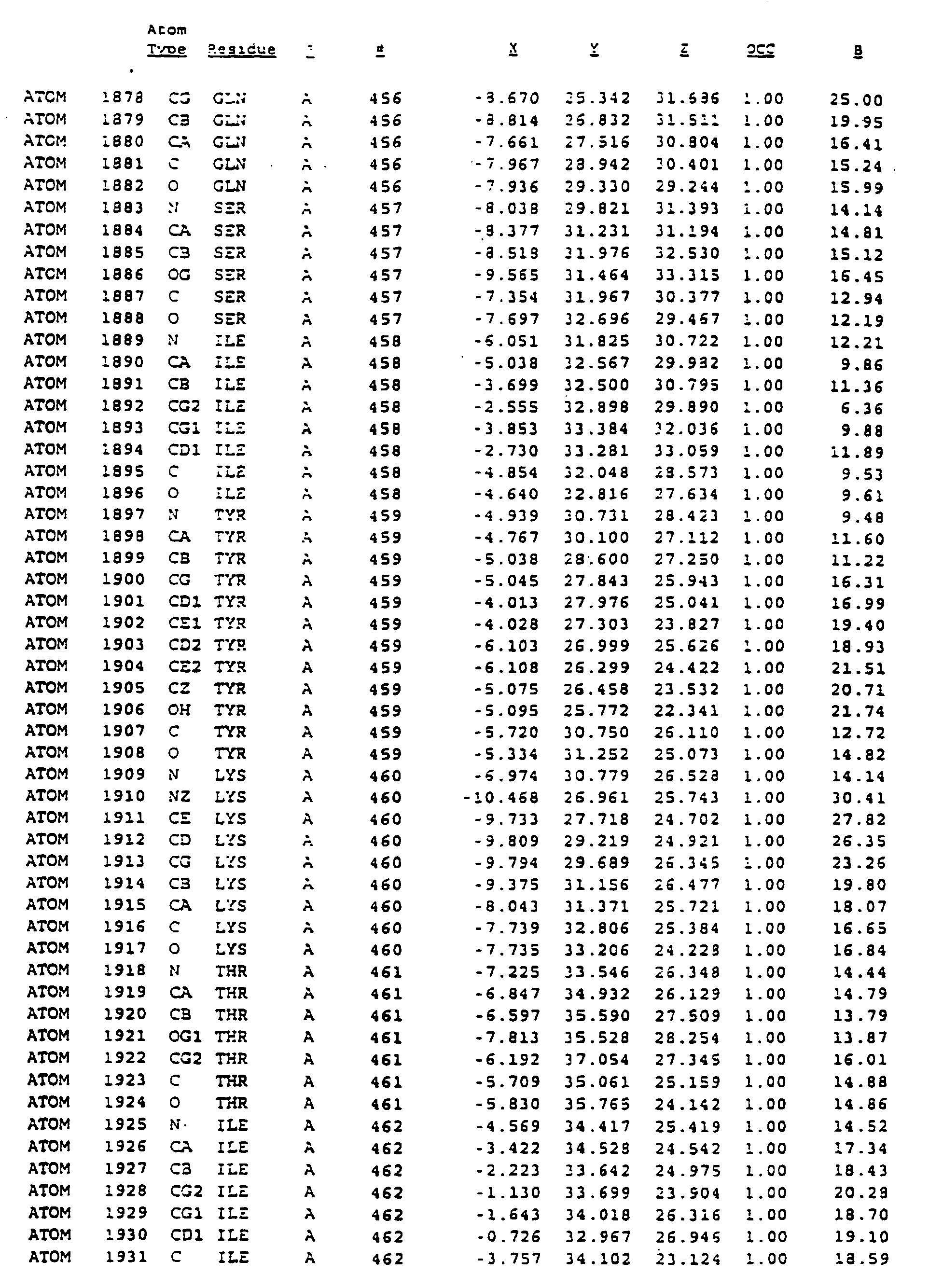

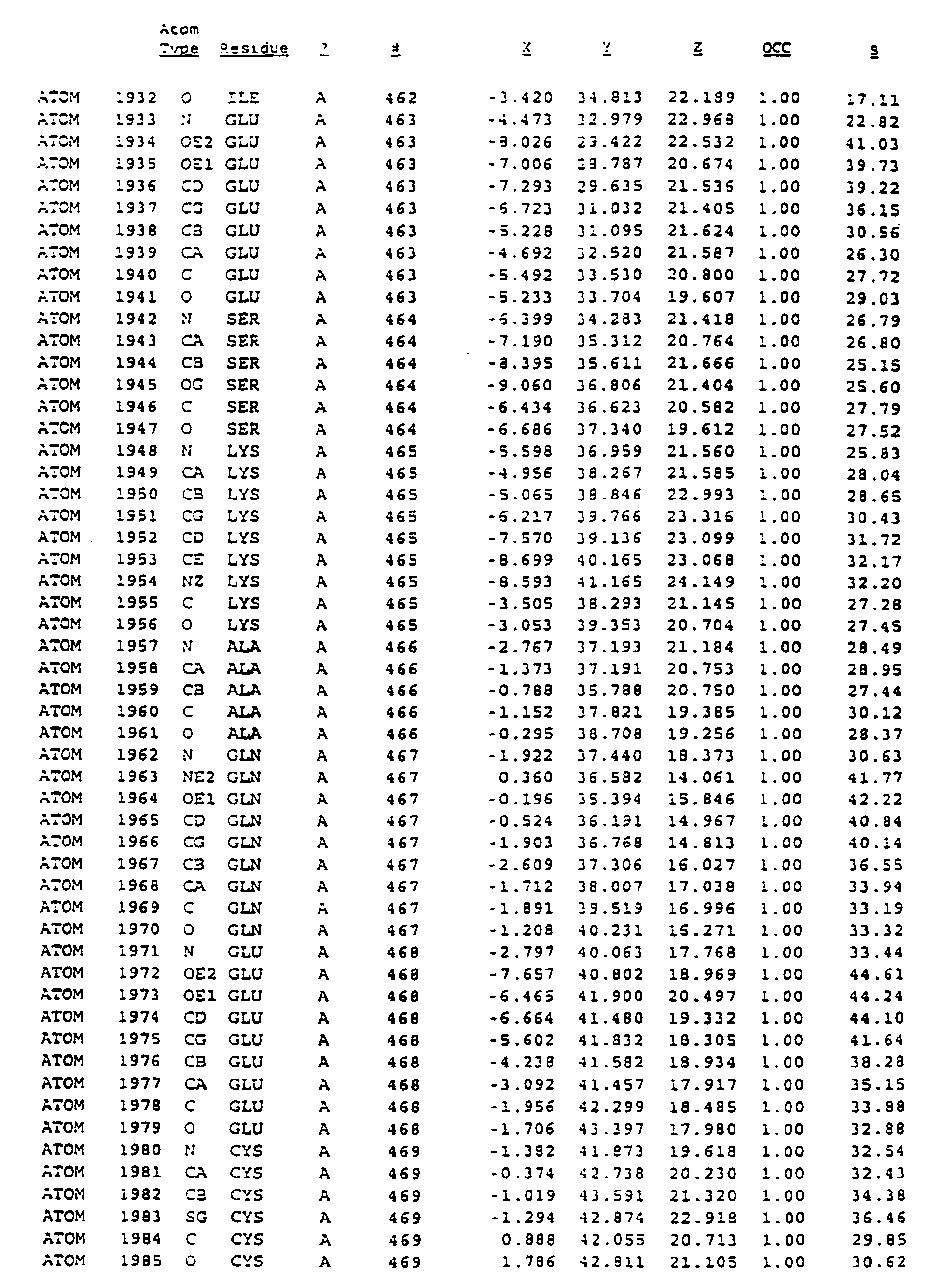

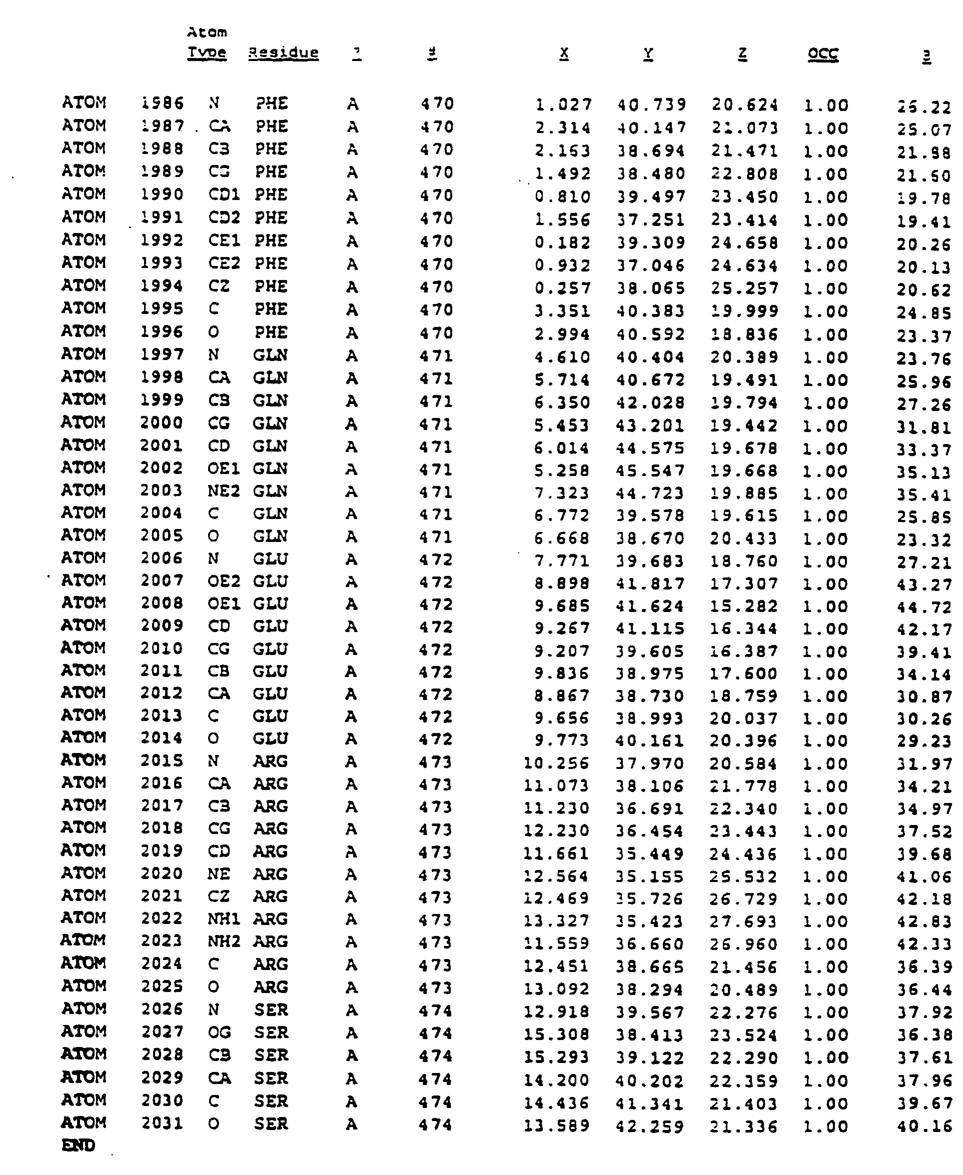

### Example 5 - TACE Inhibitor Design

The TACE x-ray diffraction coordinates were read into a Sybyl v.6.3 (Tripos Associates) software package and the x-ray structure analyzed graphically. The regions within the original x-ray coordinates were corrected for chirality and atom type. The modified x-ray model of TACE was energy minimized until all the TACE structural parameters were at their equilibrium or optimal values. The energy minimized structure was then compared to the original structure to confirm the absence of anomalies.

Sites of specific interaction(s) between TACE and the co-crystallized inhibitor were identified. The inhibitor was then removed from the X-ray complex model, leaving only the TACE structural model.

Candidate inhibitors were chosen based upon the sites of interaction with TACE and the candidate inhibitor in light of the sites of interaction identified previously for the co-crystallized inhibitor. Once specific candidate inhibitor-TACE interactions were determined, docking studies were performed to provide preliminary "modeled" complexes of selected candidate inhibitors with TACE.

Constrained conformational analysis was performed using molecular dynamics (MD) to check the integrity of the modeled TACE-inhibitor complex. Once the complex reached its most favorable conformational state, the structure as proposed by the MD study was analyzed visually to insure that the modeled complex complied with known experimental SAR/QSAR based on measured binding affinities.

The modeled candidate inhibitor-TACE complex was analyzed. The region of the complex associated with the S1' regions of TACE containing a small solvent exposed channel was chosen as a target region for modification. A single modification, a benzyl group which becomes embedded within the target region, was selected based upon computational and synthetic chemical principles. The benzyl group was oriented on an appropriate zinc chelator core so as to be projected into the S1' S3' pocket. This modification converts an inhibitor which was generally MMP selective to one which is TACE selective. IC50 data for the inhibitor with a benzyl modification confirm this selectivity.

Structure-based analoging for optimization of inhibitor potency, selectivity and physical drug-like properties was performed in an iterative manner.

### Example 6 - Measuring TACE Inhibition

250µM peptide substrate (Ac-SPLAQAVRSSSR-NH₂) was incubate with 3.7 U/µL TACE in a buffer containing 10mM TRIS HCl, pH 7.4, 10% glycerol at 25 degrees C. The reaction was quenched with 1% TFA (final concentration) after two hours. The reaction mixture was separated by HPLC on a Hewlett-Packard 1150. The product formation was monitored by absorbance at 220nm.

The linearity of the reaction was confirmed (r²>0.85). The mean (x ± sem) of the control rate was calculated and compared for statistical significance (p < 0.05) with drug-tested rates using Dunnett's multiple comparison test. Dose-response relationships were generated using multiple doses of drug and IC₅₀ values with 95 % CI were estimated using linear regression.

From the foregoing description and examples, one skilled in the art can ascertain the essential characteristics of the invention and, without departing from the scope of the invention, can make changes, modifications, and variations of the invention to adapt it to various uses and conditions.

## Claims

1. A method of identifying a compound that associates with a TNF-α-converting enzyme (TACE) polypeptide, comprising:
(A) crystallizing said TACE polypeptide, wherein said crystallizing involves
(i) obtaining a fragment of said TACE polypeptide, said fragment consisting essentially of the catalytic domain of TACE,
(ii) incubating the fragment with octylglucoside in the presence of a TACE inhibitor, and
(iii) crystallizing the fragment and determining the crystal structure of the fragment;
(B) designing an associating compound for the TACE polypeptide that forms a bond with the catalytic domain based on X-ray diffraction coordinates of the TACE polypeptide crystal;
(C) synthesizing the compound; and
(D) determining the associate capability of the compound with the polypeptide.

2. The method according to claim 1, in which the associating compound is an inhibitor, mediator or other compound that regulates TACE activity.

3. The method according to claim 1 or 2, in which the associating compound is a competitive inhibitor, un-competitive inhibitor or non-competitive inhibitor.

4. A method according to any preceding claim, in which the coordinates are the coordinates of Table 1.

5. A method according to any preceding claim, in which the TACE polypeptide crystal is co-crystallized with a binding partner.

6. The method according to claim 5, in which the binding partner is a hydroxamate-based binding partner.

7. The method according to claim 5, in which the binding partner is N-{D,L-2-(hydroxyaminocarbonyl)methyl-4-methylpentanoyl}-L-3-amino-2-dimethylbutanoyl-L-alanine,2-(amino)ethyl amide.

8. A method according to any preceding claim, in which the TACE polypeptide crystal has a crystal structure diffracting to 2.0 Å.

9. A method according to any preceding claim, in which the TACE polypeptide crystal is monoclinic.

10. A method according to any preceding claim, in which the TACE polypeptide crystal has a unit cell comprising four crystallographically independent TACE catalytic domain (TCD) molecules.

11. The method according to claim 10, in which the TCD molecules are in an asymmetric unit.

12. A method according to any preceding claim, in which the TACE polypeptide crystal is of monoclinic space group P2₁ and the cell has the constants a = 61.38 Å, b = 126.27 Å, c = 81.27 Å and β = 107.41°.

13. A method according to any preceding claim, in which the associating compound is designed to associate with the S1' region of TACE.

14. A method according to any of claims 1- 12, in which the associating compound is designed to associate with the S1'S3' pocket of TACE.

15. A method according to any of claims 1- 12, in which the associating compound is designed to incorporate a moiety that chelates zinc.

16. A method according to any of claims 1 - 12, in which the associating compound is designed to form a hydrogen bond with Leu348 or Gly349 of TACE.

17. A method according to any of claims 1 - 12, in which the associating compound is designed to introduce a non-polar group which occupies the S1' pocket of TACE.

18. A method according to any of claims 1 - 12, in which the associating compound is designed to introduce a group which lies within the channel joining S1'-S3' pockets of TACE and which makes appropriate van der Waal contact with the channel.

19. A method according to any of claims 1 - 12, in which the associating compound is designed to form a hydrogen bond with Leu348 or Gly349 on the backbone amide groups of TACE.

## Patentansprüche

1. Verfahren zum Identifizieren einer Verbindung, die sich mit einem "TNF-α-converting enzyme"-(TACE)-Polypeptid assoziiert, das die folgenden Schritte beinhaltet:
(A) Kristallisieren des genannten TACE-Polypeptids, wobei das genannte Kristallisieren Folgendes beinhaltet:
(i) Beziehen eines Fragments des genannten TACE-Polypeptids, wobei das genannte Fragment im Wesentlichen aus der katalytischen Domäne von TACE besteht,
(ii) Inkubieren des Fragments mit Octylglucosid in Anwesenheit eines TACE-Inhibitors und
(iii) Kristallisieren des Fragments und Bestimmen der Kristallstruktur des Fragments;
(B) Entwerfen einer assoziierenden Verbindung für das TACE-Polypeptid, die eine Bindung mit der katalytischen Domäne bildet, auf der Basis von Röntgenstrahlbeugungskoordinaten des TACE-Polypeptidkristalls;
(C) Synthetisieren der Verbindung; und
(D) Bestimmen der Assoziationsfähigkeit der Verbindung mit dem Polypeptid.

2. Verfahren nach Anspruch 1, wobei die assoziierende Verbindung ein Inhibitor, Mediator oder eine andere Verbindung ist, die die TACE-Aktivität reguliert.

3. Verfahren nach Anspruch 1 oder 2, wobei die assoziierende Verbindung ein kompetitiver Inhibitor, ein unkompetitiver Inhibitor oder nichtkompetitiver Inhibitor ist.

4. Verfahren nach einem der vorherigen Ansprüche, wobei die Koordinaten die Koordinaten der Tabelle 1 sind.

5. Verfahren nach einem der vorherigen Ansprüche, wobei der TACE-Polypeptidkristall mit einem Bindungspartner cokristallisiert wird.

6. Verfahren nach Anspruch 5, wobei der Bindungspartner ein Bindungspartner auf Hydroxamatbasis ist.

7. Verfahren nach Anspruch 5, wobei der Bindungspartner N-{D,L-2-(Hydroxyaminocarbonyl)methyl-4-methylpentanoyl}-L-3-amino-2-dimethylbutanoyl-L-alanin,2-(amino)ethylamid ist.

8. Verfahren nach einem der vorherigen Ansprüche, wobei der TACE-Polypeptidkristall eine Kristallstruktur hat, die auf 2,0 Å beugt.

9. Verfahren nach einem der vorherigen Ansprüche, wobei der TACE-Polypeptidkristall monoklin ist.

10. Verfahren nach einem der vorherigen Ansprüche, wobei der TACE-Polypeptidkristall eine Einheitszelle hat, die vier kristallografisch unabhängige TACE-Domänen-(TCD)-Katalysedomänen-Moleküle hat.

11. Verfahren nach Anspruch 10, wobei die TCD-Moleküle in einer asymmetrischen Einheit sind.

12. Verfahren nach einem der vorherigen Ansprüche, wobei der TACE-Polypeptidkristall zur monoklinen Raumgruppe P2₁ gehört und die zelle die Konstanten a = 61,38 Å, b = 126,27 Å, c = 81,27 Å und β = 107,41° hat.

13. Verfahren nach einem der vorherigen Ansprüche, wobei die assoziierende Verbindung dafür vorgesehen ist, sich mit der S1' Region von TACE zu assoziieren.

14. Verfahren nach einem der Ansprüche 1-12, wobei die assoziierende Verbindung dafür vorgesehen ist, sich mit der S1'S3' Tasche von TACE zu assoziieren.

15. Verfahren nach einem der Ansprüche 1-12, wobei die assoziierende Gruppe dafür vorgesehen ist, einen Anteil einzubeziehen, der Zink chelatisiert.

16. Verfahren nach einem der Ansprüche 1-12, wobei die assoziierende Verbindung dafür vorgesehen ist, eine Wasserstoffbindung mit Leu348 oder Gly349 von TACE zu bilden.

17. Verfahren nach einem der Ansprüche 1-12, wobei die assoziierende Verbindung dafür vorgesehen ist, eine nichtpolare Gruppe einzuführen, die die S1' Tasche von TACE belegt.

18. Verfahren nach einem der Ansprüche 1-12, wobei die assoziierende Verbindung dafür vorgesehen ist, eine Gruppe einzuführen, die innerhalb des die S1'-S3' Taschen von TACE verbindenden Kanals liegt und die einen angemessen van-der-Waals-Kontakt mit dem Kanal macht.

19. Verfahren nach einem der Ansprüche 1-12, wobei die assoziierende Verbindung dafür vorgesehen ist, eine Wasserstoffbindung mit Leu348 oder Gly349 auf den Rückgratamidgruppen von TACE zu bilden.

## Revendications

1. Méthode d'identification d'un composé qui s'associe à un polypeptide (TACE) de l'enzyme de conversion du TNF-α, comprenant:
(A) cristalliser ledit polypeptide TACE, où ladite cristallisation englobe:
(i) obtenir un fragment dudit polypeptide TACE, ledit fragment consistant essentiellement en le domaine catalytique de TACE,
(ii) incuber le fragment avec de l'octylglucoside en présence d'un inhibiteur de TACE, et
(iii) cristalliser le fragment et déterminer la structure cristalline du fragment;
(B) concevoir un composé d'association pour le polypeptide TACE, qui forme une liaison avec le domaine catalytique, sur la base des coordonnées de diffraction des rayons X du cristal de polypeptide TACE;
(C) synthétiser le composé; et
(D) déterminer la capabilité d'association du composé avec le polypeptide.

2. La méthode selon la revendication 1, dans laquelle le composé d'association est un inhibiteur, un médiateur ou un autre composé qui régule l'activité TACE.

3. La méthode selon la revendication 1 ou 2, dans laquelle le composé d'association est un inhibiteur compétitif, un inhibiteur incompétitif ou un inhibiteur non compétitif.

4. Méthode selon l'une quelconque des revendications précédentes, dans laquelle les coordonnées sont les coordonnées du Tableau 1.

5. Méthode selon l'une quelconque des revendications précédentes, dans laquelle le cristal de polypeptide TACE est co-cristallisé avec un partenaire de liaison.

6. La méthode selon la revendication 5, dans laquelle le partenaire de liaison est un partenaire de liaison à base d'hydroxamate.

7. La méthode selon la revendication 5, dans laquelle le partenaire de liaison est du N-{D,L-2-(hydroxyaminocarbonyl)méthyl-4-méthylpentanoyl}-L-3-amino-2-diméthylbutanoyl-L-alanine,2-(amino)éthyl-amide.

8. Méthode selon l'une quelconque des revendications précédentes, dans laquelle le cristal de polypeptide TACE a une structure cristalline qui se diffracte à 2,0 Å.

9. Méthode selon l'une quelconque des revendications précédentes, dans laquelle le cristal de polypeptide TACE est monoclinique.

10. Méthode selon l'une quelconque des revendications précédentes, dans laquelle le cristal de polypeptide TACE a une cellule unitaire comprenant quatre molécules du domaine catalytique de TACE (TCD) cristallographiquement indépendantes.

11. La méthode selon la revendication 10, dans laquelle les molécules TCD sont dans une unité asymétrique.

12. Méthode selon l'une quelconque des revendications précédentes, dans laquelle le cristal de polypeptide TACE est un groupe d'espace monoclinique P2₁ et la cellule a les constantes a = 61,38 Å, b = 126,27 Å, c = 81,27 Å et β = 107,41°.

13. Méthode selon l'une quelconque des revendications précédentes, dans laquelle le composé d'association est conçu pour s'associer à la région S1' de TACE.

14. Méthode selon l'une quelconque des revendications 1 - 12, dans laquelle le composé d'association est conçu pour s'associer à la poche S1'S3' de TACE.

15. Méthode selon l'une quelconque des revendications 1 - 12, dans laquelle le composé d'association est conçu pour incorporer un fragment qui chélate le zinc.

16. Méthode selon l'une quelconque des revendications 1-12, dans laquelle le composé d'association est conçu pour former une liaison hydrogène avec Leu348 ou Gly349 de TACE.

17. Méthode selon l'une quelconque des revendications 1-12, dans laquelle le composé d'association est conçu pour introduire un groupe non polaire qui occupe la poche S1' de TACE.

18. Méthode selon l'une quelconque des revendications 1-12, dans laquelle le composé d'association est conçu pour introduire un groupe qui se trouve dans le canal joignant les poches S1'-S3' de TACE et qui établit un contact de van der Waal approprié avec le canal.

19. Méthode selon l'une quelconque des revendications 1-12, dans laquelle le composé d'association est conçu pour former une liaison hydrogène avec Leu348 ou Gly349 sur les groupes amide d'ossature de TACE.


### SEQUENCE LISTING

<110> BLACK, Roy A.
   PAXTON, Raymond James
   BODE, Wolfram
   MASKOS, Klaus
   FERNANDEZ-CATALAN, Carlos
   CHEN, James Ming
   LEVIN, Jeremy Ian
   IMMUNEX CORPORATION
   MAX-PLANCK-INSTITUTE FOR BIOCHEMISTRY
   AMERICAN HOME PRODUCTS CORPORATION
<120> CRYSTALLINE TNF-α-CONVERTING ENZYME AND USES THEREOF
<130> 016761/0149
<140> PCT/US99/02185
   <141> 1999-02-03
<150> US 60/117,476
   <151> 1999-01-27
<150> US 09/050,083
   <151> 1998-03-30
<150> US 60/073,709
   <151> 1998-02-04
<160> 7
<170> PatentIn Ver. 2.0
<210> 1
   <211> 10
   <212> PRT
   <213> Artificial Sequence
<220>
   <223> Description of Artificial Sequence: peptide
<400> 1
   Pro Leu Ala Gln Ala Val Arg Ser Ser Ser
    1               5                   10
<210> 2
   <211> 8
   <212> PRT
   <213> Artificial Sequence
<220>
   <223> Description of Artificial Sequence: peptide
<400> 2
   Gly Ser His His His His His His
    1               5
<210> 3
   <211> 11
   <212> PRT
   <213> Artificial Sequence
<220>
   <223> Description of Artificial Sequence: zinc binding consensus motif
<220>
   <221> VARIANT
   <222> (3)..(4)
   <223> Xaa can be any amino acid
<220>
   <221> VARIANT
   <222> (6)..(7)
   <223> Xaa can be any amino acid
<220>
   <221> VARIANT
   <222> (9)..(10)
   <223> Xaa can be any amino acid
<400> 3
   His Glu Xaa Xaa His Xaa Xaa Gly Xaa Xaa His
    1               5                   10
<210> 4
   <211> 203
   <212> PRT
   <213> snake venom
<400> 4
   Glu Gln Asn Leu Pro Gln Arg Tyr Ile Glu Leu Val Val Val Ala Asp
    1               5                  10                  15
   Arg Arg Val Phe Met Lys Tyr Asn Ser Asp Leu Asn Ile Ile Arg Thr
               20                  25                  30
   Arg Val His Glu Ile Val Asn Ile Ile Asn Glu Phe Tyr Arg Ser Leu
           35                  40                  45
   Asn Ile Arg Val Ser Leu Thr Asp Leu Glu Ile Trp Ser Gly Gln Asp
       50                  55                  60
   Phe Ile Thr Ile Gln Ser Ser Ser Ser Asn Thr Leu Asn Ser Phe Gly
   65                  70                  75                  80
   Glu Trp Arg Glu Arg Val Leu Leu Thr Arg Lys Arg His Asp Asn Ala
                   85                  90                  95
   Gln Leu Leu Thr Ala Ile Asn Phe Glu Gly Lys Ile Ile Gly Lys Ala
               100                 105                 110
   Tyr Thr Ser Ser Met Cys Asn Pro Arg Ser Ser Val Gly Ile Val Lys
           115                 120                 125
   Asp His Ser Pro Ile Asn Leu Leu Val Ala Val Thr Met Ala His Glu
       130                 135                 140
   Leu Gly His Asn Leu Gly Met Glu His Asp Gly Lys Asp Cys Leu Arg
   145                 150                 155                 160
   Gly Ala Ser Leu Cys Ile Met Arg Pro Gly Leu Thr Pro Gly Arg Ser
                   165                 170                 175
   Tyr Glu Phe Ser Asp Asp Ser Met Gly Tyr Tyr Gln Lys Phe Leu Asn
               180                 185                 190
   Gln Tyr Lys Pro Gln Cys Ile Leu Asn Lys Pro
           195                 200
<210> 5
   <211> 287
   <212> PRT
   <213> mammalian or insect
<220>
   <223> Description of Artificial Sequence: polypeptide
<400> 5
   Pro Glu Glu Leu Val His Arg Val Lys Arg Arg Ala Asp Pro Asp Pro
   1               5                   10                  15
   Met Lys Asn Thr Cys Lys Leu Leu Val Val Ala Asp His Arg Phe Tyr
               20                  25                  30
   Arg Tyr Met Gly Arg Gly Glu Glu Ser Thr Thr Thr Asn Tyr Leu Ile
           35                  40                  45
   Glu Leu Ile Asp Arg Val Asp Asp Ile Tyr Arg Asn Thr Ser Trp Asp
       50                  55                  60
   Asn Ala Gly Phe Lys Gly Tyr Gly Ile Gln Ile Glu Gln Ile Arg Ile
   65                  70                  75                  80
   Leu Lys Ser Pro Gln Glu Val Lys Pro Gly Glu Lys His Tyr Asn Met
                   85                  90                  95
   Ala Lys Ser Tyr Pro Asn Glu Glu Lys Asp Ala Trp Asp Val Lys Met
               100                 105                 110
   Leu Leu Glu Gln Phe Ser Phe Asp Ile Ala Glu Glu Ala Ser Lys Val
           115                 120                 125
   Cys Leu Ala His Leu Phe Thr Tyr Gln Asp Phe Asp Met Gly Thr Leu
       130                 135                 140
   Gly Leu Ala Tyr Val Gly Ser Pro Arg Ala Asn Ser His Gly Gly Val
   145                 150                 155                 160
   Cys Pro Lys Ser Gly Ser Ser Gly Gly Ile Cys Glu Lys Ala Tyr Tyr
                   165                 170                 175
   Ser Pro Val Gly Lys Lys Asn Ser Lys Leu Tyr Ser Asp Gly Lys Lys
               180                 185                 190
   Lys Glu Ala Asp Leu Val Thr Thr His Glu Leu Gly His Asn Phe Gly
           195                 200                 205
   Ala Glu His Asp Pro Asp Gly Leu Ala Glu Cys Ala Pro Asn Glu Asp
       210                 215                 220
   Gln Gly Gly Lys Tyr Val Met Tyr Pro Ile Ala Val Ser Gly Asp His
   225                 230                 235                 240
   Glu Asn Asn Lys Met Phe Ser Asn Cys Ser Lys Gln Ser Ile Tyr Lys
                   245                 250                 255
   Thr Ile Glu Ser Lys Ala Gln Glu Cys Phe Gln Glu Arg Ser Asn Lys
               260                 265                 270
   Val Cys Gly Asn Ser Arg Val Asp Glu Gly Glu Glu Cys Asp Pro
           275                 280                 285
<210> 6
   <211> 276
   <212> PRT
   <213> Homo sapiens
<400> 6
   Gln Glu Lys His Ala Ile Asn Gly Pro Glu Leu Leu Arg Lys Arg Arg
    1               5                  10                  15
   Thr Thr Ser Ala Glu Lys Asn Thr Cys Gln Leu Tyr Ile Gln Thr Asp
               20                  25                  30
   His Leu Phe Phe Lys Tyr Tyr Gly Thr Arg Glu Ala Val Ile Ala Gln
           35                  40                  45
   Ile Ser Ser His Val Lys Ala Ile Asp Thr Ile Tyr Gln Thr Thr Asp
       50                  55                  60
   Phe Ser Gly Ile Arg Asn Ile Ser Phe Met Val Lys Arg Ile Arg Ile
   65                  70                  75                  80
   Asn Thr Thr Ala Asp Glu Lys Asp Pro Thr Asn Pro Phe Arg Phe Pro
                   85                  90                  95
   Asn Ile Ser Val Glu Lys Phe Leu Glu Leu Asn Ser Glu Gln Asn His
               100                 105                 110
   Asp Asp Tyr Cys Leu Ala Tyr Val Phe Thr Asp Arg Asp Phe Asp Asp
           115                 120                 125
   Gly Val Leu Gly Leu Ala Trp Val Gly Ala Pro Ile Tyr Leu Asn Ser
       130                 135                 140
   Gly Leu Thr Ser Thr Ser Leu Asn Thr Gly Ile Ile Thr Val Lys Asn
   145                 150                 155                 160
   Tyr Gly Lys Thr Ile Leu Thr Lys Gln Asn Tyr Gly Ser His Val Pro
                   165                 170                 175
   Pro Lys Val Ser His Ile Thr Phe Ala His Glu Val Gly His Asn Phe
               180                 185                 190
   Gly Ser Pro His Asp Ser Gly Thr Glu Cys Thr Pro Gly Glu Ser Lys
           195                 200                 205
   Asn Leu Gly Gln Lys Glu Asn Gly Asn Tyr Ile Met Tyr Ala Arg Ala
       210                 215                 220
   Thr Ser Gly Asp Lys Leu Asn Asn Asn Lys Phe Ser Leu Cys Ser Ile
   225                 230                 235                 240
   Arg Asn Ile Ser Gln Val Leu Glu Lys Lys Arg Asn Asn Cys Phe Val
                   245                 250                 255
   Glu Ser Gly Gln Pro Ile Cys Gly Asn Gly Met Val Glu Gln Gly Glu
               260                 265                 270
   Glu Cys Asp Cys
           275
<210> 7
   <211> 12
   <212> PRT
   <213> Artificial Sequence
<220>
   <223> Description of Artificial Sequence: peptide
<400> 7
   Ser Pro Leu Ala Gln Ala Val Arg Ser Ser Ser Arg
   1 5 10